# EUROPEAN PATENT APPLICATION

(11) **EP 4 309 669 A1**
(43) Date of publication of application: **24.01.2024**
(21) Application number: 22770577.9
(22) Date of filing: 16.03.2022
(51) Int. Cl.: A61K 39/00, C07K 16/28, C07K 14/435, C07K 19/00, A61P 35/00

(54) **UNIVERSAL CHIMERIC ANTIGEN RECEPTOR T-CELL AND APPLICATION THEREOF**

(30) Priority: 16.03.2021 CN 202110280575
(71) Applicant: Shanghai Iaso Biotechnology Co., Ltd., Shanghai 201203 (CN)
(72) Inventor: HU, Guang, Nanjing, Jiangsu 210031 (CN); ZHANG, Jiayuan, Shanghai 201203 (CN); GAO, Wenjing, Nanjing, Jiangsu 210031 (CN); DONG, Wenjie, Shanghai 201203 (CN); YAO, Xiaomin, Shanghai 201203 (CN); LIU, Zhi, Shanghai 201203 (CN); WANG, Xiaoqian, Shanghai 201203 (CN)
(74) Representative: Lavoix
(86) International application number: PCT/CN2022/081289
(87) International publication number: WO 2022/194224

(57) **Abstract**

A CELL COMPRISING A MUTANT PROTEIN, THE MUTANT PROTEIN CAUSING THE CELL TO BE INSENSITIVE TOWARD AN INHIBITOR WHICH AFFECTS THE ACTIVITY AND/OR KILLING FUNCTION THEREOF; A UNIVERSAL CHIMERIC ANTIGEN RECEPTOR T CELL WHICH IS RELATED TO THE CELL AND WHICH IS SUITABLE FOR ADMINISTRATION TO NON-SPECIFIC PATIENTS; AND A METHOD FOR PREPARING THE DESCRIBED CELLS AND AN APPLICATION OF THE CELLS IN CELL THERAPY.

## Description

### FIELD OF THE INVENTION

The present disclosure relates to chimeric antigen receptor T cells, especially universal chimeric antigen receptor T cells suitable for administration to non-specific patients. The present disclosure further relates to preparation methods and use of these chimeric antigen receptor T cells.

### BACKGROUND OF THE INVENTION

Adoptive immune cell therapy has shown great potential in the treatment of cancer, autoimmune diseases and infectious diseases. In recent years, the use of chimeric antigen receptor-expressing T cells (CAR-T cells) for treating cancer has received particular attention. At present, CAR-T cells are mainly prepared using the patient's own T cells. This involves isolating, modifying and expanding T cells for each patient, a time-consuming and expensive process. In addition, for patients such as newborns and the elderly, it is usually difficult to obtain T cells with good quality to produce specific CAR-T cells needed by patients.

One possible solution is to use T cells derived from healthy donors to generate universal CAR-T cells. Compared with autologous CAR-T cells, the universal CAR-T cells have many advantages:
(1) with stable batches of frozen cell products, universal CAR-T products can greatly shorten the treatment waiting period for patients and provide timely treatment for patients;
(2) a standardized preparation process is provided for CAR-T cell products;
(3) there is enough time to make various modifications to the cells;
(4) repeated administration can be achieved;
(5) they can be combined with CAR-T products with different targets; and
(6) the cost of CAR-T preparation is reduced through industrialized production processes.

Therefore, universal CAR-T products will be the main trend of CAR-T therapy in the future.

However, the development of universal CAR-T is also facing great challenges, and the following two problems need to be solved most:
(1) graft-versus-host disease (GvHD) caused by allogeneic cell infusion;
(2) failure of effective expansion of universal CAR-T due to being quickly cleared by the host's immune system in the host.

At present, the first problem has been basically solved. The researchers knock out the TRAC gene encoding the T cell surface receptor (TCR) on αβ-T cells through gene editing technology, effectively inhibiting CAR-T cells from indiscriminately attacking host cells by activating TCR, thereby avoiding the occurrence of GvHD.

In contrast, the second problem is more difficult to solve. In recent years, researchers have been exploring how to effectively expand universal CAR-T cells in the host. Currently, there are two mainstream solutions:
(1) Combination of anti-CD52 monoclonal antibody drug and universal CAR-T. After lymphodepletion using the monoclonal antibody drug Alemtuzumab against CD52 protein and chemotherapy drugs, the patients are infused with TRAC/CD52 double knockout CAR-T cells for treatment. In this solution, TRAC is knocked out to prevent graft-versus-host disease, and CD52 is knocked out to prevent the clearance of universal CAR-T by lymphodepletion drugs. At present, the universal CAR-T clinical data disclosed around the world are based on treatment strategies adopting the combination of TRAC/CD52 knockout and anti-CD52 monoclonal antibody drugs.
(2) Another strategy to reduce host rejection of the graft is to knock out the B2M gene encoding β2-microglobulin on the universal CAR-T. Disruption of β2-microglobulin (B2M knockout) prevents the expression of functional HLA -class I molecules on the surface of CAR-T cells. By destroying HLA-class I molecules, this solution avoids universal CAR-T from activating cytotoxic T cells in the host, thereby allowing them to proliferate for a long time. However, since HLA is an inhibitory ligand of NK cells, its absence will activate NK cells in patients to clear CAR-T cells, limiting their expansion in vivo and affecting their effectiveness.

So far, researchers have optimized CAR-T cells with TRAC/B2M gene knockout in various ways, but there is still a high possibility of being cleared by host immunity.

### SUMMARY OF THE INVENTION

In one aspect, provided herein is a cell comprising a mutant protein that causes the cell to be insensitive to an inhibitor affecting its activity and/or cytotoxicity.

In some embodiments, the mutant protein is a member of the tyrosine kinase family.

In some embodiments, the mutant protein is LCK.

In some embodiments, the LCK protein comprises T316 mutation.

In some embodiments, the LCK protein comprises T316I, T316A or T316M mutation.

In some embodiments, the inhibitor is a tyrosine kinase inhibitor.

In some embodiments, the inhibitor is dasatinib and/or ponatinib.

In some embodiments, the cell is a mammalian cell.

In some embodiments, the cell is a stem cell or immune cell.

In some embodiments, the immune cell is a NK cell or T cell.

In some embodiments, the cell expresses a chimeric antigen receptor (CAR).

In some embodiments, the mutant protein is formed by base editing, HDR and/or overexpression.

In some embodiments, the overexpression is achieved through transfection with lentivirus, retrovirus, adeno-associated virus and adenovirus.

In another aspect, provided herein is a method for introducing T316 mutation into the Lck gene of a cell, comprising introducing a base editor into the cell.

In some embodiments, the base editor is an ABE or CBE base editor.

In some embodiments, the method further comprises introducing sgRNA into the cell.

In some embodiments, the sgRNA comprises a nucleotide sequence as set forth in any one of SEQ ID NOs: 5-8.

In some embodiments, the CEB base editor is an A3A-CBE3 fusion protein.

In some embodiments, the cell is a mammalian cell.

In some embodiments, the cell is a stem cell or immune cell.

In some embodiments, the immune cell is a NK cell or T cell.

In some embodiments, the cell expresses a chimeric antigen receptor (CAR).

In another aspect, provided herein is a cell expressing a chimeric antigen receptor, wherein the cell has cytotoxicity or is induced to have cytotoxicity, and the cell is engineered so that its cytotoxicity is insensitive to a cell activity inhibitor.

In some embodiments, the cell activity inhibitor is a T cell activity inhibitor.

In some embodiments, at least one bioactive molecule in the cell is engineered to be insensitive to the cell activity inhibitor, and the bioactive molecule can be inhibited by the cell activity inhibitor in a normal T cell.

In some embodiments, the bioactive molecule acts on a chimeric antigen receptor signal transduction pathway.

In some embodiments, the bioactive molecule is a protease.

In some embodiments, the protease is a protein tyrosine kinase.

In some embodiments, the protease is LCK protein.

In some embodiments, the Lck protein tyrosine kinase comprises T316 mutation.

In some embodiments, the Lck protein tyrosine kinase comprises T316I, T316A or T316M mutation.

In some embodiments, the engineering is accomplished by using a base editor.

In some embodiments, the base editor is an ABE or CBE base editor.

In some embodiments, the T316I mutation is obtained by introducing a base editor CBE and sgRNA into the cell, and the target sequence of the sgRNA comprises a nucleotide sequence as set forth in any one of SEQ ID NOs: 5-8.

In some embodiments, the LCK protein in the cell comprises an amino acid sequence as set forth in any one of SEQ ID NOs: 9-11.

In some embodiments, the cell is a T cell or NK cell.

In some embodiments, the cell is further engineered to eliminate or weaken the cytotoxicity generated via TCR on the cell surface thereof.

In some embodiments, a TCR-related gene of the cell is knocked out.

In some embodiments, the TRAC gene of the cell is knocked out.

In some embodiments, the β2m gene of the cell is knocked out.

In some embodiments, the CIITA gene of the cell is knocked out.

In some embodiments, the T cell activity inhibitor is a protein tyrosine kinase inhibitor.

In some embodiments, the T cell activity inhibitor is an LCK protein tyrosine kinase inhibitor.

In some embodiments, the T cell activity inhibitor is dasatinib and/or ponatinib.

In another aspect, provided herein is use of the above-mentioned cell in the preparation of a universal CAR-T cell.

In another aspect, provided herein is a method for preparing a CAR cell, comprising engineering the CAR cell so that its CAR-mediated cytotoxicity is insensitive to a T cell activity inhibitor.

In some embodiments, at least one bioactive molecule of the CAR cell is engineered to be insensitive to the T cell activity inhibitor, and the bioactive molecule can be inhibited by the T cell activity inhibitor in a normal T cell.

In some embodiments, the bioactive molecule acts on a signal transduction pathway of the CAR.

In some embodiments, the bioactive molecule is a protease.

In some embodiments, the protease is a protein tyrosine kinase.

In some embodiments, the enzyme is an LCK protein tyrosine kinase.

In some embodiments, the LCK protein tyrosine kinase comprises T316 mutation.

In some embodiments, the LCK protein tyrosine kinase comprises T316I mutation.

In some embodiments, the T316I mutation is obtained by introducing a cytosine base editor and sgRNA into the CAR cell, and the sgRNA comprises a nucleotide sequence as set forth in any one of SEQ ID NOs: 5-8.

In some embodiments, the LCK protein tyrosine kinase in the CAR cell comprises an amino acid sequence as set forth in any one of SEQ ID NOs: 9-11.

In some embodiments, the CAR cell is a T cells or NK cell.

In some embodiments, the CAR cell is further engineered to eliminate or weaken the cytotoxicity generated via TCR on the cell surface thereof.

In some embodiments, a TCR-related gene of the CAR cell is knocked out.

In some embodiments, the TRAC gene of the CAR cell is knocked out.

In some embodiments, the β2m gene of the CAR cell is knocked out.

In some embodiments, the CIITA gene of the CAR cell is knocked out.

In some embodiments, the T cell activity inhibitor is a protein tyrosine kinase inhibitor.

In some embodiments, the T cell activity inhibitor is an LCK protein tyrosine kinase inhibitor.

In some embodiments, the T cell activity inhibitor is dasatinib and/or ponatinib.

In some embodiments, during the preparation of the CAR cell from a T cell or NK cell, the T cell or NK cell is contacted with the T cell activity inhibitor.

In some embodiments, the T cell or NK cell is contacted with the T cell activity inhibitor when the T316I mutation is carried out; preferably, the concentration of the T cell activity inhibitor is 100 nM.

In some embodiments, the intracellular signaling domain of the CAR comprises:
1) a signaling transduction domain from CD3z molecule and a co-stimulatory domain from CD28 molecule; and
2) optionally, i) hIL7 and CCL19, or ii) IL2RB and IL7Ra mutant,
wherein, preferably, the signaling transduction domain from CD3z molecule comprises an amino acid sequence as set forth in SEQ ID NO: 48; the co-stimulatory domain from CD28 molecule comprises an amino acid sequence as set forth in SEQ ID NO: 46; the hIL7 comprises an amino acid sequence as set forth in SEQ ID NO: 79; the CCL19 comprises an amino acid sequence as set forth in SEQ ID NO: 80; the IL2RB and the co-stimulatory domain from CD28 molecule form a IL2RB-CD3z peptide fragment which comprises an amino acid sequence as set forth in SEQ ID NO: 81; and the IL7Ra mutant comprises an amino acid sequence as set forth in SEQ ID NO: 82.

In another aspect, provided herein is a method for treating a disease in a patient, comprising administering to the patient the above-mentioned cells in combination with a cell activity inhibitor.

In some embodiments, the cells are not derived from the patient

In some embodiments, the cells are T cells.

In some embodiments, the cell inhibitor is a T cell activity inhibitor.

In some embodiments, the cell activity inhibitor is a protein tyrosine kinase inhibitor.

In some embodiments, the cell activity inhibitor is an LCK protein inhibitor.

In some embodiments, the T cell activity inhibitor is dasatinib and/or ponatinib.

In some embodiments, the disease is a tumor.

In some embodiments, the method further comprises stopping the administration of the cell activity inhibitor, so that the patient's own T cells regain activity and clear the administered cells; or increasing the dose of the cell activity inhibitor to inhibit the cytotoxicity of the cells in the patient.

In another aspect, provided herein is a pharmaceutical kit or pharmaceutical combination comprising the above-mentioned cells and a cell activity inhibitor.

In some embodiments, the cells are T cells.

In some embodiments, the cell activity inhibitor is a T cell activity inhibitor.

In some embodiments, the cell inhibitor is a protein tyrosine kinase inhibitor.

In some embodiments, the T cell activity inhibitor is an LCK protein tyrosine kinase inhibitor.

In some embodiments, the T cell activity inhibitor is dasatinib and/or ponatinib.

In another aspect, provided herein is use of the above-mentioned cells in combination with a cell activity inhibitor in preparing an anti-tumor drug.

In some embodiments, the cells are T cells.

In some embodiments, the cell activity inhibitor is a T cell activity inhibitor.

In some embodiments, the T cell activity inhibitor is a protein tyrosine kinase inhibitor.

In some embodiments, the T cell activity inhibitor is an LCK protein tyrosine kinase inhibitor.

In some embodiments, the T cell activity inhibitor is dasatinib and/or ponatinib.

The cells provided herein and the universal CAR-T cells prepared therefrom can be used for treating non-specific patients (allogeneic cell therapy), which overcome a series of problems caused by the need of existing CAR-T cells to be derived from the patients themselves.

### DESCRIPTION OF DRAWINGS

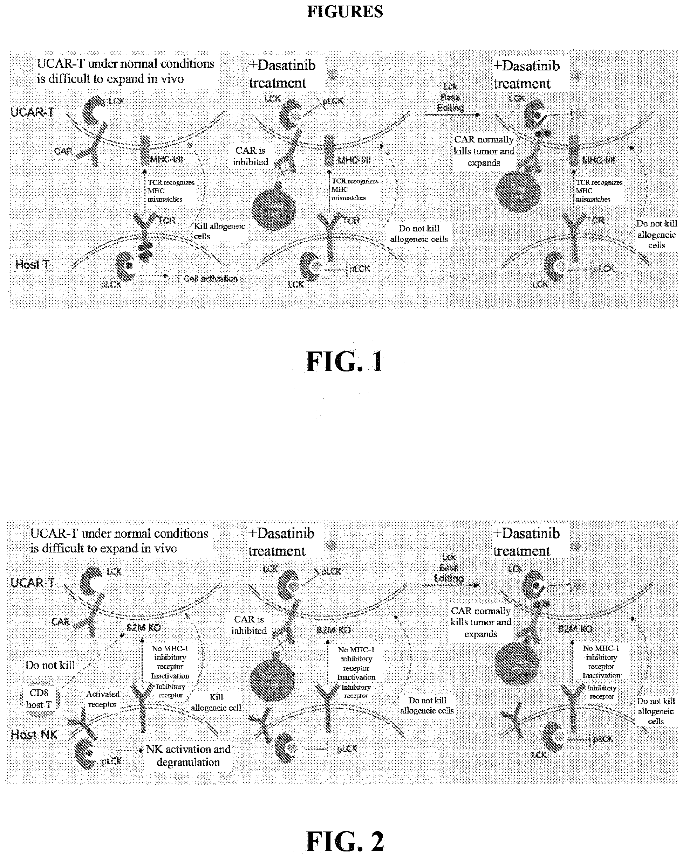

Fig. **1** is a schematic diagram of the principle of the UCART strategy in the case of effective inhibition of host T cells by dasatinib.
Fig. **2** is a schematic diagram of the principle of the UCART strategy in the case of effective inhibition of host NK cells by dasatinib.

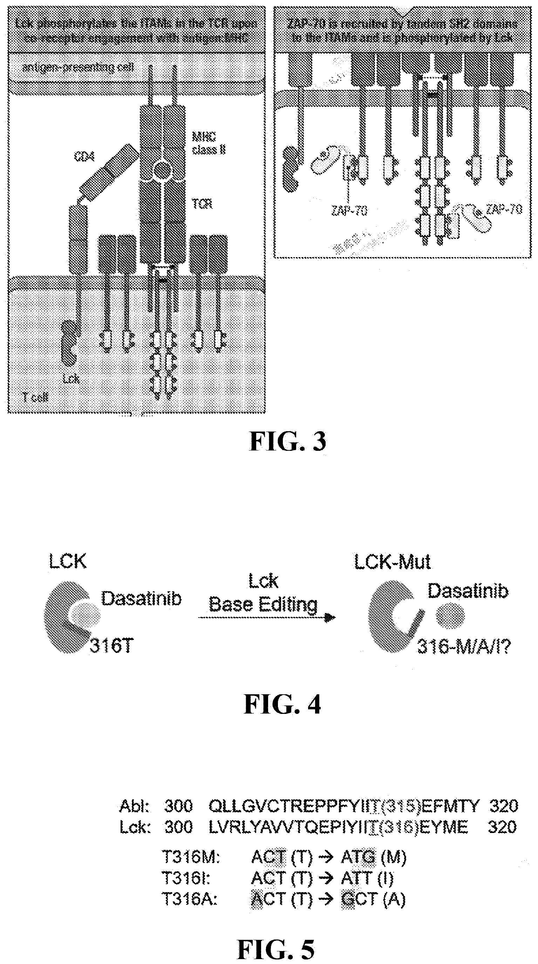

Fig. **3** is a schematic diagram of LCK mediating TCR signal transduction.
Fig. **4** is a schematic diagram of the binding relationship between dasatinib and different LCK mutations.
Fig. **5** shows a schematic diagram of the conservation of ABL and LCK kinases and the base changes of LCK mutations.

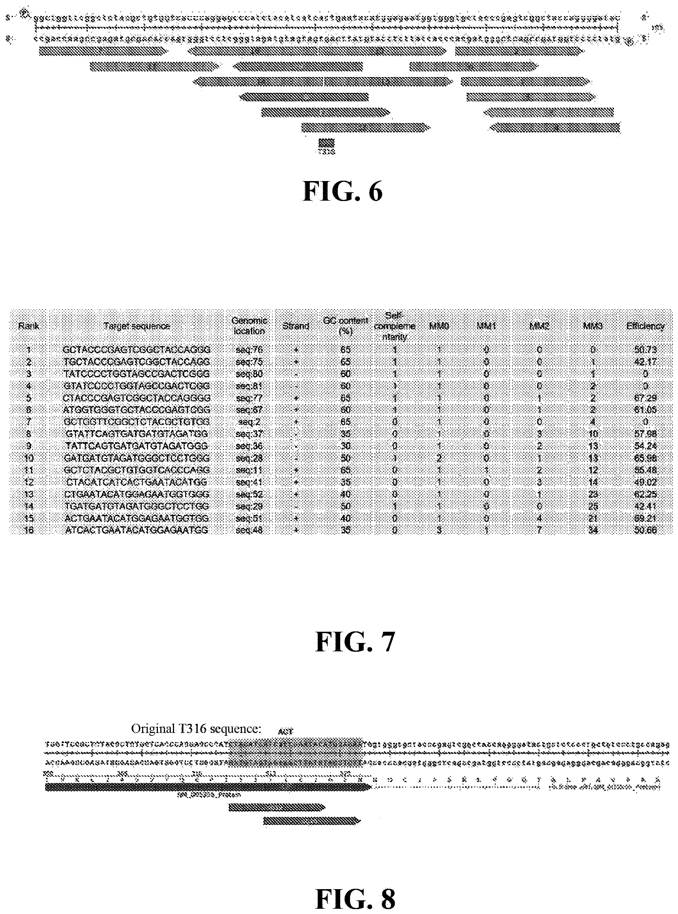

Fig. **6** shows the design and distribution of sgRNA related to LCK-T316 mutation.
Fig. **7** shows the off-target and efficiency predictions of sgRNAs associated with LCK-T316 mutation.
Fig. **8** shows the design and distribution of sgRNA suitable for CBE3-induced LCK-T316I mutation.

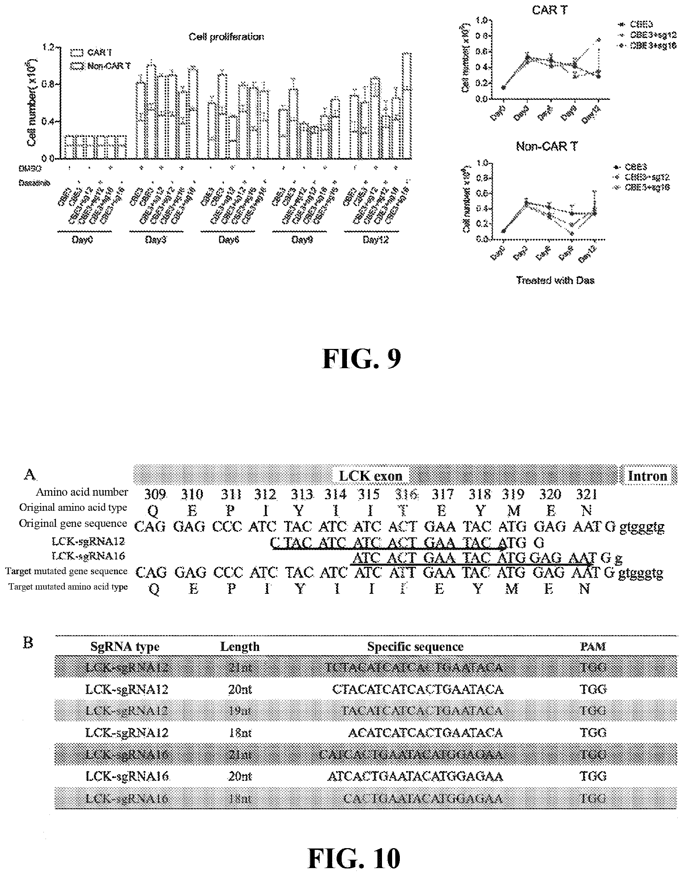

Fig. **9** shows that LCK-T316I can make anti-BCMA CAR-T cells tolerant to the inhibition of cell proliferation by dasatinib.
Fig. **10** shows the sgRNA design schematic diagram and specific sequences of single base editing for human LCK^{T316I} mutation. **10A.** Schematic diagram of sgRNA design for inducing human LCK^{T316I} mutation. The first row of numbers indicates the amino acid number of the LCK coding region, and the second row represents the type of amino acids. The uppercase letters in the third row of the original gene sequences are exon regions, and the lowercase letters are intron regions. The fourth row and the fifth row respectively indicate the target sequence of the sgRNA overlapping with the black arrow, that is, the sgRNA sequence and the PAM sequence not marked by the black arrow. The sixth row and the seventh row respectively represent the expected mutated gene sequence and amino acid sequence. Red represents the target mutation. **10B.** The specific sequence of the designed sgRNA. LCK-sgRNA12 and LCK-sgRNA16 are optimized for length, including the specific length and sequence, i.e., PAM and other information of 7 sgRNAs designed from 18nt to 21nt.

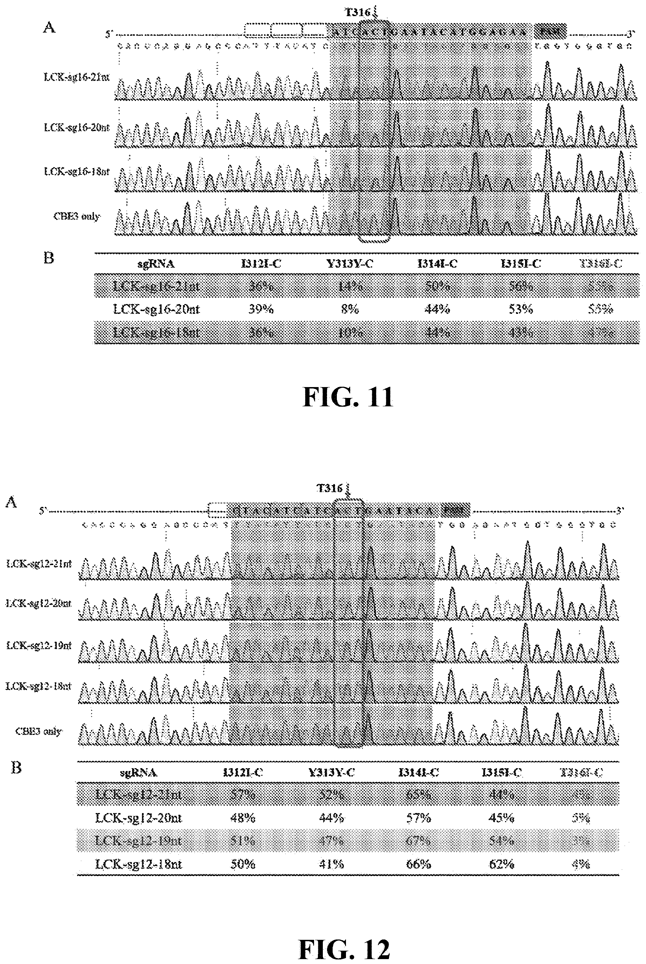

Fig. **11** shows that LCK-sgRNA16 with different lengths could efficiently induce LCK-T316I mutation. **11A.** T cells after 96 hr of electroporation editing, and the results of the peak diagram of sequencing for the LCK-T316 site. The 18nt, 20nt and 21nt LCK-sgRNA16 induced about 50% of the mutations at the cytosine at the T316 site. **11B.** The specific analysis results of mutation efficiency of the peak diagram in **11A** by using EditR.
Fig. **12** shows that LCK-sgRNA12 of different lengths could not induce LCK-T316I mutation. **12A.** T cells after 96 hr of electroporation editing, and the results of the peak diagram of sequencing for the LCK-T316 site. The 18nt, 20nt and 21nt LCK-sgRNA12 could not induce effective mutation in the cytosine at T316 site. **12B.** The specific analysis results of mutation efficiency of the peak diagram in **12A** by using EditR.

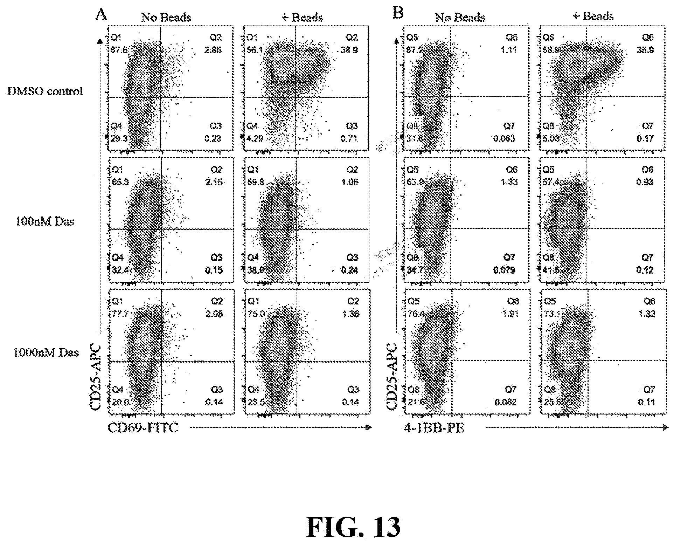

Fig. **13** shows that dasatinib could effectively inhibit TCR signals to inhibit the activation of T cells. T cells were treated with DMSO, 100nM and 1000nM dasatinib for 5hr respectively, then activated or not activated with anti-CD3/CD28 DynaBeads for 24hr, and detected for the expression of CD25, CD69 (**13A**) and 4-1BB (**13B**) by flow cytometry.

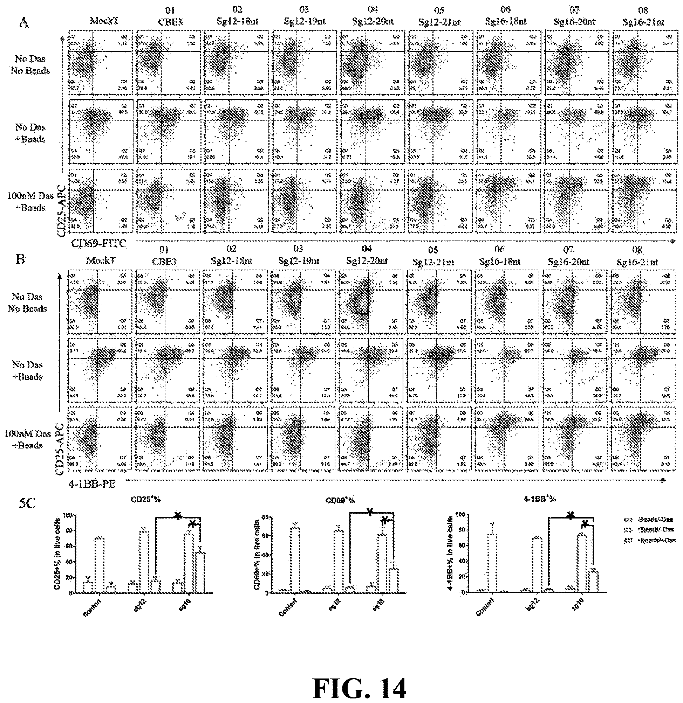

Fig. **14** shows that the LCK-T316I mutation made T cells tolerant to the inhibition of TCR activation by dasatinib. **14A, 14B.** The flow cytometry diagram shows the expression of CD25, CD69 (**14A**) and 4-1BB (**14B**) in T cells under different treatment conditions and editing conditions. The first row has no dasatinib and anti-CD3/CD28 stimulation treatment, the second row has no dasatinib but receives anti-CD3/CD28 stimulation treatment, and the third row has 100nM dasatinib pretreatment for 7hr and then receives anti-CD3/CD28 stimulation treatment. MockT is the control without electroporation, group 01 is the control with electroporation of CBE3 protein only, groups 02-05 are the controls edited by CBE3 and sgRNA12 without LCK nonsense mutation, and groups 06-08 are the LCK-T316I mutation groups edited by CBE3 and sgRNA16. **14C.** Statistics of differences in the expression percentage of T cell activation markers in different groups based on the data of **14A** and **14B.**

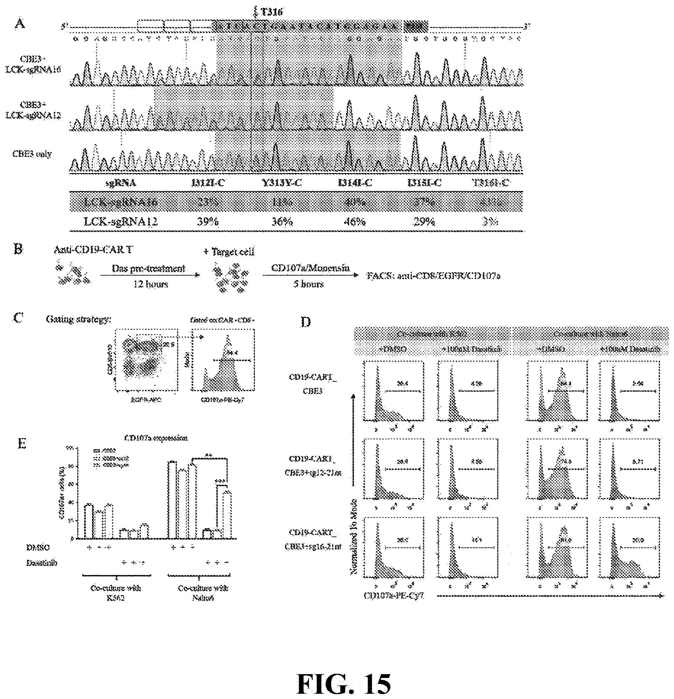

Fig. **15** shows that LCK-T316I could make anti-CD19-CAR-T cells tolerant to the inhibition of CD107a translocation by dasatinib. **15A.** Sequencing results and EditR analysis results of detection of LCK editing efficiency after anti-CD19-CAR-T cell electroporation for 72hr. Only the LCK-sgRNA16 edited group could cause LCK-T316I mutation, with a mutation efficiency of 43%. **15B.** Thumbnail of the experimental procedure in Example 5, where CAR-T cells were pretreated with 100nM dasatinib for 12hr, then the CAR-T cells were activated with the target cells, and the release of CD107a was detected after 5hr. **15C.** The gating strategy for the analysis of flow cytometry results, used to analyze the release of CD107a from CD8 and CAR double-positive T cells. **15D.** Analysis of results of flow cytometry. The left is the release of CD107a from CAR-T cells co-incubated with negative target cells K562 treated with DMSO and 100nM dasatinib, and the right is the release of CD107a from CAR-T cells co-incubated with positive target cells Nalm6 treated with DMSO and 100nM dasatinib. The three rows represent the T cells of the CBE3 electroporation control group, the LCK-sgRNA12 edited group and the LCK-sgRNA16 edited group, respectively. **15E.** Fig. **15D** Statistical analysis results of flow cytometry results.

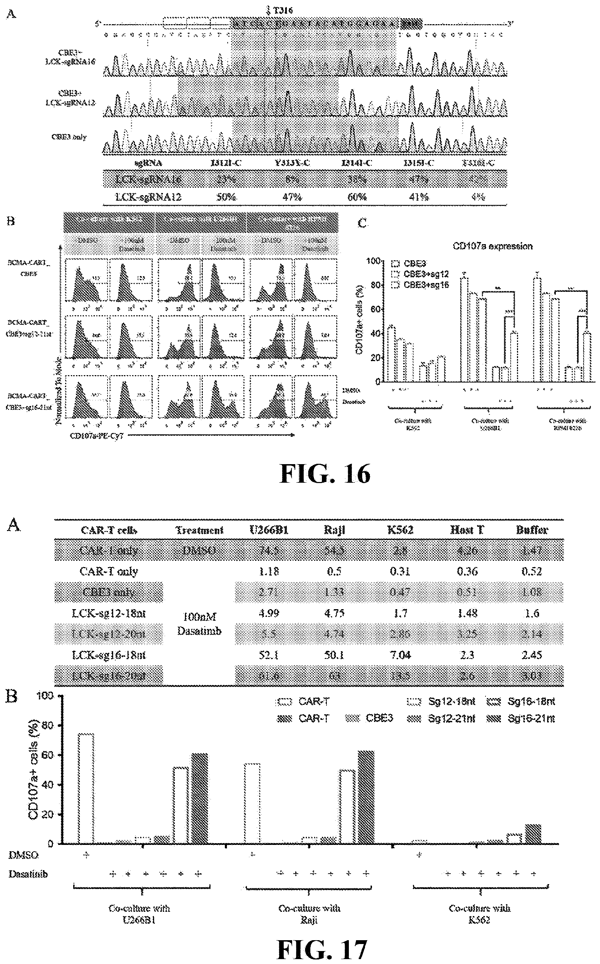

Fig. **16** shows that LCK-T316I could make anti-CMA-CAR-T cells tolerant to the inhibition of CD107a translocation by dasatinib. **16A.** Sequencing results and EditR analysis results of detection of LCK editing efficiency after anti-BCMA-CAR-T cell electroporation for 72hr. Only the LCK-sgRNA16 edited group could cause LCK-T316I mutation, with a mutation efficiency of 42%. **16B.** Analysis of results of flow cytometry. The left is the release of CD107a from CAR-T co-incubated with negative target cells K562 treated with DMSO and 100nM dasatinib, the middle is the release of CD107a from CAR-T co-incubated with positive target cell U266B1 treated with DMSO and 100nM dasatinib, and the right is the release of CD107a from CAR-T co-incubated with positive target cells RPMI8226 treated with DMSO and 100nM dasatinib. The three rows represent the T cells of the CBE3 electroporation control group, the LCK-sgRNA12 edited group and the LCK-sgRNA16 edited group, respectively. **16C.** Fig. **16B** Statistical analysis results of flow cytometry results.
Fig. **17** shows that LCK^{T316I} mutation made CD19/BCMA bispecific CAR-T cells tolerant to the inhibition of CD107a translocation by dasatinib. **17A.** Statistical data of CD107a flow cytometry results. The rows each represent the release of CD107a from different editing types of CD19/BCMA bispecific CAR-T co-incubated with different types of target cells treated with DMSO or 100 nM dasatinib. **17B.** A histogram showing the release of CD107a in Fig. **17A****.**

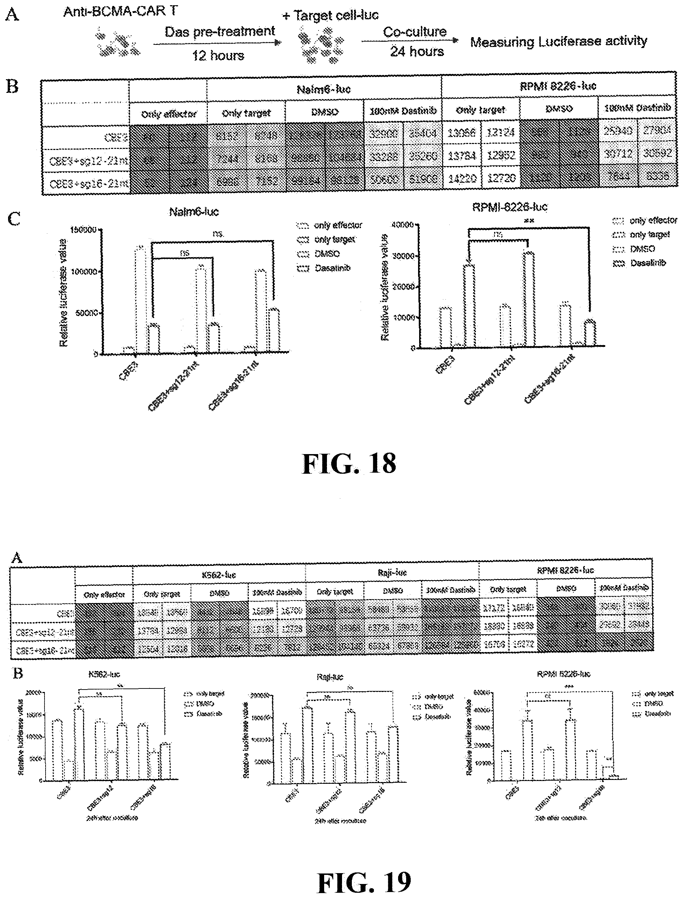

Fig. **18** shows that LCK-T316I mutation made anti-BCMA CAR-T cells tolerant to the inhibition of CAR-T cytotoxicity by dasatinib. **18A.** Thumbnail of the experimental procedure in Example 8, where CAR-T cells were pretreated with 100nM dasatinib for 12hr, then the CAR-T cells were co-incubated with the target cells for 24hr, and then the activity of luciferase in target cells was detected to indicate the killing of the target cells by CAR-T. **18B.** Numerical detection results of luciferase activity. The leftmost column shows the luciferase basal value with only CAR-T cells without the target cells, and the middle and rightmost columns show the data for CAR-T cells co-incubated with negative target cells Nalm6 and positive target cells RPMI-8226 respectively with different treatments. **18C.** Histogram and significance analysis results of the data in Fig. **18B****.**
Fig. **19** shows that LCK-T316I mutation can make anti-BCMA CAR-T cells tolerant to the inhibition of CAR-T cytotoxicity by dasatinib (repeated experiment). **19A.** Numerical detection results of luciferase activity. The leftmost column shows the luciferase basal value with only CAR-T cells without the target cells, and from the second column to the fourth column are the data of CAR-T cells co-incubated with negative target cells K562, Raji and positive target cells RPMI-8226 under different treatments. **19B.** Histogram and significance analysis results of the data in Fig. **19A****.**

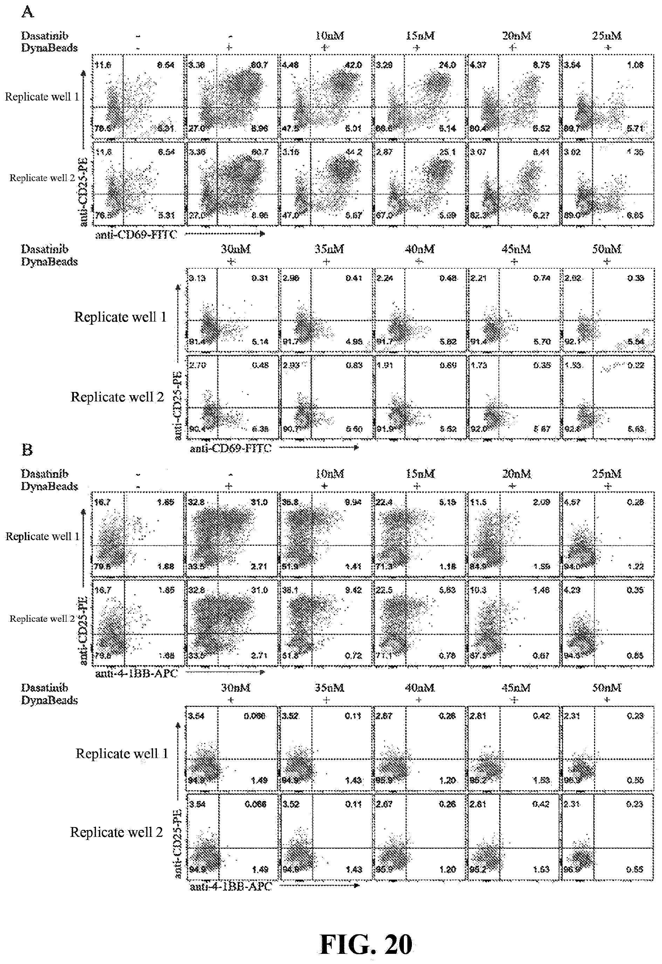

Fig. **20** shows that dasatinib at a concentration of 25nM could effectively inhibit the activation function of T cells in vitro. **20A.** Expression of CD25 and CD69 in T cells with different treatments detected by flow cytometry. 2 replicate wells for each treatment. **20B.** Expression of CD25 and 4-1BB in T cells with different treatments detected by flow cytometry. 2 replicate wells for each treatment.

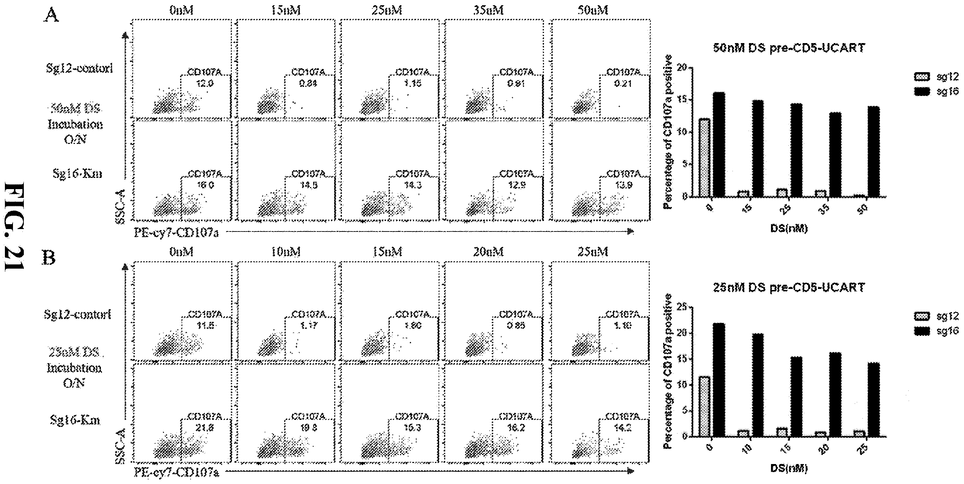

Fig. **21** shows that the inhibition of T cell activation by dasatinib has an extended effect. **21A.** Concentration adjustment experiment after pretreatment with 50nM dasatinib. On the left is the CD107a release result detected by flow cytometry. The first row shows Sg12 editing CD5-UCAR-T cells with LCK nonsense mutation, and the second row shows Sg16 editing CD5-UCAR-T cells with LCK-T316I mutation. From the leftmost column to the rightmost column, the dasatinib concentration is decreased to 0, 15, 25, 35 and 50 nM, respectively. On the right is the histogram of CD107a positive rate. **21B.** Concentration adjustment experiment after pretreatment with 25nM dasatinib. On the left is the CD107a release result detected by flow cytometry. The first row shows Sg12 editing CD5-UCAR-T cells with LCK nonsense mutation, and the second row shows Sg16 editing CD5-UCAR-T cells with LCK-T316I mutation. From the leftmost column to the rightmost column, the dasatinib concentration is decreased to 0, 10, 15, 20 and 25 nM, respectively. On the right is the histogram of CD107a positive rate.

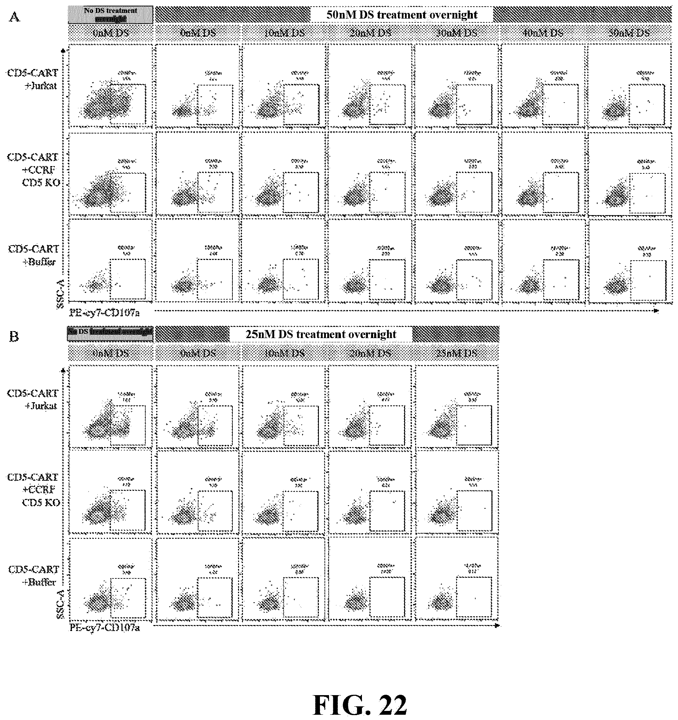

Fig. **22** shows that the inhibition of T cell activation by dasatinib has an extended effect (repeated experiments). **22A.** Results of CD107a release detected by flow cytometry, with concentration adjustment after pretreatment with 50nM dasatinib. The leftmost column shows the positive control group without dasatinib treatment, and the concentration of dasatinib is reduced to 0, 10, 20, 30, 40 and 50nM from the second column to the rightmost column, respectively. **22B.** Results of CD107a release detected by flow cytometry, with concentration adjustment after pretreatment with 25nM dasatinib. The leftmost column shows the positive control group without dasatinib treatment, and the concentration of dasatinib is reduced to 0, 10, 20, and 25nM from the second column to the rightmost column, respectively.

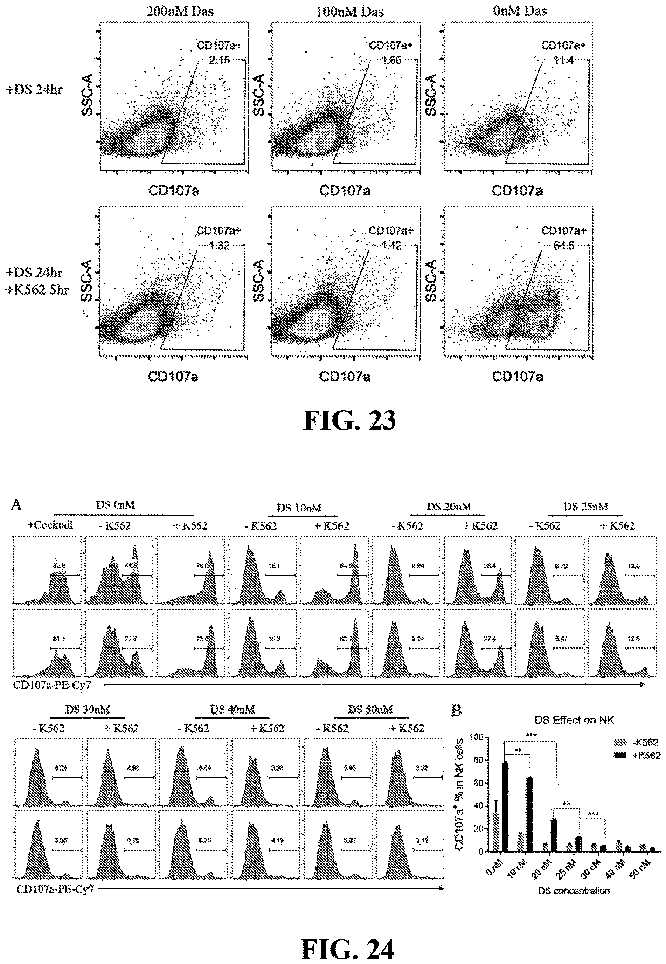

Fig. **23** shows that 100nM dasatinib could effectively inhibit the translocation of CD107a during NK cell activation. Results of CD107a release of NK cells detected by flow cytometry. The first row of NK cells were treated with different concentrations of dasatinib, but not activated by K562. The second row of cells were treated with different concentrations of dasatinib, and also activated by K562.
Fig. **24** shows that dasatinib at a concentration of 30nM could effectively inhibit NK cell activation in vitro. **24A.** Flow cytometry diagram of CD107a release of NK cells with 0-50nM dasatinib gradient treatment. The concentrations from the leftmost column in the upper row to the rightmost column in the lower row are 0, 10, 20, 25, 30, 40 and 50nM, respectively. **24B.** Histogram results of CD107a positive rate in Fig. **24A****.**

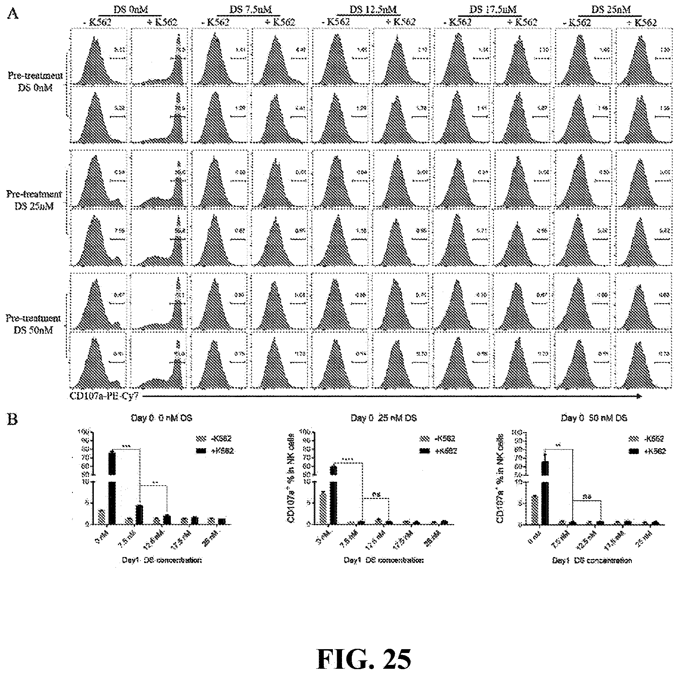

Fig. **25** shows that the inhibition of NK cell activation by dasatinib in vitro has an extended effect. **25A.** Results of CD107a release of NK cells detected by flow cytometry, with concentration adjustment after pretreatment with dasatinib of different concentrations (0, 25 and 50nM). From the leftmost group to the rightmost group, the dasatinib concentration is decreased to 0, 7.5, 12.5, 17.5 and 25nM, respectively. **25B.** Histogram of CD107a positive rate in Fig. 25A. The results show that the inhibition of NK cell activation function by dasatinib has an extended effect.

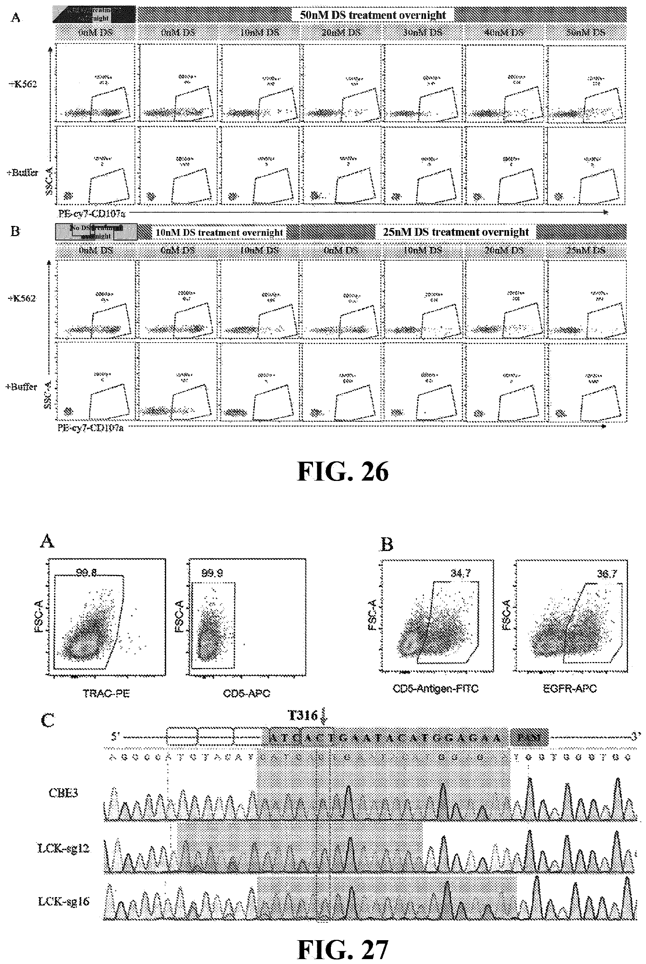

Fig. **26** shows that the inhibition of NK cell activation by dasatinib in vitro has an extended effect (repeated experiments). **26A.** Results of CD107a release of NK cells detected by flow cytometry, with concentration adjustment after pretreatment with 50nM dasatinib. The leftmost column shows the positive control without any treatment, and the concentration of dasatinib is reduced to 0, 10, 20, 30, 40 and 50nM from the second left column to the rightmost column, respectively. **26B.** Results of CD107a release of NK cells detected by flow cytometry, with concentration adjustment after pretreatment with 10nM and 25nM dasatinib. The leftmost column shows the positive control without any treatment, and the concentration is reduced to 0 and 10 nM from the second and third left columns, respectively, and the concentration of dasatinib is reduced to 0, 10, 20, and 25 nM from the fourth left column to the rightmost column, respectively.
Fig. **27** is a schematic diagram for detection of preparation parameters of CD5-UCART. **27A.** According to flow cytometric analysis, the knockout efficiency of TRAC gene is 99.8%; the knockout efficiency of CD5 gene is 99.9%. **27B.** About 35% of T cells expressing CAR are detected by detecting EGFR transduction marker and CD5 antigen. **27C.** The CBE3 group is the CD5-UCAT cell control group, the sg12 group is the CD5-UCAT cell control group with nonsense mutations with respect to LCK, and both the control groups have no LCK ^{T316I} mutation; the sg16 group (Km group) has an efficiency of LCK^{T316I} editing of 79%.

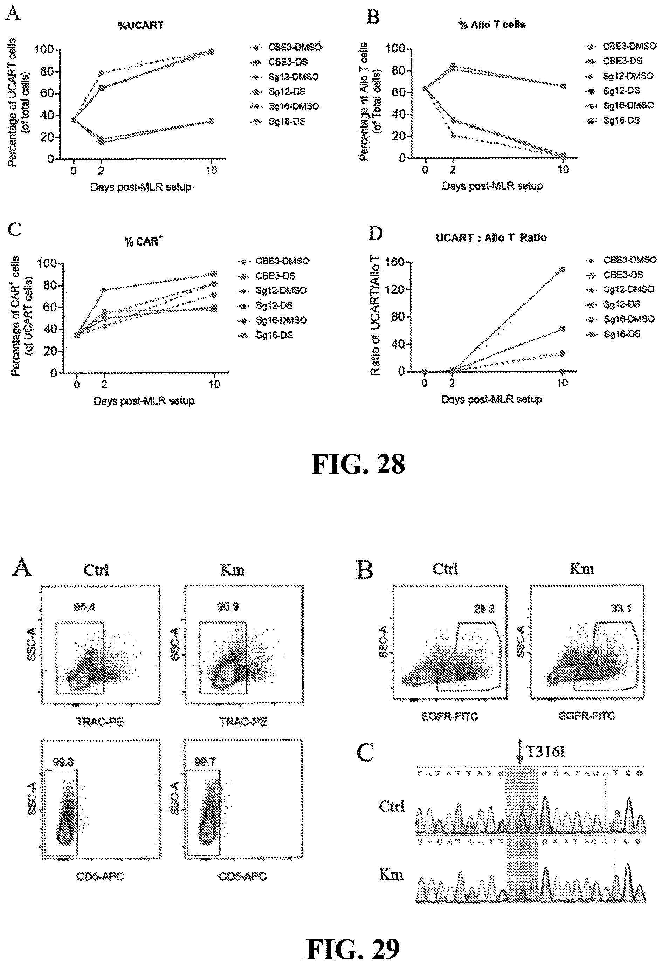

Fig. **28** shows that CD5-UCAR-T cells with LCK-T316I mutation have an expansion advantage in MLR reactions as a mixture with allogeneic T cells. **28A.** Schematic diagram of the percentage of CD5-UCAR-T cells in the overall cells at different time points in MLR reaction. **28B.** Schematic diagram of the percentage of allogeneic T cells in the overall cells at different time points in MLR reaction. **28C.** Schematic diagram of the change of CAR positive rate at different time points in MLR reaction. **28D.** Schematic diagram of changes in the ratio of UCART to allogeneic T cells at different time points in MLR reaction.
Fig. **29** is a schematic diagram for detection of preparation parameters of CD5-UCART. **29A.** Detection of TRAC, CD5 gene knockout efficiency. **29B.** The results of CAR positive rate detected by flow cytometry with anti-EGFR antibody 5 days after infection of T cells by lentivirus. **29C.** Detection of the efficiency of Km editing in CD5-UCART cells: the control group has no Km editing; the mutation efficiency of T316I in CD5-UCART cells in the sg16 (Km) group is 45%.

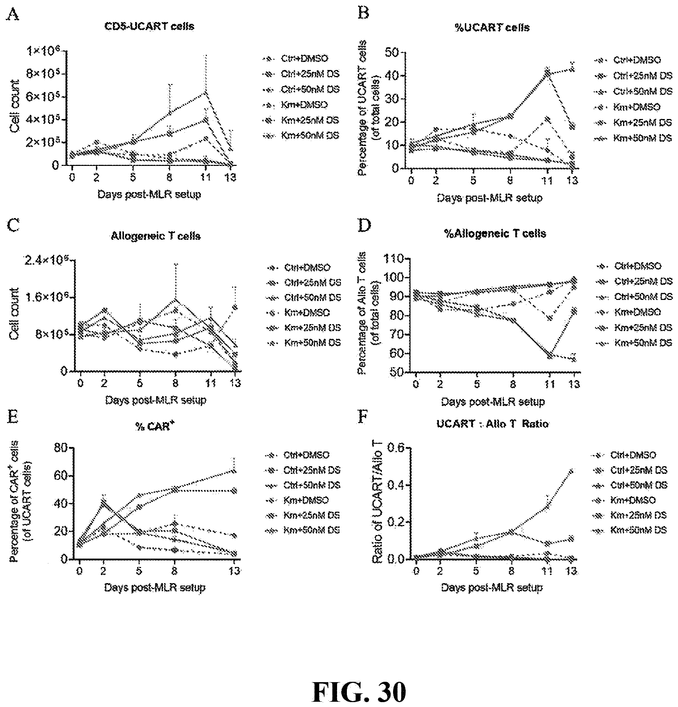

Fig. **30** shows that CD5-UCAR-T cells with LCK-T316I mutation have an expansion advantage in MLR reactions as a mixture with allogeneic T cells. **30A.** Schematic diagram of the total amount of CD5-UCAR-T cells at different time points in MLR reaction. **30B.** Schematic diagram of the percentage of CD5-UCAR-T cells in the overall cells at different time points in MLR reaction. **30C.** Schematic diagram of the total amount of allogeneic T (simulated host T) cells at different time points in MLR reaction. **30D.** Schematic diagram of the percentage of allogeneic T (simulated host T) cells in the overall cells at different time points in MLR reaction. **30E.** Schematic diagram of changes in the percentage of UCART cells expressing CAR molecules at different time points in MLR reaction. **30F.** Schematic diagram of changes in the ratio of UCART to simulated host T cells (real-time effector-to-target ratio) at different time points in MLR reaction.

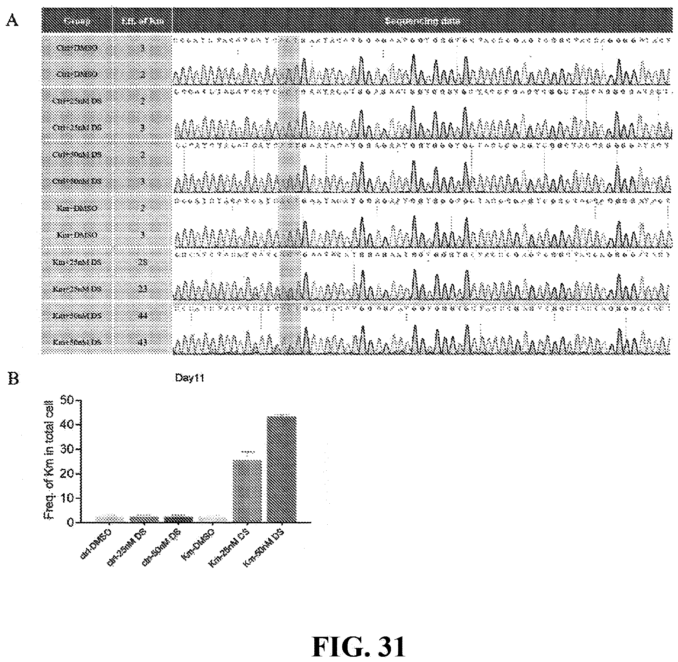

Fig. **31** shows that Km-edited UCAR-T cells tolerate the inhibition of T cell function by dasatinib. **31A.** Sequencing results of LCK gene mutations in all cells in the system on day 11 of mixed lymphoid reaction. **31B.** Results of histogram significance analysis of Fig. **31A****.**

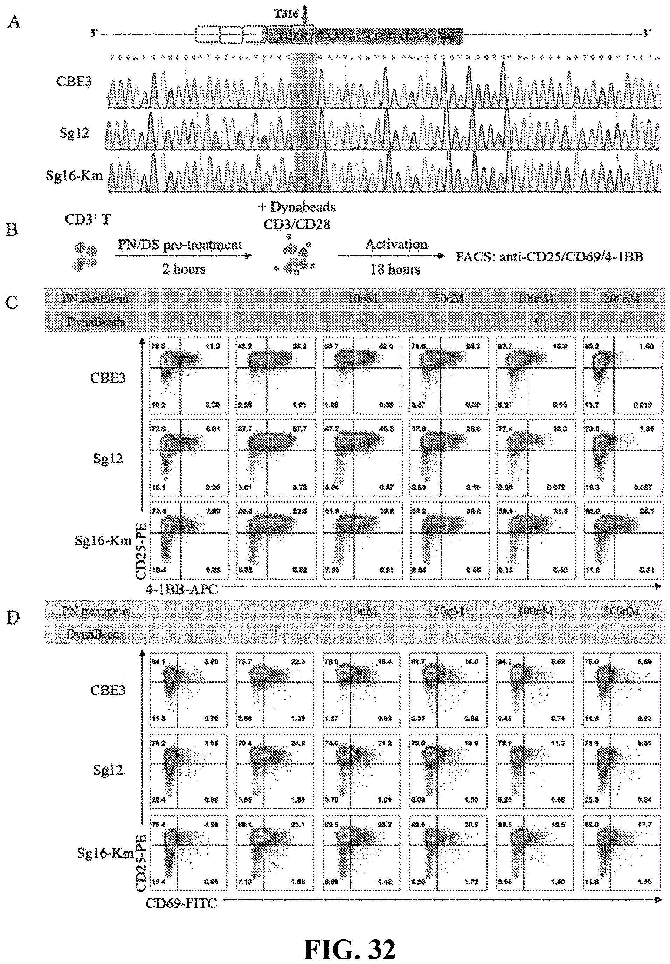

Fig. **32** shows the expression analysis results of surface marker molecules with treatments with ponatinib at concentrations of 0-200nM. **32A.** The mutation efficiency of the LCK-T316I region of T cells in three groups of different editing types as detected by Sanger sequencing. The mutation in the Sg16-Km group is 30%, while there is basically no mutation in the control groups. **32B.** Thumbnail of the experimental procedure. **32C.** T cells of different editing types are treated with different concentrations of ponatinib, and the leftmost column shows the control group without ponatinib and activation. From the second column to the rightmost column are respectively groups with 0, 10, 50, 100 and 200nM ponatinib pretreatment plus activation. The activated T cell surface marker molecule 4-1BB is detected by using the APC-labeled murine IgG monoclonal antibody, and the activated T cell surface marker molecule CD25 is detected by using the PE-labeled murine IgG monoclonal antibody. **32D.** The experiment is the same as **32C,** where the activated T cell surface molecule CD69 is detected by using the FITC-labeled murine IgG monoclonal antibody, and the activated T cell surface molecule CD25 is detected by using the PE-labeled murine IgG monoclonal antibody.

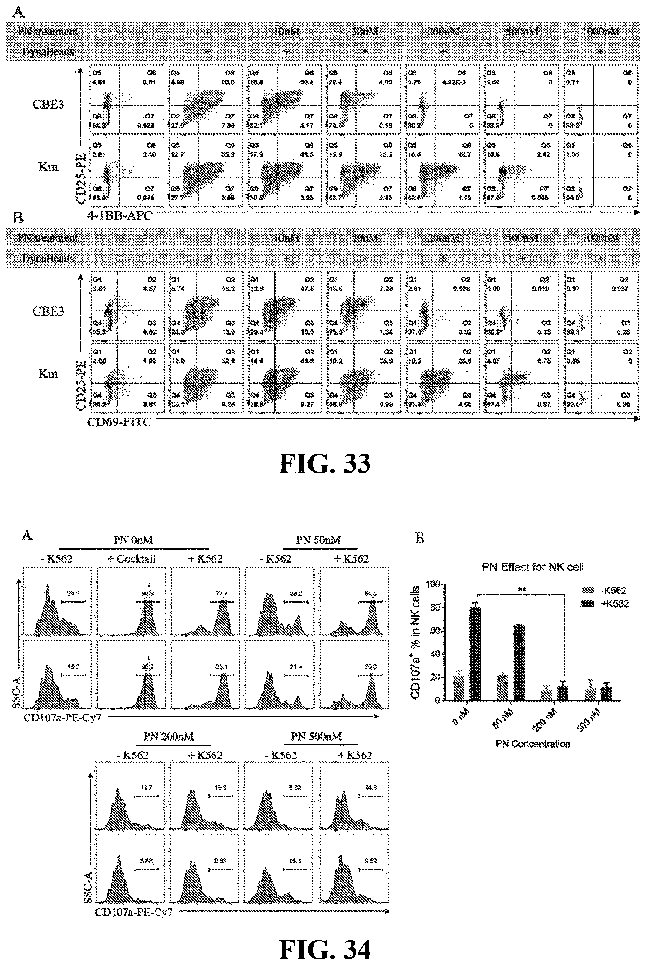

Fig. **33** shows the expression analysis results of surface marker molecules with treatments with ponatinib at concentrations of 0-1000nM. **33A.** T cells of different editing types are treated with different concentrations of ponatinib, and the leftmost column shows the control group without ponatinib and activation. From the second column to the rightmost column are respectively groups with 0, 10, 50, 200, 500 and 1000nM ponatinib pretreatment plus activation. The activated T cell surface marker molecule 4-1BB is detected by using the APC-labeled murine IgG monoclonal antibody, and the activated T cell surface marker molecule CD25 is detected by using the PE-labeled murine IgG monoclonal antibody. **33B.** The experiment is the same as 23C, where the activated T cell surface molecule CD69 is detected by using the FITC-labeled murine IgG monoclonal antibody, and the activated T cell surface molecule CD25 is detected by using the PE-labeled murine IgG monoclonal antibody.
Fig. **34** shows that ponatinib at a concentration of 200nM can effectively inhibit NK cell activation in vitro. **34A.** Flow cytometry diagram of CD107a release of NK cells with 0-500nM ponatinib concentration gradient treatment. The concentrations from the leftmost column in the upper row to the rightmost column in the lower row are 0, 50, 200 and 500nM, respectively. **34B.** The histogram results of CD107a positive rate in Fig. **34A****,** showing that 200nM ponatinib can significantly inhibit the CD107a release of NK cells activated by K562.

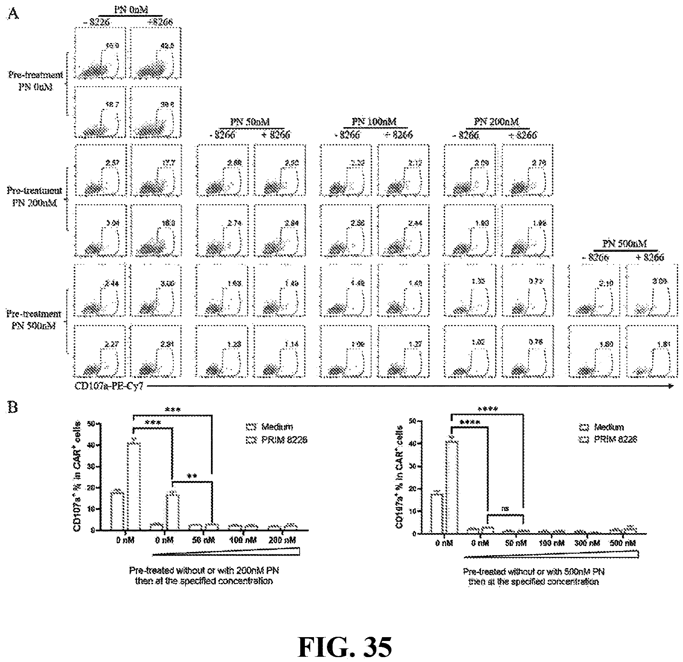

Fig. **35** shows that the inhibition of T cell activation by ponatinib has an extended effect. **35A.** Experiment of concentration adjustment after pretreatment with 0, 50 and 200nM ponatinib, results of CD107a release detected by flow cytometry. The first and second rows are incubation groups without ponatinib. For the third and fourth rows, after 200nM pretreatment, the concentration of ponatinib is decreased to 0, 50, 100 and 200nM. For the fifth and sixth rows, after 500nM pretreatment, the concentration of ponatinib is decreased to 0, 50, 100, 200 and 500nM. **35B.** Experiment of concentration adjustment after pretreatment with 0, 50 and 200nM ponatinib, histogram of CD107a positive rate of the data in 35A. The results shows that the inhibition of T cell activation by ponatinib has an extended effect.

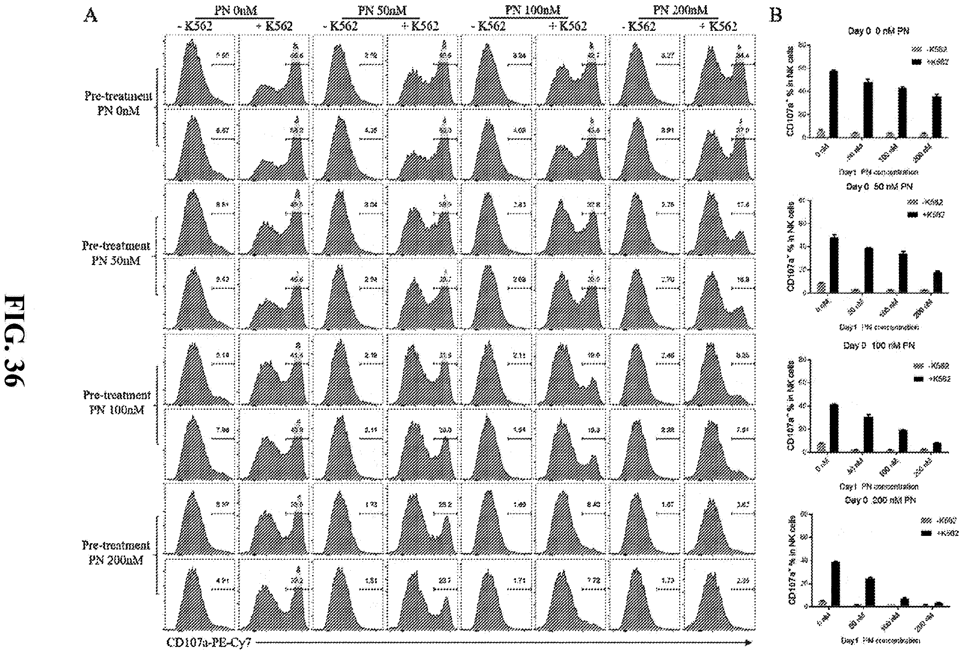

Fig. **36** shows that the inhibition of NK cell activation function by ponatinib in vitro has an extended effect **36A.** Results of CD107a release of NK cells detected by flow cytometry, with concentration adjustment after pretreatment with dasatinib of different concentrations (0, 50, 100 and 200nM). From the leftmost group to the rightmost group, the dasatinib concentration is decreased to 0, 50, 100 and 200nM, respectively. **36B.** Histogram of CD107a positive rate in Fig. 36A. The results show that the inhibition of NK cell activation function by ponatinib has an extended effect.

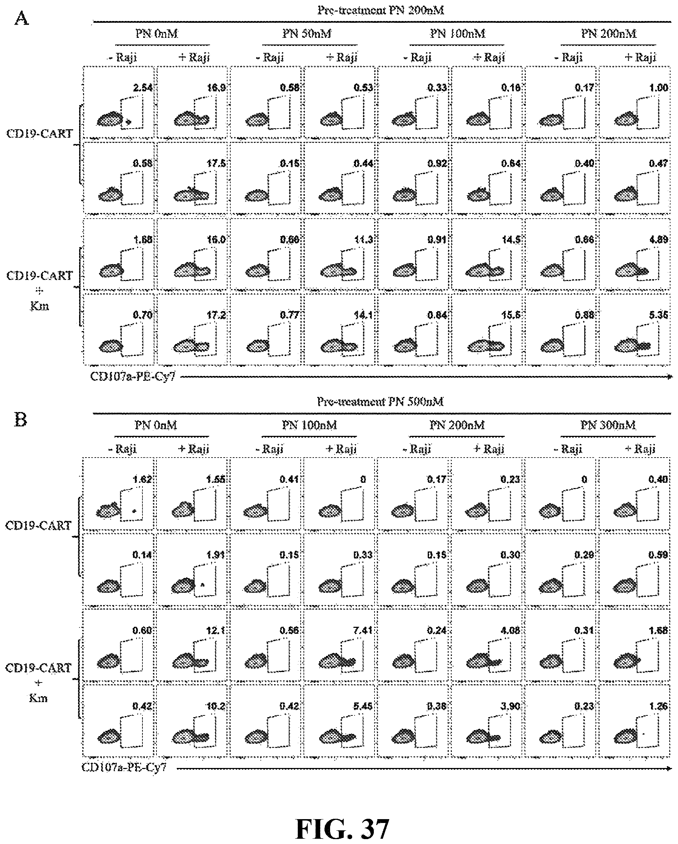

Fig. **37** shows the possibility of ponatinib as a combined drug and switch for universal CAR-T therapy. **37A.** After pretreatment with 200nM ponatinib overnight, and after co-incubation of CD19-UCART with target cells for 4hr with treatment with different concentrations (0, 50, 100 and 200nM) of ponatinib, the CD107a release results of CD19-UCART cells as detected by flow cytometry. **37B.** After pretreatment with 500nM ponatinib overnight, and after co-incubation of CD19-UCART with target cells for 4hr with treatment with different concentrations (0, 100, 200 and 300nM) of ponatinib, the CD107a release results of CD19-UCART cells as detected by flow cytometry.

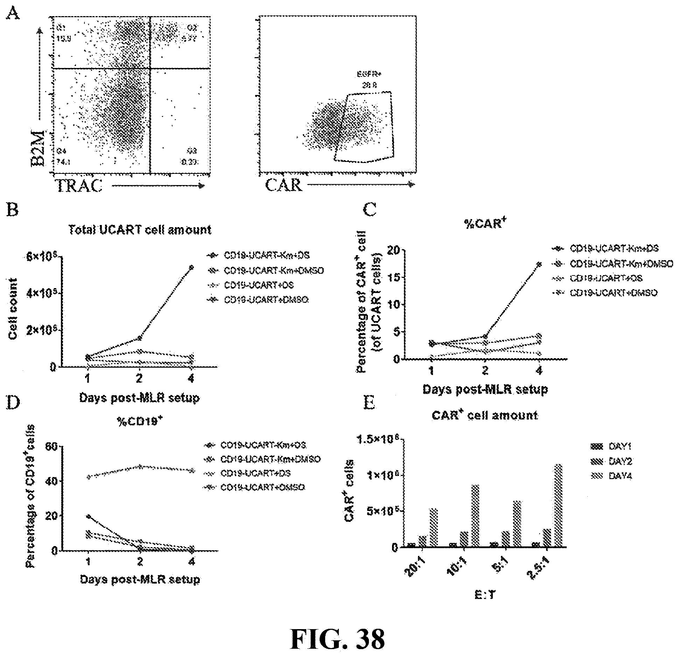

Fig. **38** shows that B2M-deficient CD19-UCAR-T cells with LCK-T316I mutation have an expansion advantage in MLR reactions as a mixture with allogeneic CD3-depleted PBMCs. **38A.** Schematic diagram of detection of B2M, TRAC knockout efficiency and CAR positive rate of prepared UCART cells. **38B.** Line graph of the total amount of UCART cells over time in the in-vitro mixed lymphocyte reaction experiment. **38C.** Line graph of CAR positive rate over time in the in-vitro mixed lymphocyte reaction experiment. **38D.** Line graph of the percentage of CD19 positive cells over time in the in-vitro mixed lymphocyte reaction experiment. **38E.** Histogram of the total amount of CAR positive cells over time with different effector-to-target ratios in the in-vitro mixed lymphocyte reaction experiment.

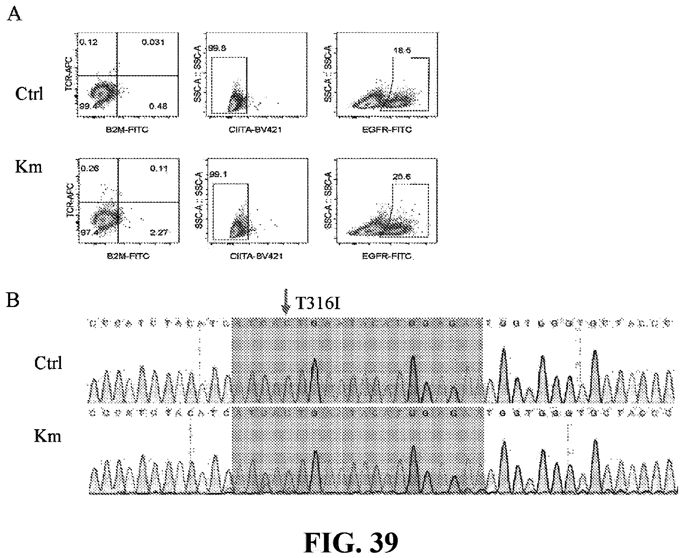

Fig. **39** shows the preparation parameters and detection results of CD19-UCART. **39A.** Results of detection of TRAC, B2M, and CUT A gene knockout efficiencies and detection of knockout rate and CAR positive rate by flow cytometry 5 days after infection of cells by lentivirus. **39B.** Detection of the efficiency of Km editing in CD19-UCART cells: the control group has no Km editing; the mutation efficiency of T316I in CD19-UCART cells in the sg16 (Km) group is 20%.

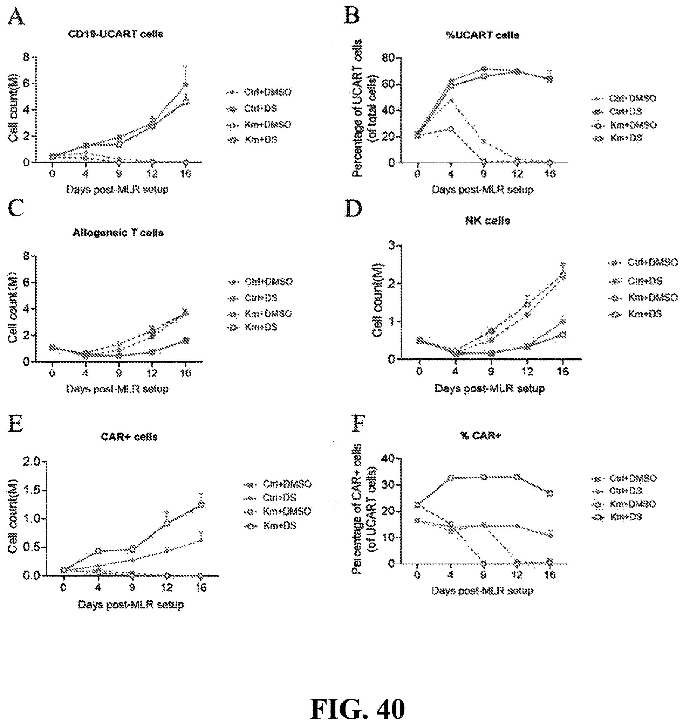

Fig. **40** shows that CD19-UCAR-T cells with LCK-T316I mutation have an expansion advantage in MLR reactions as a mixture with allogeneic PBMCs. **40A.** Schematic diagram of the total amount of CD19-UCAR-T cells at different time points in MLR reaction. **40B.** Schematic diagram of the percentage of CD19-UCAR-T cells in the overall cells at different time points in MLR reaction. **40C.** Schematic diagram of the total amount of allogeneic T (simulated host T) cells at different time points in MLR reaction. **40D.** Schematic diagram of the total amount of allogeneic NK (simulated host NK) cells in the overall cells at different time points in MLR reaction. **40E.** Schematic diagram of the total amount of cells expressing CAR molecules at different time points in MLR reaction. **40F.** Schematic diagram of the percentage of cells expressing CAR molecules in the overall cells at different time points in MLR reaction.

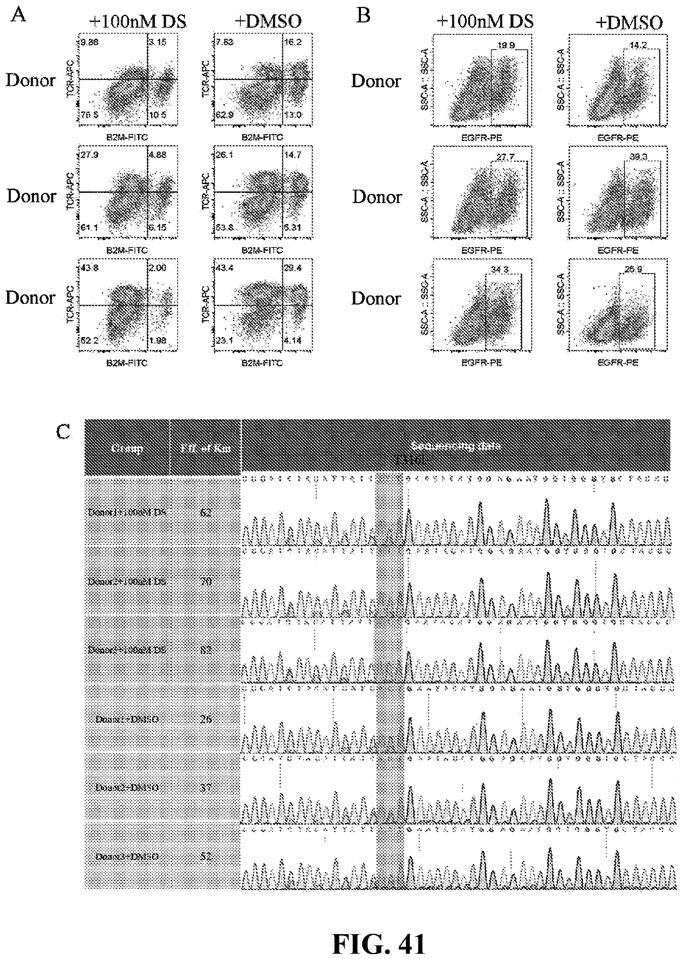

Fig. **41** shows the test results of optimizing the CD19-UCART preparation process parameters. **41A.** Detection of TRAC, B2M gene knockout efficiency. **41B.** The results of CAR positive rate detected by flow cytometry 5 days after infection of cells by lentivirus. **41C.** Detection of the efficiency of Km editing in CD19-UCART cells: the mutation efficiency of T316I in the experimental group was significantly higher than that in the control group.

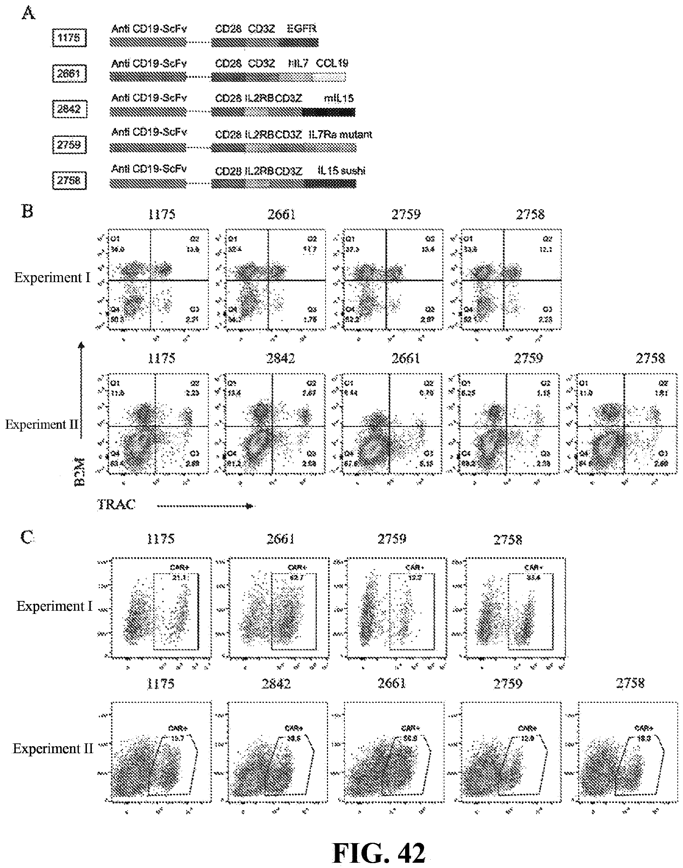

Fig. **42** shows the construction of a CAR carrying a third signal element and the results. **42A.** Schematic diagram of the structure of CD19-UCART plasmids containing various third signal structures. **42B.** The efficiency of electroporation knockout is shown by detecting the expression of TRAC and B2M. **42C.** The CAR+ percentage of UCART cells is detected by the binding of CD 19-antigen and Anti-CD 19 scFv on the CAR structure.

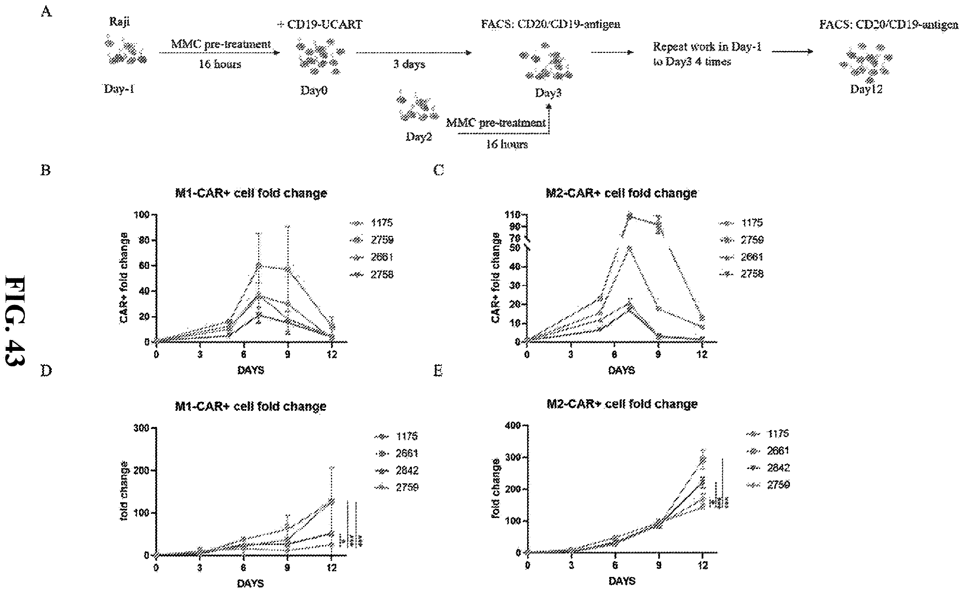

Fig. **43** shows the procedure and results of repeated antigen stimulation. **43A.** Schematic diagram of the operation procedure of the repeated antigen stimulation experiment. **43B-C.** In experiment I, CD19-UCART containing various third signal structures is co-cultured with Raji cells pretreated with mitomycin C at an effector-to-target ratio of 2:1 under M1 culture conditions (CTS basal T cell medium without supplementing any cytokines) (**43B**) and M2 culture conditions (CTS basal T cell medium + 200U/ml IL2) (**43C**). The number of CAR+ cells each time is obtained according to flow cytometry and cell counting, and then the expansion factor of CAR+ cells is obtained. **43D-E.** In experiment II, CD19-UCART containing various third signal structures is co-cultured with Raji cells pretreated with mitomycin C at an effector-to-target ratio of 2:1 under M1 culture conditions (CTS basal T cell medium without supplementing any cytokines) (**43D**) and M2 culture conditions (CTS basal T cell medium + 200U/ml IL2) (**43E**). The number of CAR+ cells each time is obtained according to flow cytometry and cell counting, and then the expansion factor of CAR+ cells is obtained.

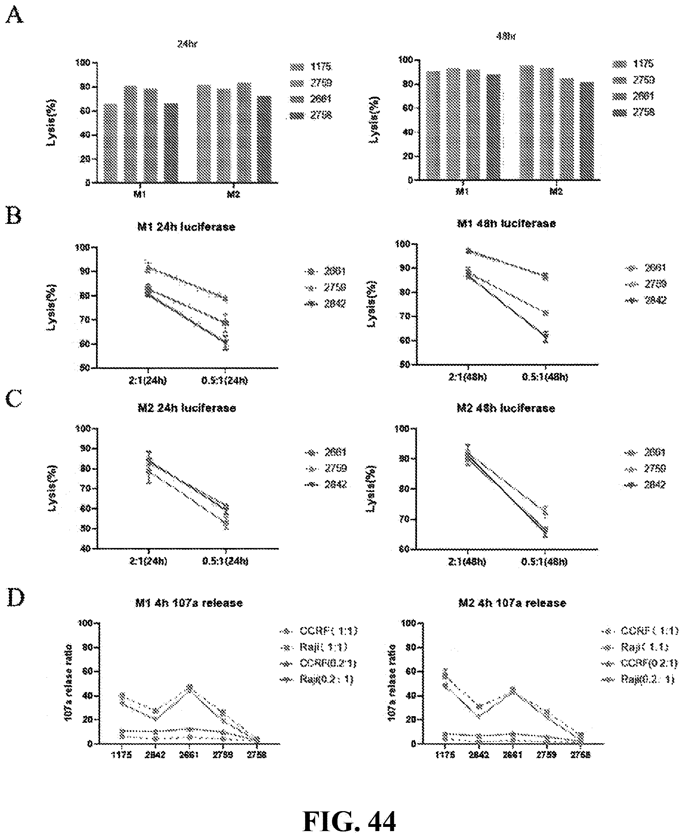

Fig. **44** shows the function detection result of CART with the third signal structure. **44A.** In experiment I, CD19-UCART containing various third signal structures is repeatedly stimulated with the antigen under M1 culture conditions (CTS basal T cell medium without supplementing any cytokines) and M2 culture conditions (CTS basal T cell medium + 200U/ml IL2), and the remaining CD19-UCART and Raji-luc cells are co-cultured at a ratio of 1:1 to carry out luciferase killing experiment. The luminosity of luciferase after co-culture is measured to calculate the rate of killing Raji-luc and draw a histogram. **44B-C.** In experiment II, CD19-UCART containing various third signal structures is repeatedly stimulated with the antigen under M1 culture conditions (CTS basal T cell medium without supplementing any cytokines) and M2 culture conditions (CTS basal T cell medium + 200U/ml IL2), and the remaining CD19-UCART and Raji-luc cells are co-cultured in ratios of 2:1 and 0.5:1 to carry out luciferase killing experiment. The luminosity of luciferase after co-culture was measured to calculate the rate of killing Raji-luc and draw a line graph. **44D.** In experiment II, CD19-UCART containing various third signal structures is repeatedly stimulated with the antigen under M1 culture conditions (CTS basal T cell medium without supplementing any cytokines) and M2 culture conditions (CTS basal T cell medium + 200U/ml IL2), and the remaining CD19-UCART and Raji, CCRF cells are co-cultured in ratios of 1:1 and 0.2:1 to detect 107a release. The 107a flow cytometry results after co-culture are measured to calculate the percentage of 107a released by UCART cells and draw a line graph.

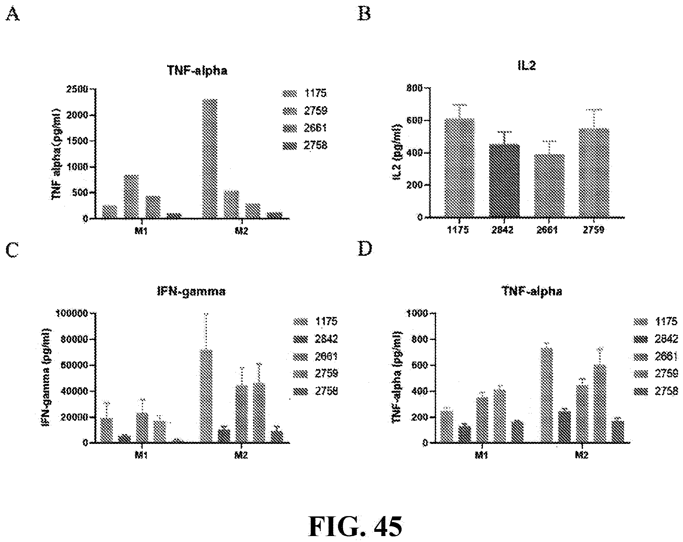

Fig. **45** shows the cytokine secretion results of CART carrying the third signal structure. **45A.** In experiment I, CD19-UCART containing various third signal structures is repeatedly stimulated with the antigen under M1 culture conditions (CTS basal T cell medium without supplementing any cytokines) and M2 culture conditions (CTS basal T cell medium + 200U/ml IL2), and the concentration of IL2 in the resulting culture medium supernatant is detected. **45B-D.** In experiment II, CD19-UCART containing various third signal structures is repeatedly stimulated with the antigen under M1 culture conditions (CTS basal T cell medium without supplementing any cytokines) and M2 culture conditions (CTS basal T cell medium + 200U/ml IL2), and the concentration of IL2 (**45B**), IFN-gamma (**45C**) and TNF-alpha (**45D**) in the resulting culture medium supernatant is detected.

### DETAILED DESCRIPTION OF THE INVENTION

Unless otherwise defined, all technical and scientific terms used herein have the meaning as commonly understood by one of ordinary skill in the art.

"Chimeric antigen receptor (CAR)" is an engineered membrane protein receptor molecule that confers a desired specificity to immune effector cells, such as the ability to bind to specific tumor antigens. A chimeric antigen receptor generally consists of an extracellular antigen-binding domain, a transmembrane domain, and an intracellular signaling domain. In some cases, the antigen-binding domain is an scFv sequence responsible for recognizing and binding to a specific antigen. An intracellular signaling domain usually includes an immunoreceptor tyrosine activation motif (ITAM), such as a signaling domain derived from a CD3z molecule, which is responsible for activating immune effector cells and producing cytotoxic effects. In addition, the chimeric antigen receptor may also comprise a signal peptide responsible for intracellular localization of the nascent protein at the amino terminus, and a hinge region between the antigen-binding domain and the transmembrane domain. In addition to signaling domain, intracellular signaling domain can also comprise costimulatory domains derived from, for example, 4-1BB or CD28 molecules.

"Bispecific chimeric antigen receptor" is intended to mean that the molecule includes at least two different antigen-binding sites in the extracellular antigen-binding domain, which respectively recognize and bind to different antigen molecules on target cells. For example, bispecific chimeric antigen receptors targeting both CD19 and BCMA are mentioned in the Examples herein.

"CAR cells" refers herein to cells that expresses CAR molecules on the cell surface. In most cases, the cells are immune cells, such as T cells, or NK cells. Accordingly, T cells expressing CAR are referred to herein as "CAR-T" or "CAR-T cells". In addition, when referring to CAR-T cells herein, unless otherwise specified, it refers not only to the cells directly modified by CAR, but also to the daughter cells produced after the proliferation of these cells in vitro or in vivo.

"Universal CAR-T cells (UCAR-T)" herein refer to such cells that are not limited to CAR-T cells infused into a specific patient. In the prior art, in order to prevent GvHD and the host's rejection of the graft, cells (such as T cells) are usually collected from the patient, modified with CAR and infused back into the patient. This method is not only time-consuming and expensive, but also in some cases cannot obtain a sufficient number of T cells of the patient for CAR modification. In contrast, the universal CAR-T cells here mean that these cells are suitable for allogeneic transplantation, the same batch of CAR-T cells can be used in different patients, and these universal CAR-T cells are usually not derived from these patients.

The present invention is based, at least in part, on novel preparation methods and use strategies of universal CAR-T discovered by the inventors.

In order to prepare universal CAR-T cells (UCAR-T), in our design of universal CAR-T cells, we try to find a simple and easy treatment protocol, which has the following two key points:
(1) with this protocol, the activation and cytotoxicity of host T cells and NK cells are effectively inhibited, and will not attack allogeneic universal CAR-T cells;
(2) with this protocol, the activation, killing and expansion functions of the universal CAR-T are not affected.

In order to suppress the cytotoxicity of host T cells and NK cells, inhibitors with inhibitory activity against both of them can be used, or inhibitors with inhibitory activity against one of them separately can be used, such as small molecule inhibitors or inhibitory antibody molecules. These inhibitors may result in a decrease in T cells and/or NK cells or may not affect their numbers. In this case, in order to achieve point (2), CAR-T cells can be engineered to make them insensitive to these inhibitors (especially to make the CAR signal transduction pathway tolerant to the inhibitory effects of these inhibitors), namely in the presence of these inhibitors, these CAR-T cells can still survive, be activated by target cells, and have target cell cytotoxicity (even if the overall cytotoxicity is reduced). There are a variety of ways that can be used to carry out such engineering of CAR-T cells, including but not limited to, introducing genetic mutations into CAR-T cells, where the mutated gene products (such as proteins or enzymes) are not sensitive to these inhibitors and have the function of the unmutated gene product; introducing foreign genes into CAR-T cells, where the proteins or enzymes produced by the expression of these foreign genes in CAR-T cells are not affected by these inhibitors and can replace the target molecules of these inhibitors to function intracellularly; introducing binding partners of these inhibitors (such as exogenous proteins capable of binding to these inhibitors, such as intrabodies) into CAR-T cells in order to neutralize these inhibitors; overexpressing the target molecules of these inhibitors (such as proteases) in CAR-T cells so as to counteract the effects of these inhibitors, etc.

Methods for introducing genetic mutations into cells are known in the art, including, but not limited to, DNA homologous recombination, site-specific cleavage with endonucleases (such as ZFN and TALEN), CRISPR-based gene editing techniques, and various base editors (such as CBE, ABE and their various improved variants).

Methods for introducing exogenous genes into cells are known in the art and include, but are not limited to, electroporation, gene gun, microinjection, liposome introduction, viral transduction (e.g., using retrovirus, lentivirus, various improved viral vectors, such as adenovirus and adeno-associated virus vectors).

Methods for overexpressing certain proteins or enzymes in cells may include, for example, increasing the copy number of the encoding gene thereof, enhancing the function of the promoter of the encoding gene thereof.

The introduction of mutations, exogenous genes, or overexpression in cells can be short-term or transient, or permanent (e.g., integration of a mutated gene or an exogenous gene into a host cell chromosome). The introduction can be in the form of DNA or RNA, for example, through lentiviral transduction so that the foreign gene can be expressed in the host cells for a long time, or through the introduction of mRNA so that the host cells can express some foreign proteins or enzymes in the short term.

The "mutant protein" used herein refers to a protein with a change in the amino acid sequence relative to the wild type, or a change in expression level compared to the expression level in normal cells, such as increased expression level (or overexpression).

Those skilled in the art should understand that the above engineering can be performed before, during, or after modifying the original cells into CAR-T cells. For example, T cells can be engineered as described above to make them insensitive to T cell and NK cell inhibitors, T cell banks are cultured and prepared, and then CAR modification is carried out according to various treatment needs, so as to obtain universal CAR-T cells for various therapeutic purposes (such as various cancers).

The "original cells" here refer to cells of interest that have undergo or are about to undergo CAR modification, such as stem cells, and immune cells, T cells, and NK cells at various developmental stages. Considering that one of the purposes of the present application is to prepare universal CAR-T cells, the source of these cells has nothing to do with the patient to be treated. Therefore, the source of these original cells is basically unlimited, for example, they can come from blood banks, and healthy volunteers.

In some embodiments, the above-mentioned inhibitor is a tyrosine kinase inhibitor. In some embodiments, these tyrosine kinases act on the signal transduction pathway of the CAR. In some embodiments, these tyrosine kinases act on both the TCR signal transduction pathway and the CAR signal transduction pathway. In some embodiments, the tyrosine kinase is LCK kinase. In some specific embodiments, the tyrosine kinase is LCK kinase and the inhibitor is dasatinib (DS) and/or ponatinib (PN).

In a specific embodiment, the engineering of LCK kinase involves the mutation of its T316 site, such as T316I, T316M, T316A and other mutations, as long as the mutation makes the mutated product insensitive to the above-mentioned inhibitors (such as dasatinib and ponatinib) while having the protease function before mutation. Preferably, the mutation is T316I.

As far as the T316I mutation is concerned, the present disclosure provides an ingenious way to achieve this mutation. The method involves the use of cytosine base editors used in conjunction with sgRNAs, achieves, on the Lck gene, the base pair C • G to T • A transition. This base pair transition produces the T316I mutation in its expression product LCK, while the cytosine base editor also causes some other base pairs C • G transitions near the target mutation site, but these other transitions happen to be nonsense mutations that do not produce amino acid changes in LCK. Those skilled in the art should understand that, although the preferred technical solution for forming T316I mutation is described in the specific Examples herein, it does not exclude other similar ways to form mutation at T316 site. For example, under the inspiration of the specific technical solution herein, gene editing technology can be used to form other mutations at the T316 site, or to change the specific sequence of the sgRNA (including length changes, target sequence changes), or even to generate multiple mutations (including T316 site) in the product LCK using these or other methods, as long as the formed product LCK can maintain its original function and is insensitive to the above inhibitors, then these changes should be included in the scope of the present invention.

In order to prevent CAR-T cells from attacking host normal cells (non-target cells), that is, GvHD, it may be considered to engineer the TCR-related genes on CAR-T cells to deactivate or reduce the activity of TCR, for example, by knocking out the TRAC gene encoding the α chain of the TCR receptor, and/or the TRBC gene encoding the β chain of the TCR receptor through gene editing or other methods.

Further, in order to prevent a few host T cells or NK cells from attacking CAR-T cells, it may be considered to reduce or avoid the expression of HLA-class I molecules on CAR-T molecules, for example, by knocking out the B2M gene encoding β2 microglobulin.

The universal CAR-T cells provided herein can be combined with cell activity inhibitors to treat patients (such as cancer patients). As already explained above, these cell activity inhibitors can be of various types, as long as they can make the immune system of the host (recipient of CAR-T cells) not cause the loss of the target cell cytotoxicity of the infused CAR-T cells. Generally, these inhibitors can prevent the host immune system (such as T cells, especially CD8+ T cells) from killing CAR-T cells. In this case, the infused CAR-T cells can be activated by their target cells to exert cytotoxic effects. At the same time, they can also proliferate in the host and exert long-term effects. Preferably, these inhibitors only suppress the function of the host's immune system without causing disruption of its function. In this case, when the inhibitor is cleared or the inhibitor is not administered, the host's immune system can restore its original function as soon as possible.

Therefore, in some embodiments, these inhibitors can be used as molecular switches to determine whether the universal CAR-T cells continue to perform their function. For example, the inhibitors can be administered to patients before the infusion of universal CAR-T cells to suppress or weaken the cytotoxicity of their immune system (especially T cells) on the universal CAR-T cells that are about to be infused. Next, universal CAR-T cells are infused to the patients, and then the inhibitors (same or different from previous inhibitors) continue to be regularly administered to patients as appropriate, so as to maintain the concentration of inhibitors in patients to a level that can continue to suppress the function of the patient's immune system. Since these infused universal CAR-T cells are engineered to be tolerant to the aforementioned inhibitors, they can recognize and kill their target cells (such as cancer cells). After the desired therapeutic effect (such as tumor regression or disappearance) is achieved, the administration of the inhibitor is stopped to allow the patient's own immune system to recover and clear the universal CAR-T cells in the body. During this process, the content and activity level of universal CAR-T in the patient as well as the disease status can be regularly detected to determine whether it is necessary to infuse universal CAR-T cells again. Using these inhibitors as molecular switches is also beneficial to drug safety. If the patient cannot tolerate the universal CAR-T cell therapy, the inhibitor can be stopped at any time so as to clear these universal CAR-T cells to avoid serious adverse reactions. In addition, by increasing the concentration of the inhibitor (for example, when ponatinib is used as the inhibitor, its concentration can be increased from 200nM to 500nM), the above-mentioned universal CAR-T cells that can tolerate the inhibitory effect of a certain dose of inhibitor become no longer tolerant to the action of the inhibitor and consequently cannot continue to proliferate and/or have cytotoxicity on target cells. Therefore, stopping the use of inhibitors or increasing the concentration of inhibitors can be used as a molecular switch to control the cytotoxicity of universal CAR-T cells in vivo.

As illustrated in the Examples below, in the case where dasatinib was used as an inhibitor, it can be administered to suppress or weaken the function of the patient's immune system at such a dose that the concentration of dasatinib in the patient is not lower than 10nM, or not lower than 25nM, or not lower than 30nM, or not lower than 50nM, or not lower than 100nM. In the case where ponatinib was used as an inhibitor, it can be administered at such a dose that the concentration of ponatinib in the patient is not lower than 100nM, or not lower than 200nM, or not lower than 300nM.

The universal CAR-T cells provided herein can also be used in combination with other cancer therapeutic agents, that is, the patient is administered with other cancer therapeutic agents during, before or after administration of the inhibitor and the universal CAR-T cells to the patient. These other cancer therapeutic agents may include chemotherapy drugs, radiotherapy drugs, other biological therapeutic agents such as antibody therapeutic agents or cell therapeutic agents.

"Patient" as used herein refers to a subject to be treated and may include any mammal such as cats, dogs, sheep, cows, mice, rats, rabbits, humans, non-human primates, and the like. In addition, a patient can be any individual who already has a disease, is at risk of developing a disease, or has been treated for it. Correspondingly, the preparation method of the universal CAR-T and treatment method provided herein can be used in humans and non-human mammals. For example, the safety and effectiveness of the universal CAR-T cells provided herein can be first verified in an animal model, and then used in human clinical research and treatment.

In a specific embodiment, we have designed the following universal CAR-T treatment strategy (in the following, dasatinib is used as the T cell activity inhibitor as an example): (1) dasatinib and universal CAR-T are used in combination, the activity of host T cells and NK cells is inhibited by dasatinib to prevent them from killing the universal CAR-T; (2) since dasatinib can also inhibit the activity of the universal CAR-T, the universal CAR-T is designed and engineered to make it tolerant to dasatinib; (3) the engineering of the universal CAR-T is carried out using the CRSIPR-Cas9-related cytosine base editor (CBE3) to make a point mutation on its Lck gene, resulting in T316I mutation of the LCK protein; and (4) the mutated UCAR-T T316I cells can show tolerance to dasatinib. Therefore, with the treatment with dasatinib, the host T cells and NK cells are inhibited and will not clear allogeneic cells, while UCAR-T can perform normal tumor cytotoxicity and expand normally; (5) In addition to editing Lck Genes aside, it still needs to edit the TRAC gene of CAR-T cells to avoid GvHD.

In the case where dasatinib effectively inhibits the activity of host T cells, the strategy mechanism of universal CAR-T is shown in Fig. 1. This strategy is designed based on the fact that dasatinib can effectively clear host T cells, and the main consideration is the clearance of UCAR-T by host T cells. Therefore, in order not to consider the possibility of clearance of UCAR-T by host NK cells, we did not knock out HLA-I molecules, directly avoiding the activation of host NK cells caused by HLA-class I molecule knockout.

As shown in Fig. 1, the leftmost column shows the reason why UCAR-T cells that are infused back into the host are cleared by host T cells in vivo and are difficult to expand under normal circumstances. The TCR of host T cells recognizes the mismatch of HLA-class I and II molecules on the surface of UCAR-T cells, mainly cytotoxic CD8-positive T cells recognize the mismatch of HLA-class I molecules, the intracellular tyrosine kinase LCK of host T cells autophosphorylates and then phosphorylates TCR and intracellular segment ITAMS signals of CD3, thereby activating T cells to clear UCAR-T. The middle column shows that after dasatinib is used in combination, it inhibits TCR activation of host T cells by inhibiting LCK autophosphorylation of host T cells. For universal CAR-T cells, dasatinib also inhibits the phosphorylation and activation of CAR molecules by inhibiting LCK autophosphorylation, and as a result, universal CAR-T cannot be effectively activated by tumor cells and expanded. The rightmost column shows the single-base mutation of LCK to the universal CAR-T, so that LCK^{m} can resist the binding of dasatinib and produce drug tolerance. Then, with treatment in combination with dasatinib, the edited LCK^{m} UCAR-T cells can be normally activated by tumor cells and expand effectively.

In the case where dasatinib effectively inhibits host NK cells, the strategy mechanism of universal CAR-T is shown in Fig. 2. This strategy is designed based on the fact that dasatinib can effectively clear host NK cells, and the main consideration is the clearance of UCAR-T by host NK cells. Therefore, in order not to consider the killing effect of host T cells (mainly CD8-positive cytotoxic T cells) on UCAR-T, we directly knocked out B2M gene to make UCAR-T not express HLA-class I molecules, thus directly avoiding the activation of host T cells caused by HLA-class I molecular mismatch.

As in Fig. 2, the leftmost column shows the principle that UCAR-T cells are cleared by NK cells in vivo after being infused back to the host under normal circumstances. Since HLA-class I molecules are the main ligands of NK inhibitory receptors, the knockout of B2M will make the NK inhibitory receptors unable to recognize the ligands, and LCK will autophosphorylate to activate NK and mediate the killing of UCAR-T. The middle column shows that after dasatinib is added, the activity of host NK and UCAR-T is inhibited, host NK does not clear UCAR-T cells, and UCAR-T cells do not have the function of killing tumors. The rightmost column shows that when UCAR-T undergoes LCK^{T316I} mutation, UCAR-T shows tolerance to binding to dasatinib, so with dasatinib treatment, host NK cell activity is inhibited, while the edited LCK^{m} UCAR-T cells can be normally activated by tumor cells and expand effectively.

### Function of LCK in T cell signal transduction

LCK is a member of the Src family of kinases. It generally binds to the intracellular region of the CD4 and CD8 co-receptor molecule of T cells and mediates the phosphorylation of the intracellular segment ITAMS of the T cell receptor molecule. Therefore, LCK is the most upstream tyrosine protein kinase in TCR signal transduction, mediating the transmission of the first signal after T cells receive antigen stimulation.

As shown in Fig. 3, when T cells receive stimulation from the antigen-MHC molecular complex of antigen presenting cells, the LCK protein bound to the intracellular segment of the co-receptor undergoes autophosphorylation and becomes activated and phosphorylated LCK, which then phosphorylates the ITAMS of the intracellular segment of TCR and CD3 molecules. At the same time, the phosphorylated SH2 domain recruits ZAP70, and LCK further phosphorylates ZAP70, which activates the downstream MEK/ERK pathway to participate in the activation of T cells. Therefore, LCK is the most upstream and crucial part in the activation pathway of T cells.

### Inhibition of T cell function by dasatinib

Inhibition of T cell function by dasatinib has been an accepted event so far. Two documents in 2008 respectively elaborated on how dasatinib inhibits T cell function [^{1,2}]. Stephen Blake et al. reported that dasatinib, as an inhibitor of Src/ABL kinase, can effectively inhibit many functions of normal human T lymphocytes in vitro, including that dasatinib can effectively block the transduction of TCR signal by binding to LCK, inhibit the activation, cytokine secretion and proliferation of T cells in vitro, but will not affect the viability of T cells [¹]. Andrew E. Schade et al. reported similar experimental results and believed that dasatinib inhibits TCR-mediated signal transduction, cell proliferation, cytokine secretion, and cellular responses in vivo by inhibiting LCK phosphorylation. But this inhibition is reversible, when dasatinib is withdrawn, the function of T cells can be restored [²].

### Inhibition of CAR-T cell function by dasatinib

Since dasatinib can generally inhibit the phosphorylation of LCK to inhibit the function of TCR, will the signal transduction of CAR-T be inhibited by dasatinib? Two documents in 2019 respectively elaborated on how dasatinib inhibits CAR-T cell function [^{3,4}]. Evan W. Weber et al. reported that dasatinib is a potential, rapid and reversible inhibitor of CAR-T cell function, which can inhibit the proliferation, cytokine secretion and tumoricidal activity of CAR-T cells in vivo [³]. Katrin Mestermann et al. reported that dasatinib can bind to LCK and inhibit the phosphorylation of CD3z and ZAP70, and therefore, dasatinib can inhibit the activation of CD28_CD3z or 4-1BB_CD3z in the molecular structure of CAR to inhibit the function of CAR molecules. Also, dasatinib will induce the functional resting state of CD8⁺ and CD4⁺ CAR-T, which can last for several days without affecting the viability of T cells. The data show that dasatinib can inhibit the secretion of cytokines and proliferation of CAR-T cells in vivo and in vitro, and this inhibition is reversible [⁴].

### Inhibition of NK cell function by dasatinib

In addition to its inhibitory effect on T cells and CAR-T cells, dasatinib can also inhibit the degranulation and cytokine release of primary human NK cells [⁵].

### Relationship between LCK mutation and tolerance to dasatinib

It has been documented that the LCK-T316M mutation can show tolerance to dasatinib [⁶], the principle of which is that the T316 site is a key gatekeeper amino acid of the LCK kinase, and the T316M mutation greatly changes the structure of LCK. As shown in Fig. 4, when the site 316 is T, dasatinib can bind to the ATP-binding region of LCK and inhibit the autophosphorylation of LCK, while when the site 316 is mutated to M, dasatinib is blocked outside the gate and cannot bind to LCK-T316M.

As a small molecule inhibitor targeting ABL kinase, dasatinib has many analogues, such as imatinib and nilotinib. Among them, the most studied is the relationship between ABL mutation and tolerance to imatinib. The most common ABL mutations that can cause tolerance to imatinib are T315I, T315A, etc. [^{7,8,9}]. We found that the activation domains are very similar between ABL kinase and LCK kinase, and as shown in Fig. 5, the amino acid sequences before and after ABL-T315 and LCK-T316 are highly conserved. Therefore, we speculated that LCK-T316I and LCK-T316A may also be tolerant to dasatinib. Therefore, we can design subsequent LCK mutations from the three directions of T316M/A/I.

### Design protocol to induce LCK mutations on T cells

CRISPR-Cas9, as one of the commonly used gene editing tools, can specifically and efficiently cleave the genome as mediated by single-stranded targeting RNA. Therefore, CRISPR-Cas9-related gene editing is very suitable for the induction of the LCK-T316 point mutation. We have designed the following two sets of CRISPR-related technical solutions, which can realize the mutation of the LCK-T316 site on T cells.

### A strategy for inducing LCK-T316M/I/A mutations based on CRISPR-Cas9-mediated homologous recombination repair

CRISPR-Cas9-mediated homologous recombination repair is a very effective technical means to achieve base/amino acid point mutations. Its principle is that after RNP is formed from Cas9 protein and sgRNA, it can be targeted by the sgRNA to a specific position in the genome, and cleaved at the first 3-4nt position of the PAM sequence to induce double-stranded DNA to break. At this time, the introduction of homologous recombination carrying the LCK-T316 site mutation can enable the cells to repair with the template sequence, so as to obtain cells with endogenous LCK-T316 mutation. The template for this homologous recombination can be double-stranded DNA or single-stranded DNA. In the point mutation induction system, the efficiency of homologous recombination mediated by single-stranded DNA template is the highest.

Therefore, we initially designed 16 sgRNAs suitable for the Cas9 system with a length of 100bp near the LCK-T316 site. As shown in Fig. 6, only three cleavage sites of sg8, 9, and 12 near T316 are 7-12nt away from the mutation site, and the cleavage sites of other sgRNAs are too far away from the mutation site, and the efficiency of homologous recombination will be obviously reduced. Therefore, we selected three sgRNAs, sg8, 9 and 12, to evaluate their cleavage efficiency and off-target situation. The specific sequences of the three sgRNAs are as set forth in SEQ ID NOs: 16, 17 and 18, and the corresponding ssDNA-induced T316I mutation sequences are as set forth in SEQ ID NOs: 19-21, the corresponding ssDNA-induced T316M mutation sequences are as set forth in SEQ ID NOs: 22-24, and the corresponding ssDNA-induced T316A mutation sequences are as set forth in SEQ ID NOs: 25-27.

The off-target situation is shown in Fig. 7, there are 3, 2 and 3 MM2s for sg8, 9 and 12, respectively. The number of MM2 indicates how many sites the whole genome has and only 2 mismatches with the sgRNA. In general, when MM0, MM1, and MM2 occur, special attention needs to be paid to the off-target of this sgRNA. Therefore, we carefully checked the off-target sites of sg8, 9 and 12, and found that sg8 is very likely to be off-targeted to the exon regions of HCK and PDGFRB genes, sg9 is very likely to be off-targeted to the exon regions of HCK and FYN genes, and sg12 is very likely to be off-targeted to the exon region of the DDR2 gene. In these cases of off-target, the Cas9 protein can cleave the off-target region, thereby knocking out related genes, resulting in a great off-target risk. Therefore, although this protocol can induce the mutation of T316 to multiple amino acids such as M/I/A, we did not choose this protocol in the end.

The sequence of LCK-T316I protein is set forth in SEQ ID NO: 9, the sequence of LCK-T316A protein is set forth in SEQ ID NO: 10, and the sequence of LCK-T316M protein is set forth in SEQ ID NO: 11.

Another method to induce LCK-T316M/I/A mutations by CRISPR-Cas9-mediated homologous recombination is by cleavage with two sgRNAs plus repair with a single-stranded ssDNA template. Induction of the T316I/M/A mutation can also be achieved by combining sgRNA1 (SEQ ID NO: 14) with sgRNA11 (SEQ ID NO: 15), or sgRNA1 with sgRNA12 (SEQ ID NO: 18). Among them, the T316I/M/A mutant ssDNA template sequences required for combination of sgRNA1 and sgRNA11 are set forth in SEQ ID NOs: 28-30, respectively, and the T316I/M/A mutation ssDNA template sequences required for combination of sgRNA and sgRNA12 are set forth in SEQ ID NOs: 31- 33, respectively.

### Strategy to induce LCK-T316I mutation by CRISPR-Cas9-based cytosine base editor CBE3

Another tool capable of inducing LCK-T316 mutation is cytosine base editor based on CRISPR-Cas9 technique. The principle of CBE3 (cytosine base editor 3) is the fusion expression of Cas9n protein and APOBEC-3A, a protein that induces cytosine deamination (A3A-CBE3) to induce cytosine deamination to mutate when Cas9n cleaves the single strand of the target site. Cas9n makes mutation D10A in the RuvC1 domain of the Cas9 protein, making it a Cas9-nickase protein (Cas9n), which only retains the enzymatic activity of the HNH domain. Cas9n does not cause DNA double-strand break, but can only cleave DNA single strands complementary to sgRNA on the genome, thereby inducing base mismatch repair (BER). At this time, APOBEC-3A can induce the deamination of cytosine on the other DNA single strand to form uracil, and finally promote the mutation of cytosine to thymine (C->T mutation) in the presence of UGI protein.

The inventor cloned the A3A-CBE3 gene of the Escherichia coli expression system, purified and expressed the functional A3A-CBE3 protein through Escherichia coli, the protein sequence of which is set forth in SEQ ID NO: 34.

As shown in Fig. 8, we found two sgRNAs, sg12 and sg16, that conform to cytosine base editing from the sgRNAs near the LCK-T316 site. When T316 (ACT) is base-edited, its second codon cytosine (C) is directionally mutated to thymine (T), which can induce the formation of I316 (ATT).

Compared with the Cas9-mediated homologous recombination protocol, the advantages of this system are: (1) since the Cas9n protein is used in the CBE3 system, it will not cleave the double-stranded DNA of the genome, so the risk due to sgRNA off-target in this system is extremely low; (2) no template ssDNA is required, and the RNP formed by direct electroporation of CBE3 protein and sgRNA can realize gene editing; (3) the most important thing is that although the current CBE3 base editing is not yet accurate enough to achieve the site-specific targeting of a single base within the sgRNA coverage, near the LCK-T316 site, the mutation of any cytosine covered by the sgRNA except the T316 site will not cause amino acid mutations, that is, the CBE3 strategy only results in single amino acid mutation T316I.

This strategy still has two issues to be verified:
(1) Whether the designed two sgRNAs, sg12 and sg16, can efficiently induce LCK-T316I mutation;
(2) Whether the LCK-T316I mutation can cause tolerance to dasatinib.

Based on this, as shown in Table 1, we optimized the lengths of sg12 and sg16, and designed a total of 7 sgRNAs for subsequent mutation induction and functional verification.

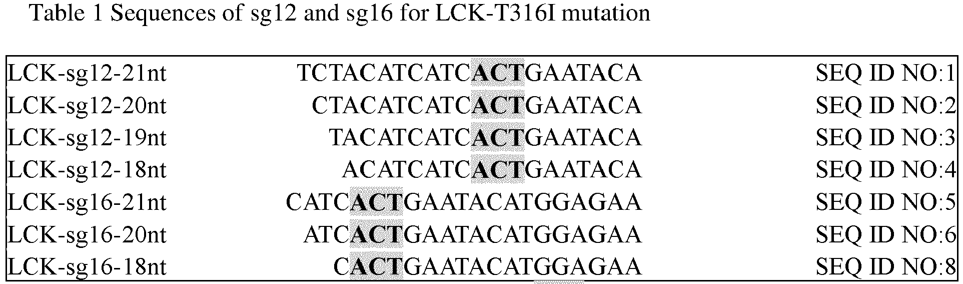

The inventors also designed LCK-sg16-19nt (TCACTGAATACATGGAGAA, SEQ ID NO: 7) as the sgRNA for LCK-T316I mutation, but did not verify it by experiments. It is theoretically deduced that the sgRNA of 19nt in length has functions similar or identical to those of 18nt, 20nt and 21nt.

In addition, the inventors also designed two suitable sa-sgRNAs for saCas9-induced base editing, namely LCK-saCas9-sgRNA1 and sgRNA2, whose sequences are set forth in SEQ ID NOs: 12 and 13.

### Functional validation of sgRNA for induction of LCK-T316I mutation

### sgRNA16 can efficiently induce LCK-T316I mutation

We isolated CD3-positive T cells from cryopreserved PBMCs, activated the cells with CD3/CD28 DynaBeads for 48 hrs and then used them in electroporation of the RNP complex of CBE3 protein and sgRNA. T cell genomes were extracted 96 h after editing, and detected for LCK gene editing efficiency by PCR and Sanger sequencing.

As specifically described in the Examples below, for the T cells in the group electroporated with only CBE3 protein, no base mutation occurs in the Lck gene region of its genome, while all the RNPs formed by LCK-sgRNA16 and CBE3 proteins of different lengths can cause T316I mutation with a mutation efficiency of about 50%. Meanwhile, the first four cytosines at the T316 site also undergo C->T mutations, but none of them causes amino acid changes. Therefore, in T cells, these three LCK-sgRNA16 with different lengths can all induce LCK-T316I mutation efficiently. In order to reduce the impact of sgRNA off-target on editing, we prefer to use 21nt sgRNA16 for subsequent experiments.

### sgRNA12 can not induce the LCK-T316I mutation

Similar to the above gene editing experiment, we also tested the induction efficiency of sgRNA12 on T cell LCK-T316 mutation. Compared with the control group CBE3, LCK-sgRNA12 of different lengths basically did not edit the cytosine at the T316 site, and induced about 50% of C->T mutations on the four cytosines before the T316 site, but still failed to cause any changes in amino acids. Therefore, LCK-sgRNA12 cannot effectively induce LCK-T316I mutation, but it can be used as a negative control for sgRNA16, so we chose 21nt LCK-sgRNA16 as a control for subsequent experiments.

### Validation of the relationship between LCK-T316I mutation and inhibition of T/CAR-T signal activation by dasatinib

### Verification of the functions of dasatinib by preliminary experiments to determine that dasatinib can efficiently inhibit the activation of T cell TCR and the translocation of CD107a in NK cells

In order to verify the inhibition of TCR signal activation of T cells by dasatinib, we pretreated T cells with dasatinib or DMSO for 5hr, then activated T cells with CD3/CD28 DynaBeads for 24hr, and then detected the expression of activation molecules CD25, CD69 and 4-1BB by flow cytometry. The results show that in the DMSO control group, after 24hr of magnetic bead activation, the activation molecule CD25 was significantly up-regulated, and the CD69-positive or 4-1BB-positive cells were significantly grouped. However, after treatment with 100nM/1000nM dasatinib, there was no CD69/4-1BB-positive activated cell population after activation with CD3/CD28, suggesting that 100nM dasatinib can effectively inhibit the activation of TCR signaling.

Next, in order to verify the inhibition of NK cell activation by dasatinib, NK cells isolated from human PBMCs were cultured and expanded in vitro. The cells were treated with different concentrations of dasatinib for 24 h, one group was not activated, and the other group was stimulated with target cells K562 for NK activation for 5hr before CD107a detection, and then the CD107a translocation of NK cells was uniformly detected. The results show that for the NK cells in the non-activated group, after treatment with dasatinib, the basal CD107a translocation was also reduced to the baseline of 2%. After activation by adding K562 target cells, the CD107a translocation in the untreated group was 64.5%, while that in the treated group was reduced to the baseline of 2%, suggesting that 100nM dasatinib is sufficient to inhibit the translocation of CD107a in NK cells and inhibit the activation of NK cells.

### LCK-T316I can make T cells tolerant to the inhibition of TCR activation by dasatinib

In order to verify the tolerance of the LCK-T316I mutation to the function of dasatinib, we performed the following experiments on LCK-edited T cells. Three different treatments were set for each group of edited cells and MockT cells: in the first group, dasatinib was not added, and the cells were not activated with CD3/CD28 magnetic beads; in the second group, dasatinib was not added, but the cells underwent TCR activation with CD3/CD28 magnetic beads for 24hr; in the third group, the cells were pretreated with 100nM dasatinib for 5hr, and then CD3/CD28 magnetic beads were added for TCR activation for 24hr. The effect of gene editing on the inhibition of TCR activation by dasatinib was observed.

The results show that in the control group, the percentage of CD25/CD69 positive cells in the group with 100nM dasatinib was significantly lower than that in the activation group, indicating that dasatinib inhibits the activation of TCR by CD3/CD28. Meanwhile, in the sg16 editing group, there was still a high percentage of CD25/CD69 positive cells in the group with dasatinib, and the same result could be obtained when 4-1BB was used as the activation marker. Therefore, a part of cells after sg16 editing could still be activated to become CD69/4-1BB positive after dasatinib treatment, which proves that they can tolerate the inhibition of T cell activation by dasatinib.

We further statistically analyzed the percentages of CD25, CD69, and 4-1BB single-positive cells in the control group, sg12 editing group, and sg16 editing group, and found that in the sg16 editing group, with the treatment with dasatinib and CD3/CD28 magnetic beads, the expression percentages of the three activation markers were significantly higher than those in the control group and the sg12 editing group. It is further suggested that T cells in the sg16 editing group (-50% LCK-T316I), which show tolerance to dasatinib, can be activated by CD3/CD28.

### LCK-T316I makes anti-CD19-CAR-T cells tolerant to the inhibition of CD107a translocation by dasatinib

In order to verify that the LCK-T316I mutation can not only make T cells tolerant to the inhibition of TCR activation by dasatinib, but also make CAR-T cells tolerant to the inhibition of CD107a translocation by dasatinib during CAR activation, we designed the following experiment. First, we edited anti-CD19 CAR-T using CBE3 protein, CBE3+sg12 and CBE3+sg16. After 72 h of stabilization after editing, we pre-treated each group of CAR-T cells with 100nM dasatinib for 12hr, followed by stimulation with different target cells to activate CAR signals and mediate the release of CD107a in the target cells killed by CAR-T. 5hr before detection, monensin and anti-CD107a antibody were added, and staining for CD8 and CAR positive was performed 5hr later to detect the enrichment of CD107a translocation of CD8 positive CAR positive cells.

The sequence of the anti-CD19-CAR molecule used here is the same as that used in the subsequent examples, and the specific sequence is set forth in SEQ ID NOs: 35-56. The overall structure of this CAR molecule is a second-generation CAR molecule, which consists of anti-CD19 scFv (SEQ ID NOs: 35-38), plus a linker region (SEQ ID NOs: 39-40), plus a CD8a hinge region (SEQ ID NOs: 41-42), plus a CD8a transmembrane domain (SEQ ID NOs: 43-44), plus a CD28 intracellular domain (SEQ ID NOs: 45-46), plus a CD3z intracellular signaling transduction domain (SEQ ID NOs: 47-48), plus a T2A sequence (SEQ ID NOs: 49-50), plus a CSF2RA signal (SEQ ID NOs: 51-52), plus a tEGFR signal (SEQ ID NOs: 53-54). The total sequence of the final anti-CD19-CD28z-CAR is set forth in SEQ ID NOs: 55-56.

The results show that regardless of whether anti-CD19-CAR-T cells were edited or not, in the DMSO control group, the negative target cell K562 could not activate CAR-T for effective CD107a translocation, while the positive target cell Nalm6 could fully activate CAR-T to release CD107a with a positive rate of above 70%. With the 100nM dasatinib treatment, the negative control group K562 hardly had any CD107a translocation enrichment, while under the positive target cell Nalm6 stimulation, only the sg16-LCK-T316I editing group showed 50% positive CD107a translocation, suggesting that the LCK-T316I mutation can indeed make anti-CD19-CAR-T cells tolerant to the inhibition of CD107a translocation by dasatinib, and this tolerance has significant differences.

### LCK-T316I makes anti-BCMA-CAR-T cells tolerant to the inhibition of CD107a translocation by dasatinib

In order to verify that in addition to anti-CD19 CAR-T, LCK-T316I mutation can make other CAR-T cells tolerant to the inhibition of CD107a translocation by dasatinib, we performed three groups of editing of LCK on anti-BCMA CAR-T, i.e. CBE3, CBE3+LCK-sg12 and CBE3+LCK-sg16, and the experimental procedure is exactly the same as that described above. There were three groups of target cells used to stimulate anti-BCMA CAR-T, namely K562 negative target cells, U266-B1 positive target cells and RPMI-8226 positive target cells. The results show that in the DMSO-treated control group, the three groups of edited anti-BCMA CAR-T all had higher levels of CD107a translocation under the stimulation with positive target cells, while with the treatment with 100nM dasatinib, only the LCK-T316I mutation group (CBE3+sg16 group) showed tolerance to dasatinib, with 39.6% and 44.7% positive CD107a release upon activation with the two positive target cells, respectively. Meanwhile, there was a significant difference in this degree of CD107a release compared to other edited controls, suggesting that the LCK-T316I mutation can make anti-BCMA-CAR-T cells tolerant to the inhibition of CD107a translocation by dasatinib.

### LCK-T316I makes anti-CD19/BCMA dual-target UCAR-T cells tolerant to the inhibition of CD107a translocation by dasatinib

In addition, basically consistent with the above experiment, we further verified that the LCK-T316I mutation makes the CAR-T cells tolerant to the inhibition of CD107a translocation by dasatinib in anti-CD19 and BCMA dual-target UCAR-T cells (with TRAC knockout). The results show that under normal circumstances, the negative target cell K562 basically do not activate dual UCAR-T, and the two positive target cells U266 and Raji can activate dual UCAR-T. With the treatment with 100nM dasatinib, only the sg16 editing group could show tolerance to the inhibition of the translocation of CD107a by dasatinib under target cell activation, which is completely consistent with the previous results.

### Validation of the relationship between LCK-T316I mutation and inhibition of CAR-T cytotoxicity by dasatinib

### LCK-T316I makes anti-BCMA CAR-T cells tolerant to the inhibition of CAR-T cytotoxicity by dasatinib

As mentioned above, the mutation of LCK-T316I can make T cells tolerant to the inhibition of TCR activation by dasatinib, and can also make CAR-T cells tolerant to the inhibition of CAR activation and CD107a translocation by dasatinib. We wanted to further explore whether this tolerance can be reflected in the function of T cells, so we designed the following experiments to explore the relationship between the LCK-T316I mutation and the inhibition of CAR-T cytotoxicity by dasatinib.

We performed three different editing treatments on anti-BCMA CAR-T: CBE3 protein, CBE3+sg12 and CBE3+sg16. After the editing was stabilized for 72hr, we pretreated each group of CAR-T cells with 100nM dasatinib for 12hr. Next, the cells were co-incubated with different luciferase-positive target cells (luciferase+) at an effector-to-target ratio of 1:1 for 24hr. After the incubation, the cytotoxicity of CAR-T cells on target cells was evaluated by detecting the activity of luciferase. The lower the luciferase value, the fewer surviving target cells and the stronger the cytotoxicity of CAR-T. The results show that in the DMSO control group, anti-BCMA CAR-T could reduce the luciferase value of RPMI-8226 positive target cells, but did not affect the luciferase value of negative target cells Nalm6, proving the normal cytotoxicity of anti-BCMA CAR-T. With the treatment with 100nM dasatinib and under the stimulation with positive target cells RPMI-8226, the luciferase value in the CBE3 and CBE3+sg12-LCK unediting groups was significantly higher than that in the DMSO group, proving that with the treatment with dasatinib, the cytotoxicity of anti-BCMA CAR-T was significantly inhibited. The luciferase value in the CBE+sg16-LCK editing group was significantly lower than that in the CBE3 and CBE3+sg12-LCK unediting groups, proving that the anti-BCMA CAR-T after the LCK-T316I mutation developed tolerance to inhibition of CART cytotoxicity by dasatinib.

In addition, we repeatedly edited another batch of anti-BCMA CAR-T cells, and obtained results consistent with the above experiment. In this replicate, we used two other negative target cells, K562-luciferase and Raji-luciferase. Luciferase results show that in the DMSO control group, all the edited anti-BCMA CAR-T cells would not kill K562 and Raji negative target cells, but would target RPMI-8226 positive target cells, with their luciferase value basically decreased to the baseline, suggesting that anti-BCMA CAR-T has normal specific cytotoxicity. Subsequently, with 100 nM dasatinib treatment, high luciferase values could still be detected in the CBE3 and CBE3+sg12 groups in the positive target cell groups, suggesting that the cytotoxicity of CAR-T was still inhibited. The luciferase value of CBE3+sg16-LCK-T316I editing group decreased significantly, which once again demonstrates the tolerance of LCK-T316I mutation to the inhibition of CAR-T cytotoxicity by dasatinib.

The CAR molecule sequence of the anti-BCMA-CAR-T cells used here is the same as that used in the subsequent examples, and the specific sequence is set forth in SEQ ID NOs: 77-78.

### Validation of the relationship between LCK-T316I mutation and inhibition of T/CAR-T cell proliferation by dasatinib

### LCK-T316I makes anti-BCMA CAR-T cells tolerant to the inhibition of cell proliferation by dasatinib

Since LCK-T316I can produce tolerance to the functions of inhibiting TCR/CAR activation and cytotoxicity of CAR-T cell of dasatinib, we would like to further explore the inhibition of T/CAR-T proliferation in vitro by dasatinib reported in the literature to see whether it can be resisted in CAR-T cells with LCK-T316I mutation. Therefore, we designed the following experiments to verify CAR-T proliferation in vitro. Firstly, the anti-BCMA CAR-T cells were edited with CBE3, CBE3+sg12 and CBE3+sg16. After the editing was stabilized for 72hr, the target cells U266 were added at an effector-to-target ratio of 10:1 for stimulation every 3 days, and at the same time the total number of cells and CAR positive rate were detected once every three days. During the experiment, the cells were divided into two groups treated with DMSO and 100nM dasatinib. As shown in Fig. 9, the results show that although the CAR-T cells in the DMSO group did not well expand, in the dasatinib treatment group, there was a difference in the CAR positive rate between CBE3+sg16 and the other two groups on Day12, and the total number of cells in this whole group of cells was also the largest. It preliminarily suggests that LCK-T316I can make anti-BCMA CAR-T cells tolerant to the inhibition of cell proliferation by dasatinib.

### Preliminary brief data summary

We have found a method for site-directed mutation of LCK-T316I, that is, cytosine base editor CBE3 in combination with LCK-sgRNA16 to achieve efficient T cell LCK-T316I mutation through electroporation;

LCK-T316I mutation can resist many functions of dasatinib on T cells:
Dasatinib can efficiently inhibit the activation of TCR signals by CD3/CD28;
Dasatinib can efficiently inhibit the activation of CAR-T cells by target cells;
LCK^{T316I} makes T cells tolerant to the inhibition of TCR activation by dasatinib;
LCK^{T316I} makes CAR-T cells tolerant to the inhibition of CAR activation by dasatinib;
LCK^{T316I} makes anti-CD19-CAR-T cells tolerant to the inhibition of CD107a translocation by dasatinib;
LCK^{T316I} makes anti-BCMA-CAR-T cells tolerant to the inhibition of CD107a translocation by dasatinib;
LCK^{T316I} makes anti-CD19/BCMA dual-target CAR-T cells tolerant to the inhibition of CD107a translocation by dasatinib;
LCK^{T316I} makes CAR-T cells tolerant to the inhibition of CAR-T cytotoxicity by dasatinib;
LCK^{T316I} makes CAR-T cells tolerant to the inhibition of cell proliferation by dasatinib.

In addition, our further research found that:
dasatinib at a concentration of 25nM can effectively inhibit the activation of T cells in vitro;
The inhibition of T cell activation function by dasatinib in vitro has an extended effect, and CD5-UCART with LCK^{T316I} mutation can tolerate this inhibition;
Dasatinib can efficiently inhibit the activation of NK cells;
Dasatinib at a concentration of 30nM can effectively inhibit the activation of NK cells in vitro;
The inhibition of NK cell activation function by dasatinib in vitro has an extended effect;
The strategy of combining LCK^{T316I} mutation with dasatinib gives CD5-UCART advantages in expansion and survival in the in vitro mixed lymphocyte reaction at an effector-to-target ratio of 5:1;
The strategy of combining LCK^{T316I} mutation with dasatinib gives CD5-UCART advantages in expansion and survival in the in vitro mixed lymphocyte reaction at an effector-to-target ratio of 50-100:1;
Ponatinib (PN) at a concentration of 200nM can effectively inhibit the activation of T cells in vitro, and LCK^{T316I} mutation can tolerate this inhibition.
Ponatinib at a concentration of 200nM can effectively inhibit the activation of NK cells in vitro;
The inhibition of T cell activation function by ponatinib in vitro has an extended effect;
The inhibition of NK cell activation function by ponatinib in vitro has an extended effect;
The possibility of ponatinib as a combined drug and switch for universal CAR-T therapy of the present invention is verified;
It is proved that in the strategy of the present invention, the universal CAR-T cells with B2M knockout can resist the cytotoxicity of NK cells.

Finally, by LCK-T316I editing of the universal CAR-T, combined with dasatinib/ponatinib treatment, the activation and cytotoxicity of host T and NK cells are inhibited, so that the UCAR-T with LCK^{T316I} could not be cleared by the immune system of the host, and the cells have normal tumor-killing function, and can effectively expand and function in the body.

The present invention will be further described below through specific examples.

### Example 1: Methods and materials

### Methods

### 1.1 Sorting and activation of CD3+ T cells

Frozen healthy donor PBMCs were resuscitated, totally 1.0×10⁸ cells per tube, thawed quickly and resuspended in 8mL of preheated Rinsing buffer. A small amount of cell suspension was taken for cell counting. The PBMC suspension was centrifuged at 400g for 10 min, with an increasing speed setting of 8 and a decreasing speed setting of 8 (hereinafter referred to as ↑8 ↓8). After centrifugation, the supernatant was discarded, and 20ul/10⁷ CD3 microbeads were added. The system was mixed well, and then incubated in a 4°C refrigerator for 20 min, during which the tube wall was flicked several times every 10 min to avoid cell sedimentation. After the incubation, the cells were rinsed with Rinsing buffer once, then centrifuged (400g 10min ↑8 ↓8), and then resuspended with 500µl of Rinsing buffer. At the same time, an LS sorting column was placed on a Miltenyi magnetic sorting rack, rinsed with 2ml of Rinsing buffer once, to which 500µl of cell suspension was added. After the cell suspension was added dropwise, 2ml of Rinsing buffer was added twice onto the LS column. The cells of interest were rinsed out from the LS column with 5mL of Rinsing buffer and collected, and counted after appropriate dilution. About 1×10⁵ cells were taken and the purity of the sorted T cells was determined by flow cytometry. Then, the cell suspension was centrifuged at 300g for 10 min, the cell density was adjusted to 1×10⁶ with fresh T cell culture medium, and anti-CD3/-CD28 antibody magnetic beads were added at a concentration of 10ul/10⁶ cells for activation. The cells were seeded into a 12-well plate at 4mL/well, and cultured in a 37°C, CO₂ incubator.

### 1.2 T cell CAR-positive lentiviral transduction

CD3-positive T cells were activated with anti-CD3/CD28 magnetic beads for 24-48hr and then underwent viral transduction. Viability detection and cell counting were performed on the cell suspension. The cells were centrifuged at 300g for 10 min and collected. The cell density was adjusted to 1×10⁶/mL, lentivirus was added at a volume of 1mL per well in a 24-well plate, the MOI was adjusted to 3, and 800ng/uL PolyBrene and 1ug/uL DEAE were added to assist in the transduction. After mixing well, the cells were further cultured in a 37°C incubator. After 24hr, the virus was removed by centrifugation at 300 g for 10min, and the T cells were cultured.

### 1.3 T cell gene knockout

Taking TRAC and CD5 knockout as an example: T cells were activated with anti-CD3/anti-CD28 antibody magnetic beads for 24hr, then the magnetic beads were removed, the cells were counted and the cell viability was determined. Several portions of cell suspensions, 2×10⁶/portion, were taken and placed in centrifuge tubes and centrifuged at 100g for 10min. After centrifugation, the culture medium was completely removed and the cells were resuspended in Lonza electroporation buffer. At the same time, for each portion, RNP complexes of 35pmol Cas9 protein + 75pmol TRAC-sgRNA; 35pmol Cas9 protein + 60pmol CD5-sgRNA were prepared and incubated for 15min at room temperature. Then the cell suspension and RNP complex were well mixed and added to the Lonza 16-well electroporation cuvette, and placed in the 4D-Nucleofector^{™} X unit for electroporation with the EH-115 program. To the electroporation cuvette, pre-culture at 80ul/well was added to incubate at 37°C for 15min, then the cells were transferred to a 48-well plate for further culturing with 500uL of T cell complete medium.

### 1.4 LCK-T316I gene editing (introduction of Km editing)

T cells were taken, put as per 2×10⁶/cell suspension in a centrifuge tube for each group, and centrifuged at 100g for 10min. After centrifugation, the culture medium was completely removed and the cells were resuspended in Lonza electroporation buffer. At the same time, RNP complexes (per poriton) were prepared for the above groups: the RNP complexes of ① 60pmol CBE3 protein; ② 60pmol CBE3 protein + 100pmol K-sgRNA12; ③60pmol CBE3 protein + 100pmol K-sgRNA16 were respectively incubated at room temperature 15min. Then the cell suspension and RNP complex were well mixed and added to the Lonza 16-well electroporation cuvette, and placed in the 4D-Nucleofector^{™} X unit for electroporation with the EH-115 program. To the electroporation cuvette, 80ul of pre-culture was added to incubate at 37°C for 15min, then the cells were transferred to a 48-well plate for further culturing with 500uL of T cell complete medium.

### 1.5 Detection of gene knockout efficiency

Taking TRAC and CD5 knockout as an example: 72hr after T cells were electroporated to knock out TRAC and CD5 genes, the efficiency of TRAC and CD5 gene knockout was detected by flow cytometry. The specific steps are as follows: about 2×10⁵ cells were taken and put in a 1.5mL centrifuge tube, washed twice with PBS+2% fetal bovine serum buffer. The supernatant was completely discarded. The cells were resuspended with 100µL of buffer, 1 µL of each of PE-anti-human-TRAC and APC-anti-human-CDS antibodies were added, the cells were mixed well, incubated at 4°C in the dark for 30min, washed once with buffer, and tested on the machine.

### 1.6 CAR positive rate detection

The CAR positive rate was detected by flow cytometry 5 days after infection of T cells by lentivirus. The specific steps are as follows: Two portions of about 1×10⁵cells were taken, and placed in a 1.5mL centrifuge tube, washed twice with PBS+2% fetal bovine serum buffer, and the supernatant was completely discarded. For one portion, the cells were resuspended with 100µl of buffer, and 5µl of FITC-labeled-human-target antigen protein was added. For another portion, the cells were resuspended with 100µl of buffer, and 1µL of APC-anti-human-EGFR (transduction marker) antibody was added (if there is EGFR expression). The cells were mixed well and incubated at 4°C in the dark for 30min, washed once with buffer, and then tested on the machine.

### 1.7 Detection of editing efficiency of LCK-T316I gene editing

The T316I mutation rate was detected 72hr after Km editing of T cells or CAR-T cells. About 1×10⁵ cells in each group were taken for genomic DNA extraction, and the genomic DNA was used as a template to amplify a DNA fragment covering the Km region and including about 200 bp upstream and downstream sequences by PCR. The base sequence map was obtained by Sanger sequencing. The .ab1 format file of Sanger sequencing in the EditR web version was uploaded and the 20bp sgRNA sequence were input, DataQC and Predict Editing were clicked to view the analysis results, and the results were downloaded through Download Report.

The main reagent materials used are listed in Table 2.

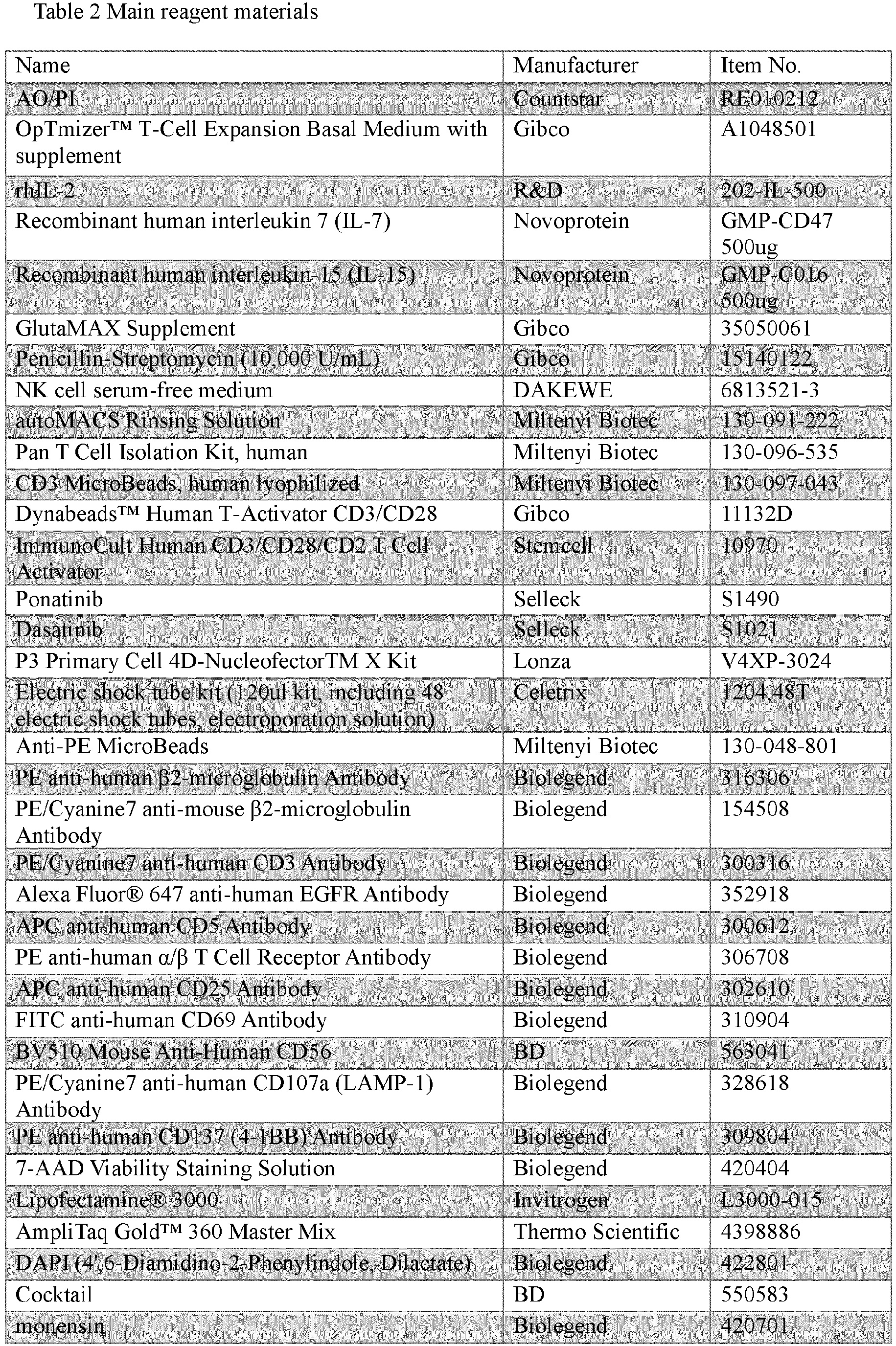

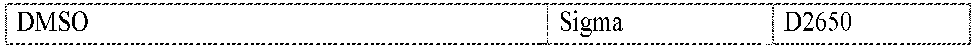

### Example 2: Using single base editor A3A-CBE3 to efficiently induce human LCK^{T316I} mutation (Km for short) in T cells

The inventors used the single-base cytosine editor A3A-CBE3[¹⁰] based on CRISPR-Cas9 technique to induce T316I mutation in the human LCK protein, that is, the position 316 amino acid of the human LCK protein was mutated from threonine (T) to Isoleucine (I), referred to as Km for fort.

The A3A-CBE3 protein is a nickase fusion protein of APOBEC3A and Cas9-D10A mutations, which can be targeted to a specific position in the genome by a sgRNA of a specific sequence, and mediates the mutation of cytosine to thymine within the targeting range of the sgRNA, thereby realizing base editing and protein mutation. Cas9-D10A makes the D10A mutation on the RuvC1 domain of the Cas9 protein, making it a Cas9-nickase protein (Cas9n), which only retains the enzymatic activity of the HNH domain, so that Cas9n will not cause DNA double-strand to break, but can only cleave the DNA single strand complementary to sgRNA on the genome, thereby inducing base mismatch repair (BER). At this time, APOBEC3 can induce the deamination of cytosine on the other DNA single strand to form uracil, and finally promote the mutation of cytosine to thymine (C->T mutation) in the presence of UGI protein.

The inventors designed the sgRNA for inducing LCK^{T316I} mutation.

As shown in Fig. 10A, by analyzing the gene sequence encoding the LCK protein, the inventors designed sgRNAs that meet the characteristics of cytosine base editing at the T316 site near the LCK-T316 site, and finally there are two sgRNAs that can meet the single-base editing requirements -- LCK-sgRNA12 and LCK-sgRNA16. When T316 (ACT) is base-edited, its second codon cytosine (C) is directionally mutated to thymine (T), which can induce the formation of I316 (ATT). At the same time, the inventors improved the length of the sgRNA in order to increase its mutation-inducing efficiency, and designed sgRNAs ranging from 18nt to 21nt in length, as shown in Fig. 1B.

The inventors verified the function and efficiency of sgRNA on the induction of LCK^{T316I} mutation. LCK-sgRNA16 can efficiently induce LCK-T316I mutation, while LCK-sgRNA12 cannot induce LCK - T316I mutation.

The inventors isolated CD3-positive T cells from frozen human PBMCs, and the sorting positive rate was over 95%. The sorted CD3 positive T cells were stimulated and activated with anti-CD3/CD28 DynaBeads magnetic beads for 48hr and then used for mutation induction. After the A3A-CBE3 protein and LCK-sgRNA16 of different lengths were co-incubated to form RNP complexes in vitro, the activated T cells were electroporated by using LONZA Nucleofector-4D electroporation instrument and P3 Primary Cell 4D-Nucleofector^{™} X Kit with electroporation program of EH-115. After electroporation, the culture was continued for 96hr, the T cell genome was extracted, and the efficiency of lck gene editing was detected by PCR and Sanger sequencing.

As shown in Fig. 11A, T cells in the group only electroporated with A3A-CBE3 protein without sgRNA did not have any base mutation in the lck gene region of the genome (single peak map for each sequencing site), while the RNPs formed by LCK-sgRNA16 of different lengths and A3A-CBE3 protein can all cause the mutation of T316I (a red sequencing peak representing thymine T appears on the blue sequencing peak of cytosine C at the T316 site, forming a nested peak). According to the peak analysis of the nested peak, the mutation efficiency was about 50%. At the same time, the four cytosines before the T316 site also underwent C->T mutation, but since these four cytosines are all in the third degenerate codon position of the amino acid, the main mutations except the mutated cytosine at the T316 site did not result in amino acid changes.

As shown in Fig. 11B, the inventors analyzed the sequencing results through EditR[¹¹], and statistically analyzed the mutation frequency of cytosine at the main mutation site of LCK under the induction by 18nt, 20nt and 21nt sgRNAs. The results show that the induction efficiencies of cytosine mutation at the T316 site by sgRNAs of different lengths were basically the same, all around 50%. Therefore, in activated T cells, these three LCK-sgRNA16 of different lengths can all induce LCK-T316I mutation efficiently. In order to reduce the impact of sgRNA off-target on editing, we used 21nt sgRNA16 for subsequent experiments.

The inventors verified that LCK-sgRNA12 can not induce LCK-T316I mutation.

Similar to the above gene editing experiment, the inventors also tested the induction efficiency of LCK-sgRNA12 on T cell LCK-T316 mutation. As shown in Fig. 12, compared with the sequencing results of the control group only electroporated with A3A-CBE3 protein, LCK-sgRNA12 of different lengths basically did not edit the cytosine at the T316 site, and induced about 50% of C->T mutations on the four cytosines before the T316 site, but still failed to cause any changes in amino acids. Therefore, LCK-sgRNA12 cannot effectively induce LCK-T316I mutation, but it can be used as a negative control for sgRNA16, so we chose 21nt LCK-sgRNA12 as a control for subsequent experiments to ensure the same length as Lck-sgRNA16-21 nt.

### Example 3: Verification of fact that dasatinib can effectively inhibit TCR signals to inhibit the activation of T cells

Dasatinib, as an inhibitor of Src/ABL kinase, can effectively inhibit many functions of normal human T lymphocytes in vitro, including: dasatinib can effectively block the transduction of TCR signal by binding to LCK, inhibit the activation, cytokine secretion and proliferation of T cells in vitro, but will not affect the viability of T cells [¹]. It is known that dasatinib inhibits TCR-mediated signal transduction, cell proliferation, cytokine secretion and cellular response in vivo by inhibiting LCK phosphorylation. But this inhibition is reversible, and when dasatinib is withdrawn, the function of T cells can be restored [²]. Meanwhile, dasatinib is also a potential, rapid and reversible inhibitor of CAR-T cell function, which can inhibit the proliferation, cytokine secretion and in vivo tumor killing activity of CAR-T cells [³], can inhibit the secretion of cytokines and proliferation of CAR-T cells in vivo and in vitro, and this inhibition is reversible [⁴].

In order to verify that the LCK^{T316I} mutation can make universal CART cells resistant to the inhibition of T cell activation by dasatinib, the inventors first repeated that dasatinib can effectively inhibit TCR signals to inhibit T cell activation.

The inventors pre-treated T cells with dasatinib or DMSO for 5hr, then activated T cells with anti-CD3/CD28 DynaBeads for 24hr, and then detected the expression of activation molecules CD25, CD69 and 4-1BB by flow cytometry. As shown in Fig. 13, after 24hr of magnetic bead activation in the DMSO control group, the activated molecule CD25 and CD69 double-positive cells were significantly up-regulated, from 2.85% to 38.9% (Fig. 13A), while after treatment with 100nM or 1000nM dasatinib for 5hr, activation with magnetic beads for 24hr could not up-regulate the percentage of CD25 and CD69 double-positive cells (Fig. 13A, middle and bottom). Similarly, after 24hr of magnetic bead activation in the DMSO control group, the activated molecule CD25 and 4-1BB double-positive cells were also significantly up-regulated, from 1.11% to 35.9% (Fig. 13B), while after treatment with 100nM or 1000nM dasatinib for 5hr, activation with magnetic beads for 24hr could not up-regulate the percentage of CD25 and 4-1BB double-positive cells (Fig. 13B, middle and bottom). These data suggest that dasatinib can inhibit the activation of TCR signaling, and the concentration of 100nM is sufficient to function.

### Example 4: LCK^{T316I} mutation makes T cells tolerant to the inhibition of TCR activation by dasatinib

In order to verify the tolerance of the LCK-T316I mutation to the function of dasatinib, the inventors carried out experimental verification on the T cells with LCK edited with different sgRNA lengths prepared in Example 2. Three groups of different treatments were set for each group of electroporated T cells and MockT cells without electroporation: as shown in Fig. 14A, the first group was not treated with dasatinib and not activated with CD3/CD28 magnetic beads (Fig. 14A, the first row); the second group was not treated with dasatinib, but had TCR activation with anti-CD3/CD28 magnetic beads for 24hr (Fig. 14A, the second row); the third group was pre-treated with 100nM dasatinib for 5hr, followed by TCR activation with anti-CD3/CD28 magnetic beads for 24hr (Fig. 14A, the third row); and finally the expression of T cell activation signals such as CD25, CD69 and 4-1BB was detected by flow cytometry to verify that the LCK-T316I mutation can tolerate the inhibition of T cell activation by dasatinib.

In this example, the T cells were edited and grouped as follows: the MockT group was T cells without electroporation, the 01CBE3 group was T cells electroporated with only CBE3 protein; groups 02-05 were T cells electroporated with RNPs formed by CBE3 protein and LCK-sgRNA12 of different lengths, which can be used as negative controls for LCK editing because sgRNA will not cause any amino acid mutation of LCK protein; groups 06-08 were T cells electroporated with RNPs formed by CBE3 protein and LCK-sgRNA16 of different lengths, these T cells had LCK-T316I mutation with mutation efficiency of about 50%. The data are shown in Fig. 11 of Example 2.

The inventors proved that LCK-T316I mutation can make T cells tolerant to the inhibition of TCR activation by dasatinib.

As shown in Fig. 14, without dasatinib treatment and without T cell activation by anti-CD3/CD28 magnetic beads (Fig. 14A, Fig. 14B, the first row), all detected T cells had no expression of activation molecules CD69 (Fig. 14A) and 4-1BB (Fig. 14B). When T cells were activated with anti-CD3/CD28 magnetic beads (Fig. 14A, Fig. 14B, the second row), the expression of CD25, CD69 and 4-1BB was significantly increased in each group of cells, proving that whether LCK is edited or not, the T cells can be effectively activated by anti-CD3/CD28 magnetic beads to transduce TCR signals. After pretreatment with 100nM dasatinib for 7hr, T cells were activated with anti-CD3/CD28 magnetic beads (Fig. 14A, Fig. 14B, the third row). At this time, except for the T cells in the LCK-T316I edited groups (groups 06-08), the expression of activated molecules CD25, CD69 and 4-1BB in the cells without LCK editing (MockT, groups 01-05) was consistent with that in the first row of unactivated groups, suggesting that 100nM dasatinib can completely inhibit TCR signaling activation and increased expression of CD69 and 4-1BB due to T cell activation. However, T cells with LCK-T316I mutation (groups 06-08) could still be activated by anti-CD3/CD28 stimulation to transduce TCR signals with 100nM dasatinib treatment, showing significantly increased expression of activated molecules CD25, CD69 and 4-1BB, etc. (Figs. 14A, 14B, the third row, groups 06-08), which further proves that the LCK-T316I mutation can make T cells tolerant to the inhibition of TCR activation by dasatinib.

The inventors further statistically analyzed the data, set groups MockT and 01-CBE3 as blank control groups, set groups 02-05 as sgRNA12 editing groups (nonsense mutation groups), and set groups 06-08 as sgRNA16 editing groups (LCK-T316I mutation groups), and statistically analyzed the percentages of positive cells expressing CD25, CD69 and 4-1BB under the three treatment conditions, respectively. As shown in Fig. 14C, in the sgRNA16 editing group, with treatment by dasatinib and anti-CD3/CD28 magnetic beads, the expression percentages of the three activation markers were significantly higher than those in the control group and the sgRNA12 editing group. It is further suggested that T cells in the sgRNA16 editing group (-50% LCK-T316I mutation) show tolerance to inhibition of T cell activation function by dasatinib, and T cells with LCK - T316I mutation can still be activated.

In addition, however, compared with the cells in the sgRNA16 group directly activated by anti-CD3/CD28 magnetic beads without dasatinib treatment, the expression of activation molecules in the dasatinib treatment group was significantly reduced (about 20%-50%, Fig. 14C). This is due to the fact that the efficiency of the sgRNA16 editing group was about 50%, and it cannot achieve 100% editing. Some T cells with LCK-T316I mutation showed tolerance with the treatment with dasatinib, while others unedited cells still cannot be effectively activated, which eventually leads to a decrease in the activation percentage. These data well illustrate the phenomenon of tolerance of LCK-T316I mutation to inhibition of T cell activation function by dasatinib.

### Example 5: LCK^{T316I} mutation makes anti-CD19-CAR-T cells tolerant to the inhibition of CD 107a translocation by dasatinib

Based on the fact that LCK-T316I mutation can make T cells tolerant to the inhibition of TCR activation by dasatinib, which had been verified, in order to verify that LCK-T316I mutation can not only make T cells tolerant to the inhibition of TCR activation by dasatinib, but also make CAR-T cells tolerant to the inhibition of CD107a translocation by dasatinib during CAR activation, the inventors designed and carried out the following experiments to prove that LCK-T316I mutation makes anti-CD19-CAR-T cells tolerant to the inhibition of CD107a translocation by dasatinib.

First, the inventors edited the prepared anti-CD19 CAR-T by electroporation with the CBE3 protein, the RNP complex of CBE3 and LCK-sgRNA12, and the RNP complex of CBE3 and LCK-sgRNA16 respectively, to achieve three different editing treatments. 72hr after editing, the mutation efficiency was detected, and the LCK-T316I mutation reached about 43% (Fig. 15A). According to the protocol shown in Fig. 15B, the inventors pretreated the CAR-T cells of each group with 100nM dasatinib for 12hr, and then co-incubated anti-CD19-CAR-T cells with different target cells (positive target cells Nalm6 expressing CD19 antigen and negative target cells K562 not expressing CD19 antigen) to stimulate and activate CAR signals, and mediate killing of target cells by CAR-T and accumulation of CD107a in CAR-T. 5hr before detection, monensin and anti-CD107a antibodies were added, and staining for CD8 and CAR positive was performed 5hr later to detect the enrichment of CD107a translocation of CD8 positive CAR positive cells.

By analyzing the data of flow cytometry, in the gating strategy shown in Fig. 15C, the CD107a translocation enrichment was analyzed in CD8 and CAR double-positive cells. The results show that no matter whether anti-CD19-CAR-T cells were edited or not, in the DMSO treatment control group, the negative target cell K562 could not activate CAR-T for effective CD107a translocation (the first column from the left in Fig. 15D), while the positive target cell Nalm6 could fully activate CAR-T to release CD107a with a positive rate of above 70% (the third column from the left in Fig. 15D). In the 100nM dasatinib treatment group, the negative control group K562 hardly had any CD107a translocation enrichment (the second column from the left in Fig. 15D), while under the positive target cell Nalm6 stimulation, only the CAR-T cells in the LCK-sgRNA16 editing group (LCK-T316I mutation percentage, 43%) showed 50% positive CD107a translocation (the fourth column from the left in Fig. 15D), suggesting that LCK-T316I mutation can indeed make anti-CD19-CAR-T cells tolerant to the inhibition of CD107a translocation by dasatinib.

The inventors performed statistical analysis on the two replicates of the above experiment. According to the results shown in Fig. 15E, the positive target cells Nalm6 could activate all anti-CD19-CAR-T cells to release CD107a. After treated with 100nM dasatinib, the CD107a release in the CBE3 control group and the LCK-sgRNA12 nonsense mutation group was significantly reduced, while the CD107a release in the LCK-sgRNA16-T316I mutation group was still at a high level, significantly different from the control group. This phenomenon shows that dasatinib can not only inhibit the activation of TCR signals in T cells, but also inhibit the activation of CAR signals in CAR-T cells; and also, LCK-T316I mutation can resist the inhibitory effect of dasatinib on the activation of CAR signals in CAR-T cells.

### Example 6: LCK^{T316I} mutation makes anti-BCMA-CAR-T cells tolerant to the inhibition of CD107a translocation by dasatinib

In Example 5, the inventors had demonstrated that LCK-T316I mutation makes anti-CD19-CAR-T cells tolerant to the inhibition of CD107a translocation by dasatinib. In order to strengthen this conclusion, in this example, the inventors verified that it can still be observed in BCMA-targeted CAR-T that LCK-T316I mutation makes BCMA-CAR-T cells tolerant to the inhibition of CD107a translocation by dasatinib.

First, the inventors edited the prepared anti-BCMA CAR-T by electroporation with CBE3 protein, the RNP complex of CBE3 and LCK-sgRNA12, and the RNP complex of CBE3 and LCK-sgRNA16 respectively, to achieve three different editing treatments. 72hr after editing, the mutation efficiency was detected, and the LCK-T316I mutation reached about 42% (Fig. 16A). The inventors pretreated the CAR-T cells of each group with 100nM dasatinib for 12hr, and then co-incubated anti-BCMA-CAR-T cells with different target cells (positive target cells U266B1 and RPMI-8226 expressing BCMA antigen and negative target cells K562 not expressing BCMA antigen) to stimulate and activate CAR signals, and mediate killing of target cells by CAR-T and accumulation of CD107a in CAR-T. 5hr before detection, monensin and anti-CD107a antibodies were added, and staining for CD8 and CAR positive was performed 5 h later to detect the enrichment of CD107a translocation of CD8 positive CAR positive cells.

By analyzing the data of flow cytometry, the CD107a translocation enrichment was analyzed in CD8 and CAR double-positive cells. The results are shown in Fig. 16B. No matter whether anti-BCMA-CAR-T cells were edited or not, in the DMSO treatment control group, the negative target cell K562 could not activate CAR-T for effective CD107a translocation (the first column from the left in Fig. 16B), while the positive target cells U266B1 and RMPI-8228 could fully activate CAR-T to release CD107a with a positive rate of above 68% (the third and fifth columns from the left in Fig. 16B). In the 100nM dasatinib treatment group, the negative control group K562 hardly had any CD107a translocation enrichment (the second column from the left in Fig. 16B), while under stimulation by the positive target cells U266B1 or RMPI-8226, only the CAR-T cells in the LCK-sgRNA16 editing group (LCK-T316I mutation percentage of 42%) showed 39.6% and 44.7% positive CD107a translocation (the fourth and sixth columns from the left in Fig. 16B), suggesting that LCK-T316I mutation can indeed make anti-BCMA-CAR-T cells tolerant to the inhibition of CD107a translocation by dasatinib.

The inventors performed statistical analysis on the two replicates of the above experiment. According to the results shown in Fig. 16C, the positive target cells U266B1 and RMPI-8228 could activate all anti-BCMA-CAR-T cells to release CD107a. After treated with 100nM dasatinib, the CD107a release in the CBE3 control group and the LCK-sgRNA12 nonsense mutation group was significantly reduced, while the CD107a release in the LCK-sgRNA16-T316I mutation group was still at a high level, significantly different from the control group. This result further demonstrates that the LCK-T316I mutation can resist the inhibitory effect of dasatinib on the activation of CAR signals in CAR-T cells.

### Example 7: LCK^{T316I} mutation makes anti-CD19/BCMA dual-target UCAR-T cells tolerant to the inhibition of CD107a translocation by dasatinib

In Example 5 and Example 6, the inventors had demonstrated that LCK-T316I mutation makes anti-CD19-CAR-T cells and anti-BCMA-CAR-T cells tolerant to the inhibition of CD107a translocation by dasatinib. In order to strengthen this conclusion, in this example, the inventors further verified that it can still be observed in CD19 and BCMA bispecific CAR-T cells that LCK-T316I mutation resists the inhibition of CD107a translocation by dasatinib.

The inventors edited the prepared CD19/BCMA dual-target CAR-T by electroporation with the CBE3 protein, the RNP complexes (18nt and 21nt) of CBE3 and LCK-sgRNA12, and the RNP complexes (18nt and 21nt) of CBE3 and LCK-sgRNA16 respectively, to achieve three different groups of editing treatments. The inventors pretreated the CAR-T cells in each group for 12hr with 100nM dasatinib, and then co-incubated the anti-BCMA-CAR-T cells with different target cells to verify the activation of CAR signaling by different target cells and the release of CD107a. These target cells include U266B1 (BCMA positive), Raji (CD19 positive), K562 (negative control), allogeneic T cells (negative control) and blank Buffer control (negative control). 5hr before detection, monensin and anti-CD107a antibodies were added, and staining for CD8 and CAR positive was performed 5 h later to detect the enrichment of CD107a translocation of CD8 positive CAR positive cells.

The results are shown in Fig. 17A. By analyzing the data of flow cytometry, the CD107a translocation enrichment was analyzed in CD8 and CAR double-positive cells. Unedited CD19/BCMA bispecific CAR-T cells could be effectively activated by U266B1 and Raji cells with DMSO treatment, with CD107a expression positive being 74.5% and 54.5%, but could not be activated by K562, host T and Buffer (Fig. 17A, the first row). With 100nM dasatinib treatment, CAR-T cells without LCK-T316I editing (Fig. 17A, the second row to the fifth row) could not be activated by the two positive target cells, U266B1 and Raij to release CD107a, and only CAR-T cells with LCK-T316I mutation (Fig. 17A, the sixth and seventh rows) could resist the inhibition of dasatinib on CAR signaling activation and were activated by U266B1 and Raji to release CD107a, with the positive rate being above 50%. Fig. 17B is a histogram representation of the data from Fig. 17A. These data suggest that LCK-T316I can make CD19/BCMA bispecific CAR-T cells tolerant to the inhibition of CD107a translocation by dasatinib.

### Example 8: LCK^{T316I} mutation makes anti-BCMA CAR-T cells tolerant to the inhibition of CAR-T cytotoxicity by dasatinib

The above Example 4 demonstrates that the mutation LCK-T316I can make T cells tolerant to the inhibition of TCR activation by dasatinib, and Examples 5-7 demonstrate that the mutation LCK-T316I can make CAR-T cells tolerant to the inhibition of CAR activation and CD107a translocation by dasatinib. In order to further prove that the LCK-T316I mutation can make CAR-T cells resist the inhibition of the cytotoxicity of CAR-T by dasatinib, the inventors carried out the following experiments on anti-BCMA CAR-T cells.

The inventor's experiment proved that the anti-BCMA CAR-T after LCK-T316I mutation becomes tolerant to the inhibitory function of dasatinib on CAR-T cytotoxicity.

The inventors conducted experiments using the edited anti-BCMA CAR-T cells in Example 6, wherein the LCK-T316I mutation efficiency was 42% (Fig. 16A). As shown in Fig. 18A, the inventors pretreated the CAR-T cells of each group with 100nM dasatinib for 12hr, and subsequently co-incubated them with different luciferase-positive target cells (luciferase+) at an effector-to-target ratio of 1:1 for 24hr, with two replicates for each group. After incubation, the killing of target cells by CAR-T cells was evaluated by detecting the activity of luciferase. The lower the value of luciferase, the fewer surviving target cells and the stronger the killing effect of CAR-T.

The results are shown in Figs. 18B and 18C. In the DMSO control group, whether LCK-T316 was edited or not, anti-BCMA CAR-T could significantly reduce the luciferase value of RPMI-8226 positive target cells, but did not affect the luciferase value of negative target cells Nalm6, proving that anti-BCMA CAR-T cells have the normal function of killing target cells. With the treatment with 100nM dasatinib and after stimulation with positive target cells RPMI-8226, the luciferase value in the CBE3 protein control group and the LCK unedited group (CBE3+sg12) was significantly higher than that in the DMSO group, proving that with the treatment with dasatinib, the cytotoxicity of anti-BCMA CAR-T is significantly inhibited. The luciferase value in the CAR-T cells with LCK-T316I mutation (CBE+sg16) was significantly lower than that in the other two groups, proving that the anti-BCMA CAR-T after LCK-T316I mutation develops tolerance to inhibition of CART cytotoxicity by dasatinib.

The inventor's repeated experiments proved that the anti-BCMA CAR-T after LCK-T316I mutation becomes tolerant to the inhibitory function of dasatinib on CART cytotoxicity.

The inventors performed repeated killing experiments on the above-mentioned LCK-edited and non-edited anti-BCMA CAR-T cells. The experimental procedure was consistent with Fig. 18A in this example. The CAR-T cells of each group were pretreated with 100nM dasatinib for 12hr, and subsequently co-incubated with different luciferase-positive target cells at an effector-to-target ratio of 1:1 for 24hr, with two replicates for each group. After incubation, the killing of target cells by CAR-T cells was evaluated by detecting the activity of luciferase. The lower the value of luciferase, the fewer surviving target cells and the stronger the killing effect of CAR-T.

The results are shown in Figs. 19A and 19B. In the DMSO control group, whether LCK-T316 was edited or not, anti-BCMA CAR-T could significantly reduce the luciferase value of RPMI-8226 positive target cells, but did not affect the luciferase value of negative target cells K562 and Nalm6, proving that anti-BCMA CAR-T cells have the normal function of killing target cells. With the treatment with 100nM dasatinib and after stimulation with positive target cells RPMI-8226, the luciferase value in the CBE3 protein control group and the LCK unedited group (CBE3+sg12) was significantly higher than that in the DMSO group, proving that with the treatment with dasatinib, the cytotoxicity of anti-BCMA CAR-T is significantly inhibited. The luciferase value in CAR-T cells with LCK-T316I mutation (CBE+sg16) was significantly lower than that of the other two groups. In addition, although the negative target cell K562 partially activated LCK-T316I-edited CAR-T cells with 100nM dasatinib treatment, the negative target cell Raji did not activate anti-BCMA-CART cells, which is a good negative control. These data again prove that the anti-BCMA CAR-T after LCK-T316I mutation becomes tolerant to the inhibitory function of dasatinib on CART cytotoxicity.

### Example 9: Dasatinib at a concentration of 25nM can effectively inhibit the activation of T cells in vitro

In the above examples, the inventors proved that dasatinib at a concentration of 100nM can effectively inhibit the activation of TCR and CAR signals, that is, at this concentration, the activation of normal T cells and CAR-T cells is inhibited. At this point, the LCK-T316I mutation can make T cells tolerant to the inhibition of its activation by dasatinib, that is, the UCAR-T cells with the LCK-T316I mutation can retain the ability of being activated by and killing target cells in the environment of 100nM dasatinib.

Based on this, the strategy proposed in the present invention to realize universal CAR-T is to use dasatinib to pretreat patients to inhibit the activation function of host T cells, and then infuse back the UCAR-T cells with LCK-T316I mutation. In this case, the host T cells are unable to clear UCAR-T cells due to the inhibition of TCR activation by dasatinib; at the same time, UCAR-T cells are able to resist the inhibition of CAR signal activation by dasatinib due to the LCK - T316I mutation, and target malignant cells and mediate tumor clearance.

The inventors have demonstrated in Example 3 that 100nM dasatinib is sufficient to inhibit the activation of TCR signals in T cells. In order to further explore the concentration threshold of dasatinib to inhibit T cell activation and guide clinical medication, the inventors further explored lower concentrations of dasatinib.

The inventors isolated CD3-positive T cells from human peripheral blood and divided them into eleven portions for processing, with 1e5 cells in each portion. One of these portions was not treated with dasatinib, anti-CD3/CD28 DynaBeads were not added to activate TCR, and this portion was as the initial control group; the other 10 portions of cells were pretreated with different concentrations of dasatinib for 4hr, ten concentrations ranging from 0nM to 50nM were set, and then anti-CD3/CD28 DynaBeads were added to activate TCR signals overnight. On the second day, the expression of CD25, CD69 and 4-1BB activation signals was detected, and the activation of TCR signals was detected. Two replicates were set for each group of experiments.

As shown in Fig. 20A, after T cells not treated with dasatinib were activated by anti-CD3/CD28 magnetic beads, 60.7% of CD25/CD69 double-positive cells were activated (the first row and second column of Fig. 20A ), proving that anti-CD3/CD28 magnetic beads can effectively activate this batch of T cells. With the treatment with dasatinib at a concentration of 10-50 nM added in a gradient, the activation of TCR signals by anti-CD3/CD28 magnetic beads was gradually weakened (Fig. 20A, the first row, the third column to the sixth column, and the second row, the first column to the fifth column). From 10nM to 20nM dasatinib treatment interval, the percentage of CD25 and CD69 double-positive cells gradually decreased from 60.7% to 8.75%, showing an obvious dose-dependent effect. When the concentration of dasatinib was 25nM, the percentage of CD25 and CD69 double-positive cells was significantly reduced to 1.06%, and when the concentration continued to increase to 50nM, the percentage of double-positive cells basically remained below 1%.

Meanwhile, as shown in Fig. 20B, CD25 and 4-1BB were used as indicators of activated T cells, and the conclusion was consistent with Fig. 11A. With the treatment with dasatinib at a concentration of 10-50 nM added in a gradient, the activation of TCR signals by anti-CD3/CD28 magnetic beads was gradually weakened (Fig. 20B, the first row, the third column to the sixth column, and the second row, the first column to the fifth column). From 10nM to 20nM dasatinib treatment interval, the percentage of CD25 and 4-1BB double-positive cells gradually decreased from 31.0% to 2.09%, showing an obvious dose-dependent effect. When the concentration of dasatinib was 25nM, the percentage of CD25 and CD69 double-positive cells was significantly reduced to 0.26%, and when the concentration continued to increase to 50nM, the percentage of double-positive cells basically remained below 1%.

The above data show that dasatinib at a concentration of 25nM can effectively inhibit the activation function of T cells in vitro. It can basically guide the minimum in vivo concentration of dasatinib required to inhibit TCR signal activation in clinical medication (25nM). It is speculated that when the concentration of dasatinib in vivo is above 25nM, it can inhibit the activation of host T cells.

### Example 10: The inhibition of T cell activation function by dasatinib has an extended effect

In Example 9, the inventors proved that dasatinib at a concentration of 25nM can effectively inhibit the activation function of T cells in vitro, so as to guide the concentration used in vivo. According to the existing pharmacokinetic data of dasatinib [¹²], the inventors found that compared with other tyrosine kinase inhibitors, the half-life of dasatinib in vivo is extremely short, 3-4hr. Meanwhile, for patients with CML and ALL in different stages, the dosage of dasatinib is 100mg or 140mg per day, or twice a day with halved dose. Therefore, the metabolism of dasatinib in vivo is relatively rapid, and it is very likely that there will be several hours in the interval of administration when its concentration is lower than 25nM. In order to fully consider fluctuations in the concentration of dasatinib in vivo, the inventors designed a dynamic concentration experiment of dasatinib in vitro to detect whether its inhibition on T cell activation is still effective with dynamic concentration changes.

In this example, CDS-UCAR-T cells with double knockout of CDS and TRAC were used, and edited by CBE3+LCK-sgRNA16 or CBE3+sgRNA12 at the same time to prepare UCAR-T cells with LCK-T316I mutation and control UCAR-T cells. Subsequently, they were treated overnight with dasatinib at a concentration of 50nM or 25nM, respectively. The next day, the concentration of dasatinib was reduced, as shown in Fig. 21A, after 50nM pretreatment overnight, the concentration was adjusted to 0, 15, 25, 35 and 50nM, respectively. As shown in Fig. 21B, the concentration was adjusted to 0, 10, 15, 20 and 25nM respectively after overnight pretreatment at 25nM. At the same time, Jurkat cells were used as positive target cells for activation, the cells were co-incubated at an effector-to-target ratio of 1:1, and CD107a and monensin were also added. After 4hr of co-incubation, CD107a release in CD8 and CAR double-positive cells was detected.

The results show that the sg12 control group CD5-UCART cells with LCK nonsense mutation were pretreated with 50nM dasatinib, then the concentration was reduced to 0nM, and the cells were activated by Jurkat target cells for 4hr to produce 12% positive CD107a release (Fig. 21A, the first row, the first column); when the concentration was reduced to 15, 25, 35, 50nM, there was no release of CD107a (Fig. 21A, the first row, the second column to the fifth column), proving that changing the concentration of dasatinib to 15nM after 50nM pretreatment is sufficient to inhibit the activation of UCAR-T cells within 4hr. In addition, the CD5-UCART cells in the sg16 editing group with LCK-T316I mutation were pretreated with 50nM dasatinib, then the concentration was reduced to 0nM, and the cells were activated by Jurkat target cells for 4hr to produce 16% positive CD107a release (Fig. 21A, the second row, the first column); when the concentration was reduced to 15, 25, 35, 50nM, there was still high level of release of CD107a (Fig. 21A, the second row, the second column to the fifth column), proving that LCK-T316I mutation can tolerate the inhibition of CAR signals by dasatinib.

Consistent with the experimental results of 50nM dasatinib pretreatment, the inventors proved that after 25nM dasatinib pretreatment, adjusting its concentration to 10nM is enough to inhibit the CD5-UCAR-T cells in the control group from being activated by the target cells and releasing CD107a (Fig. 21B, the first row, the first and second columns), while the CD5-UCART cells in the sg16 editing group with LCK-T316I mutation still had a high level of CD107a release after the concentration of dasatinib was reduced (Fig. 21B, the second row, the second column to the fifth column), proving once again that LCK-T316I mutation can tolerate the inhibition of CAR signals by dasatinib.

The above experiment proved that the inhibition of T cell activation function by dasatinib has an extended effect. Dasatinib can inhibit the activation function of T cells with the dynamic concentration changes in vitro from 50nM to 15nM or from 25nM to 10nM, and LCK-T316I mutation can resist this inhibition.

In order to repeatedly verify that the inhibition of T cell activation function by dasatinib has an extended effect, the inventors repeated the above experiment. CD5-CAR-T cells were pretreated with 50nM or 25nM dasatinib overnight, the drug concentration was adjusted and decreased the next day, the cells were co-incubated with positive or negative target cells, and blocker monensin and anti-CD107a antibody were also added. After 4hr of co-incubation, CD107a release from CD8/CAR double-positive cells was detected.

As shown in Fig. 22A, 23.6% of CD5-CART cells were activated by Jurkat to become CD107a positive in the environment without dasatinib (Fig. 22A, the first row, the first column), the concentration was reduced to 0nM after pretreatment with 50nM dasatinib, the cells were activated by Jurkat target cells for 4hr and produced 12.1% positive CD107a release (Fig. 22A, the first row, the second column). When the concentration was reduced to 10, 20, 30, 40 and 50nM, there was no significant release of CD107a (Fig. 13A, the first row, the third column to the seventh column), proving that changing the concentration of dasatinib to 10nM-20nM after 50nM pretreatment is enough to inhibit the activation of CAR-T cells within 4hr. Meanwhile, CD5 knockout CCRF cells as negative target cells and Buffer negative control group could not effectively activate CD5-CAR-T cells to release CD107a (Fig. 22A, the second row and the third row).

As shown in Fig. 22B, consistent with the results of the 50nM dasatinib pretreatment experiment, the inventors proved that after 25nM dasatinib pretreatment, adjusting its concentration to 10nM is sufficient to inhibit the activation of CD5-CAR-T cells by positive Jurkat target cells to release CD107a (Fig. 22B, the first row, the third column).

Summarizing the above two experiments, it is basically proved that the inhibition of T cell activation function by dasatinib has an extended effect. Dasatinib can inhibit the activation function of T cells with the dynamic concentration changes in vitro from 50nM to 10-15nM or from 25nM to 10nM, and LCK-T316I mutation can resist this inhibition.

The sequence of the anti-CD5-CAR molecule used here is the same as that used in the following examples, and it is a CAR molecule related to the single domain antibody sequence. See SEQ ID NOs: 57-76 for the specific sequence. The overall structure of this CAR is a secondgeneration CAR molecule, which consists of two anti-CD5 single-domain heavy chains (SEQ ID NOs: 57-60), plus a linker region (SEQ ID NOs: 61-62), plus a CD8a region (SEQ ID NOs: 63-64), plus a CD28 intracellular domain (SEQ ID NOs: 65-66), plus a CD3z intracellular signaling domain (SEQ ID NOs: 67-68), plus a T2A sequence (SEQ ID NOs: 69-70), plus a CSF2RA signal (SEQ ID NOs: 71-72), plus a tEGFR signal (SEQ ID NOs: 73-74). The total sequence of the final anti-CD19-CD28z-CAR signal is set forth in SEQ ID NOs: 75-76.

### Example 11: Verifying that dasatinib can efficiently inhibit the activation of NK cells

In addition to its inhibitory effect on the activation of T cells and CAR-T cells, dasatinib can also inhibit the degranulation and cytokine release of primary human NK cells [⁵]. Being cleared by functional host T and NK cells is the main reason why UCAR-T is difficult to expand in the host. Therefore, in the present invention, the treatment with dasatinib in the UCAR-T therapy can enhance the survival of UCAR-T cells in the host not only by inhibiting the activation of host T cells but also by inhibiting the activation of host NK cells.

The inventors first verified that dasatinib can efficiently inhibit the activation of NK cells.

The inventor used Miltenyi human NK sorting magnetic beads to separate NK cells from human peripheral blood, and used DAKEWE NK medium to culture and expand them in vitro. On Day 8, a population of NK cells with more than 95% having positive CD56 expression could be obtained. These NK cells were taken out and treated with 0nM, 100nM and 200nM dasatinib respectively for 24hr, and then divided into two groups, one of which was not activated, and the other group was stimulated with MHC-class I molecule-deficient K562 cells for 5hr. Then, the CD107a release of NK cells were detected in both of them.

As shown in Fig. 23, when NK cells in the non-activated group were not treated with dasatinib, the translocation of CD107a was 11.4% (the first row, the third column), and when treated with dasatinib, the basal CD107a translocation decreased to the baseline of 2% (the first row, the first and second columns). After K562 target cells were added to activate NK cells, the CD107a translocation in the untreated group was activated to 64.5% (the second row, the third column), while the CD107a translocation in the dasatinib treatment group was reduced to the baseline of 2% (the second row, the first and second columns), suggesting that 100nM dasatinib is enough to inhibit the CD107a translocation of the NK cells and inhibit the activation of NK cells.

### Example 12: Dasatinib at a concentration of 30nM can effectively inhibit the activation of NK cells in vitro

In the above examples, the inventors proved that dasatinib at a concentration of 100nM can effectively inhibit the activation of NK cells. In order to further explore the concentration threshold of dasatinib to inhibit NK cell activation and guide clinical medication, the inventors further explored lower concentrations of dasatinib.

The inventors isolated NK cells from human PBMCs, and then cultured and differentiated them in NK medium to obtain CD56-positive NK cells with a purity higher than 95%. The exploration of the threshold of NK activation with dasatinib was carried out with seven concentrations in the range of 0-50nM, namely 0, 10, 20, 25, 30, 40 and 50nM, and the treatment was carried out overnight. The next day, the cells were resuspended with NK medium and activated with K562. Lymphocyte activator was used as a positive control for activation, and a part of NK cells that were not activated by K562 were also kept. Two replicates were set for each group. anti-CD107a antibody and monensin were added while carrying out activation, and after co-incubation for 6hr, the CD107a release of CD56-positive NK cells was detected by flow cytometry.

As shown in Fig. 24A, without dasatinib treatment, the CD107a translocation of NK cells with lymphocyte activator is more than 80% (Fig. 24A, the upper row, the first column), and the activation of NK cells by K562 also reaches more than 76% (Fig. 24, the upper row, the third column). As the concentration of dasatinib increases, the percentage of NK cells activated by K562 to express CD107a gradually decreases. When the concentration of dasatinib reaches 30nM, K562 is basically unable to activate NK cells to promote the release of CD107a (Fig. 24A, the lower row, the second column), proving that 30nM dasatinib can effectively inhibit the activation of NK cells in vitro. As shown in Fig. 24B, the inventors analyzed the significance of the above experimental data using the histogram, and basically confirmed that compared with the lower concentrations of dasatinib, the concentration of 30nM can significantly inhibit the activation of NK cells.

The above data can basically guide the minimum in vivo concentration of dasatinib required to inhibit NK cells in clinical medication (30nM). It is speculated that when the concentration of dasatinib in vivo is above 30nM, it can inhibit the activation of host NK cells.

### Example 13: The inhibition of NK cell activation function by dasatinib in vitro has an extended effect

In Example 12, the inventors proved that dasatinib at a concentration of 30nM can effectively inhibit the activation function of NK cells in vitro, so as to guide the concentration used in vivo. As mentioned in Example 10, compared with other tyrosine kinase inhibitors, the half-life of dasatinib in the body is extremely short, its metabolism in the body is fast, and it is very likely that there will be several hours in the interval of administration when its concentration will be lower than 30nM [¹²]. In order to fully consider the fluctuations in the concentration of dasatinib in vivo, the inventors designed a dynamic concentration experiment of dasatinib in vitro to detect whether its inhibition of NK cell activation is still effective with dynamic concentration changes.

The inventors isolated NK cells from human PBMCs, and then cultured and differentiated them in NK medium to obtain CD56-positive NK cells with a purity higher than 95%. Three pretreatments were done respectively (1) absence of dasatinib pretreatment; (2) pretreatment with 25nM dasatinib; (3) pretreatment with 50nM dasatinib, and the cells were cultured overnight. The next day, the concentration of dasatinib was reduced, and the concentration was adjusted to 0, 7.5, 12.5, 17.5 and 25nM respectively. At the same time, K562 cells were used as positive target cells to activate NK cells, the cells were co-incubated at an effector-to-target ratio of 1:1, and CD107a and monensin were added at the same time. Two replicates were set for each group. After 5hr of co-incubation, CD107a release in CD56 positive NK cells was detected.

As shown in Fig. 25A, after pretreatment with three different concentrations of dasatinib, and then reducing the concentration to 0nM, NK cells could be significantly activated by K562, and the release of CD107a was above 58% (Fig. 25, the second column). When the concentration was reduced to 7.5nM, the NK cells in the 25nM and 50nM dasatinib pretreatment groups were basically unable to be activated by K562, and the release of CD107a was lower than 1% (Fig. 25, the fourth column). When the concentration was changed to 12.5, 17.5 and 25nM the next day, the NK cells in the 25nM and 50nM dasatinib pretreatment groups were more unable to be activated by K562, and the release of CD107a was lower than 1% (Fig. 25, the sixth, eighth and tenth columns).

As shown in Fig. 25B, the inventors analyzed the significance of the above experimental data using the histogram, and further clarified that NK cells could not be effectively activated by K562 cells after being pretreated with 25nM or 50nM dasatinib and then further treated for 4hr at a concentration reduced to 7.5nM. The cells without pretreatment were directly treated with 7.5nM dasatinib for 4hr, about 4.4% of the cells were still activated. There was a significant difference between the two situations. Therefore, the above experiment proved that the inhibition of NK cell activation function by dasatinib has an extended effect. Dasatinib has basically inhibited the activation of NK cells with the dynamic concentration changes in vitro from 25nM or 50nM pretreatment to a reduced concentration of 7.5nM.

The above experiment proved that the inhibition of NK cell activation function by dasatinib has an extended effect. Dasatinib can inhibit the activation function of NK cells with the dynamic concentration changes in vitro from 25nM or 50nM pretreatment to a reduced concentration of 7.5nM.

In order to repeatedly verify that the inhibition of NK cell activation function by dasatinib has an extended effect, the inventors repeated the above experiment. NK cells were pretreated with 50nM, 25nM and 10nM dasatinib overnight, the drug concentration was adjusted and decreased the next day, the cells were co-incubated with positive K562 or negative Buffer, and blocker monensin and anti-CD107a antibody were also added. After 4hr of co-incubation, CD107a release from CD56 positive NK cells was detected.

As shown in Fig. 26A, 61.5% of NK cells were activated by K562 to become CD107a positive in the environment without dasatinib (Fig. 26A, the first row, the first column), the concentration was reduced to 0nM after pretreatment with 50nM dasatinib, the cells were activated by K562 target cells for 4hr and produced 39.7% positive CD107a release (Fig. 26A, the first row, the second column). When the concentration was reduced to 10, 20, 30, 40 and 50nM, there was no significant release of CD107a (Fig. 26A, the first row, the third column to the seventh column), proving that changing the concentration of dasatinib to 10nM after 50nM pretreatment is enough to inhibit the activation of NK cells within 4 h. At the same time, in the Buffer negative control group, the NK cells could not be effectively activated to release CD107a (Fig. 17A, the second row).

As shown in Fig. 26B, consistent with the results of the 50nM dasatinib pretreatment experiment, the inventors proved that after 10nM and 25nM dasatinib pretreatments, adjusting its concentration to 10nM is also sufficient to inhibit the activation of NK cells by positive K562 target cells to release CD107a (Fig. 26B, the first row, the third column to the fifth column). In this experiment, 10nM almost completely inhibited the activation of NK.

Summarizing the above two experiments, it is basically proved that the inhibition of NK cell activation function by dasatinib has an extended effect. Dasatinib can inhibit the activation function of NK cells with the dynamic concentration changes in vitro as the concentration was reduced from 50nM to 10nM or from 25nM to 10nM.

### Example 14: The strategy of combining LCK^{T316I} mutation with dasatinib gives CD5-UCART advantages in expansion and survival in the in-vitro mixed lymphocyte reaction at a 5:1 effector-to-target ratio

In-vitro mixed lymphocyte reaction was carried out using enough CD5-UCART and BCMA-UCART prepared, so as to simulate whether the combination of LCK-T316I-UCART (hereinafter referred to as Km-UCART) and dasatinib can obtain living space and even proliferation advantages in the host T environment.

The present invention relates to the preparation of universal CAR-T cells by knocking out the TRAC gene. Given that the CD5 antigen is expressed on chronic B lymphocytic leukemia cells, the universal CAR-T cells involved in the following embodiments target CD5. However, it is known that all peripheral blood T cells and a very small part of B cells also express CD5, and UCART cells targeting CD5 will recognize and kill autologous or allogeneic T cells. Therefore, the present invention uses gene editing technology to simultaneously knock out the CD5 gene and the TRAC gene in T cells to prepare CD5-UCART cells.

CD5-UCART cells were prepared from healthy donors in a small number. In the cell samples prepared in this batch, the inventors measured by flow cytometry that the knockout efficiency of TRAC gene was >90%; the knockout efficiency of CD5 gene was >99%; and the T cells expressing CAR were about 35% as determined by detecting the EGFR transduction marker. The inventor carried out further gene editing on the successfully constructed CD5-UCART cells that can be used for in vitro testing. This gene editing used the CBE3 base editor to cause the T316I mutation at the LCK site on the CD5-UCART cells: the CBE3 group was CDS-UCAT cell control group; the sg12 group was the CD5-UCAT cell control group with nonsense mutation of LCK; the sg16 group (Km group) was the CDS-UCAT cell experimental group with LCK-T316I edited.

The present invention is intended to carry out LCK-T316I on UCART cells to construct UCART tolerant to the inhibition of dasatinib, then in the host T cell environment where dasatinib exists, the host T cells have their cytotoxicity (almost) completely inhibited as they do not receive genetic engineering for tolerating the inhibitory effect of dasatinib. At this time, the UCART in the present invention can get rid of the immune rejection originally mediated by the host T cells and maintain a normal proliferation rate.

The inventors used a mixed lymphocyte reaction (MLR) model to evaluate the ability of Km-CD5-UCART cells to resist immune rejection mediated by T cells in vitro and to clear allogeneic T cells (host T cells are also the target cells of CD5-UCART cells because of the absence of endogenous CD5 gene knockout).

The method comprises the steps of:
(1) Sorting and activation of CD3+ T cells;
(2) CRISPR-Cas9 and sgRNA-mediated knockout of TRAC and CD5 genes;
(3) Lentivirus infection of TRAC/CD5 knockout T cells;
(4) Detection of TRAC and CD5 gene knockout efficiency: the results are shown in Fig. 27A, the knockout efficiency of TRAC gene is >90%, and the knockout efficiency of CD5 gene is >99%;
(5) Detection of CAR positive rate: 5 days after T cells were infected with lentivirus, the CAR positive rate was detected by flow cytometry. The results are shown in Fig. 27B. The percentage of T cells expressing CAR is about 35%-40% as measured by using two different markers. At that time, the inventors believed that CD5-UCART cells that can be used for in vitro testing have been obtained;
(6) T316I editing (Km) of LCK gene mediated by CBE3 and sgRNA;
(7) Detection of Km editing efficiency in CD5-UCART cells: as shown in Fig. 27C, Km editing did not occur in CBE3 group and sg12 group; T316I mutation efficiency in CD5-UCART cells in sg16(Km) group was 79%, that is, theoretically, 79% of CD5-UCART cells could be tolerant to the inhibitory effect of dasatinib;
(8) Acquisition of simulated host T cells in in-vitro experiments: In order to test the function of Km-CD5-UCART in the environment of allogeneic T cells, the inventors also need to obtain donor-derived T cells different from the HLA-ABC protein of the donor involved in the preparation of Km-CD5-UCART as allogenic host T cells. The freshly sorted allogeneic host T cells were not activated with anti-CD3/-CD28 antibody magnetic beads, and were pretreated with 100 nM dasatinib, that is, dasatinib was added into the T cell complete medium before the assay started. Such treatment is designed to simulate the need to pre-treat patients with dasatinib to inhibit the effector function mediated by host T cells before using Km-UCART as a therapeutic drug;
(9) Using mixed lymphocyte reaction to test the survival of Km-CD5-UCART cells in the environment of host T cells: this mixed lymphocyte reaction aims to test the ability of Km-CD5-UCART cells to resist T cell-mediated immune rejection in vitro and the ability to clear allogeneic T cells, so the inventors regard Km-CD5-UCART as theoretical effector cells, and the allogeneic host T cells as target cells.

The effector cells in this mixed lymphocyte reaction were divided into 3 groups: (1) the CBE3 group was CDS-UCAT cell control group; (2) the sg12 group was the control group of CDS-UCAT cells with nonsense mutation of LCK; (3) the sg16 group (Km group) was the experimental group of CDS-UCAT cells with LCKT316I edited. Only allogeneic T cells from the same donor source were used as the target cells in this mixed lymphocyte reaction. Before the start of the MLR assay, dasatinib was added to the complete medium of T cells, that is, the simulated host T cells (target cells) and CD5-UCART cells (effector cells) were pretreated with dasatinib for 12hr.

This example was carried out at a 1:5 effector-to-target ratio. What needs to be explained here is that the inventors define the effector-to-target ratio as the ratio of the absolute number of CD5-UCART cells expressing CAR molecules (the CAR positive rate in this example is 35%) to the absolute number of simulated host T cells. The 3 groups of effector cells were separately plated in two wells in a flat-bottomed 96-well plate at 8.09×10⁴, 5.20×10⁴, and 1.26×10⁴ cells (100µl volume), and the target cells were plated at 1.42×10⁵, 9.10× 10⁴, 2.20×10⁴ cells (100µL volume) respectively and mixed with the effector cells, the total volume in each well was 200µL, and all were cultured in T cell complete medium. At present, there were 3 groups of mixed wells of effector cells and target cells, two wells each, one of which was used as the experimental well to which 100nM Dasatinib was added, and the other well was the control well to which the same volume of DMSO was added (the solvent for dasatinib in this example was DMSO). The plate was placed in a 37°C incubator to culture.

In this test, the inventors predict that the effector cells in the sg16 group could exhibit a growth advantage over other groups in the dasatinib treatment groups. Therefore, the inventors need to collect the following data during the mixed lymphocyte reaction: cell count at different time points; percentage and absolute number of effector cells and target cells at different time points; CAR positive rate of effector cells.

In order to obtain the above data, the inventors took part of the cells from each well on day 2 and day 10 of the co-culture (the day of plating was defined as day 0) for cell counting. It needs to determine the percentage of the cells taken out relative to the overall cells in the well, and the absolute number of cells in each well is calculated by using this percentage and the counting result of the cells taken out. The cells taken out also need to be used for flow cytometric analysis to determine the percentages of effector cells and target cells and the CAR positive rate of effector cells.

The inventor combined the results of cell counting and flow cytometry analysis at each time point to calculate the indicators of interest. Fig. 28A shows the percentage of UCAR-T in the total MLR system. In MLR reaction, the UCART cells in the DMSO group were able to kill the host T cells, and on day 10 of the test, the host T cells were reduced to less than 5%; with the treatment with 100nM dasatinib, in the control group, the killing of host T cells by UCAR-T was inhibited, and the proliferation of host T was obvious. Only Km-edited UCAR-T could resist the inhibition by dasatinib and return to the normal cytotoxicity of UCAR-T.

As for the changes of host -T in the overall system, as shown in Fig. 28B, all the UCAR-T in the DMSO group could kill the host T cells, and the host T cells were reduced to less than 5% on D10; with the treatment with 100nM dasatinib, in the control group, the killing of host T cells by UCAR-T was inhibited, and the proliferation of host T cells was obvious. Only Km-edited UCAR-T could resist the inhibition by dasatinib and return to the normal cytotoxicity of UCAR-T.

Meanwhile, as shown in Fig. 28C, the CAR positive rate of each group gradually increased to 70% with the increase of MLR days, and there was no difference between the groups. In this test, the functional components considered by the inventors, that is, CAR positive cells were not enriched faster than in the control group due to the combination of Km editing and dasatinib. In this test, the real-time effector-to-target ratio is helpful to reflect the dynamic relationship between the effector components and the simulated host T cells in the system. As shown in Fig. 28D, the Km-CD5-UCART group with dasatinib treatment showed a continuous increase in the effector-to-target ratio, whereas there was no such phenomenon in other control groups. Therefore, the inventors confirmed that dasatinib can inhibit the clearance of host T cells by CD5-UCART, and the KT316I mutation can effectively restore the function of UCAR-T.

### Example 15: The strategy of combining LCK^{T316I} mutation with dasatinib gives CD5-UCART advantages in expansion and survival in the in-vitro mixed lymphocyte reaction at a 50-100:1 effector-to-target ratio

This example is a supplement and optimization of Example 14. With the more extreme amount of allogeneic T, it aims to evaluate the ability of Km-CD5-UCART cells to resist T cell-mediated immunological rejection in vitro and the ability to clear allogeneic T cells through the mixed lymphocyte reaction (MLR) model.

The method comprises the steps of:
(1) Sorting of CD3+ T cells;
(2) TRAC, CD5 gene knockout, and T316I editing of LCK gene (Km) in unactivated T cells;
(3) Activation of T cells after electroporation;
(4) Lentivirus infection of T cells;
(5) TRAC and CD5 gene knockout efficiency, as shown in Fig. 29A; the knockout efficiency of TRAC gene is >95%, and the knockout efficiency of CD5 gene is >99.5%;
(6) Detection of CAR positive rate, about 30% as shown in Fig. 29B;
(7) Detection of the efficiency of Km editing, as shown in Fig. 29C: the Ctrl group has no Km editing; the mutation efficiency of T316I in CD5-UCART cells in the Km group is 45%. At that time, the inventors believed that Km-CD5-UCART cells that can be used for in vitro testing have been obtained;
(8) Acquisition of simulated host T cells in in-vitro experiments: In order to test the function of Km-CD5-UCART in the environment of host T cells, the inventors also needed to obtain donor-derived T cells different from the HLA-ABC protein of the donor involved in the preparation of Km-CD5-UCART as host T cells. For the acquisition of this batch of T cells, refer to step (1) in this example, and the cell purity is also checked by flow cytometry. The freshly sorted host T cells were not activated with anti-CD3/-CD28 antibody magnetic beads, and were pretreated with 100 nM dasatinib, that is, dasatinib was added into the T cell complete medium before the assay started. Such treatment is designed to simulate the need to pre-treat patients with dasatinib to inhibit the effector function mediated by host T cells before using Km-UCART as a therapeutic drug.
(9) Using mixed lymphocyte reaction to test the survival of Km-CD5-UCART cells in the environment of host T cells: this mixed lymphocyte reaction aims to test the ability of Km-CD5-UCART cells to resist T cell-mediated immune rejection in vitro and the ability to clear host T cells, so the inventors regard Km-CD5-UCART as theoretical effector cells, and the host T cells of the host as target cells.

The effector cells in this mixed lymphocyte reaction were divided into 2 groups: (1) the Ctrl group was the control group of CDS-UCAT cells with nonsense mutation of LCK; (2) the Km group was the experimental group of CDS-UCAT cells with LCKT316I editing. Only homologous host T cells from the same donor source were used as the target cells in this mixed lymphocyte reaction. Before the start of the MLR assay, dasatinib was added to the complete medium of T cells, that is, the simulated host T cells (target cells) and CD5-UCART cells (effector cells) were pretreated with dasatinib for 24hr.

This example was planned to be carried out with an effector-to-target ratio of 1:50-100. The 2 groups of effector cells were separately plated in 6 wells in a flat-bottomed 96-well plate at 2.5×10⁵ (250µl volume), and the target cells were plated at 1.5×10⁶ cells (250µL volume) respectively and mixed with the effector cells, the total volume in each well was 500µL, and all were cultured in T cell complete medium. There were currently 2 groups of mixed wells of effector cells and target cells, 6 wells for each, and 3 treatments were given respectively, and 2 technical replicates were set for each treatment: treatment 1 was DMSO, treatment 2 was 25nM dasatinib, treatment 3 was 50nM dasatinib. The plate was placed in a 37°C incubator to culture. Dasatinib and DMSO in the culture system were supplemented every two days.

In this test, the inventor predicted that the effector cells in the Km group could show a growth advantage over other groups in the dasatinib treatment groups, and based on the data obtained in the above examples, the inventor speculated that the Km group treated with 50nM dasatinib has more growth advantages than the Km group treated with 25nM dasatinib, that is, the growth advantage obtained by Km-CD5-UCART under dasatinib treatment was dose-dependent on dasatinib. To verify the above hypothesis, the inventors needed to collect the following data during the mixed lymphocyte reaction: cell count at different time points; percentage and absolute number of effector cells and target cells at different time points; CAR positive rate of effector cells.

In order to obtain the above data, the inventors took part of the cells from each well on day 2, 5, 8, 11 and 13 of the co-culture (the day of plating was defined as day 0) for cell counting. It needs to determine the percentage of the cells taken out relative to the overall cells in the well, and the absolute number of cells in each well is calculated by using this percentage and the counting result of the cells taken out. The cells taken out also need to be used for flow cytometric analysis to determine the percentages of effector cells and target cells and the CAR positive rate of effector cells.

In this mixed lymphocyte reaction, the inventor combined the results of cell counting and flow cytometry analysis at each time point to calculate the indicators of interest. The actual number of plated cells on day 0 is obtained by cell counting and flow cytometry after plating, not by the amount of cells to be added in the experimental design. Because the error caused by adding samples will cause the actual plated volume to be inconsistent with the planned plated cell volume, and secondly, because technical repetitions are set, counting the actual plated volume makes it easier to perform biostatistical analysis on the results. The actual CAR positive rate of effector cells was about 10% during the test, and the actual effector-to-target ratio obtained by combining the results of cell counting and flow cytometry was 1:50-1:100. After the test, according to the indicators obtained at each time point, the inventors carried out exhaustive statistical analysis on the dynamic changes in the number of effector cells (Fig. 30A), the dynamic changes in the number of target cells (Fig. 30C) and the positive CAR rate in effector cells (Fig. 30E), respectively.

As shown in Fig. 30A, with DMSO treatment, neither Ctrl-CD5-UCART nor Km-CD5-UCART can effectively expand in the environment of host T cells, suggesting that host T cells are activated by UCART cells and exert their cytotoxicity to gradually clear UCART cells. With the treatment with 25nM dasatinib, the proliferation of Ctrl-CD5-UCART in the control group was inhibited, while Km-CD5-UCART could expand effectively, suggesting that the function of host T cells was inhibited by dasatinib, while due to tolerance to the inhibition by dasatinib, Km-CD5-UCART could obtain normal proliferation and effectively kill host T cells. The trend obtained with 50nM dasatinib treatment was consistent with that of 25nM treatment group. As of day 11 of the test, compared with 25nM treatment, Km-CD5-UCART with 50nM dasatinib treatment obtained a more obvious sustained proliferation advantage, suggesting that 50nM is more effective in inhibiting the activation of host T cells, thus more significantly reducing the survival pressure of Km-CD5-UCART in the environment of host T cells.

In addition, from the analysis of the percentage of effector cells relative to the overall cells, as shown in Fig. 30B, the percentages in the Km-CD5-UCART culture system with dasatinib treatment all increased significantly over time, while the other groups were on the contrary, suggesting that Km-CD5-UCART can tolerate the inhibition of its activation by dasatinib, and they can still expand effectively in the environment of high-fold host T cells. Also, the proliferation advantage of Km-CD5-UCART in the 50nM dasatinib treatment group was more sustainable than that in the 25nM dasatinib treatment group. These data validate the inventor's hypothesis that the growth advantage obtained by Km-CD5-UCART with dasatinib treatment is dose-dependent on dasatinib.

Regarding the dynamic changes of host T cells, as shown in Fig. 30C, the number of host T cells in each group showed dynamic changes, and did not show different changing rules between groups. However, from the analysis of the percentage of host T cells (Fig. 30D), it is not difficult to find that as of day 11 of the test, the percentage of host T cells in the Km-CD5-UCART group continued to decrease with dasatinib treatment, whereas the percentage of host T cells in the control group remained stable for a long time. The inventors analyzed that the cytotoxicity of the host T cells was (almost) completely suppressed because they had not been genetically engineered to be tolerant to the inhibition of dasatinib, and Km-CD5-UCART became the main functional component in the culture system because of its tolerance to the inhibition by dasatinib, and could continuously clear the host T cells, resulting in the decreasing percentage of the host T cells. This result validated the inventor's hypothesis from another perspective, that is, the combination of Km-CD5-UCART and dasatinib can help UCART cells overcome the immune clearance mediated by host T cells, thus providing a prerequisite for UCART to perform tumor cytotoxicity.

Fig. 30E revealed that the absolute number of CAR-positive cells in UCART cells continued to increase in the dasatinib-treated Km-CD5-UCART group, while those in Ctrl-CD5-UCART and DMSO treatment groups showed the same trend and continued to decrease. It suggests that the main functional population in the Km-CD5-UCART group treated with dasatinib was CAR-positive cells, and the combination of Km editing and dasatinib gave CAR-positive cells a survival advantage and enrichment. The CAR positive rate was significantly increased only in the Km-CD5-UCART of the dasatinib treatment group, and there was no significant increase in the CAR positive rate in the Ctrl-CD5-UCART and DMSO treatment groups. From Day 8 of the test, the percentage of CAR positive cells in all groups began to decrease, suggesting exhausted function of CART cells.

The inventors believed that in this test, the real-time effector-to-target ratio is helpful to reflect the dynamic relationship between the effector components and the simulated host T cells in the system. As shown in Fig. 30F, the Km-CD5-UCART group with dasatinib treatment showed a continuous increase in the effector-to-target ratio, whereas there was no such phenomenon in other control groups. It is suggested that in a high-fold allogeneic T cell environment (even if starting with effector-to-target ratio of 1:50-1:100), Km-CD5-UCART cells can still continuously reduce the effector-to-target ratio when used in combination with dasatinib. The acquisition of this data made the inventors have great confidence in the effective survival and proliferation of Km-UCART in vivo.

In addition, by further analyzing the genome sequences of each group of cells in the mixed lymphocyte reaction on D11, the inventors amplified the LCK-T316 region of the remaining cells on D11 in the entire reaction, and analyzed the enrichment level of LCK-T316I mutation of the remaining cells by Sanger sequencing. The results are shown in Fig. 31A. No matter whether treated with dasatinib or not, the CD5-UCART cells not edited by LCK-T316I still did not show any enrichment of T316I mutation on D11 of the MLR reaction (Fig. 31A, the blue upper six rows). In CD5-UCART cells with LCK-T316I mutation, when the MLR reaction was treated with DMSO, there was no enrichment of T316I mutation; when treated with 25nM dasatinib, more than 23% T316I mutation occurred; when the concentration was further increased to 50nM, the percentage of mutation enrichment rose to more than 43% (Fig. 31A, the lower six rows). The statistical data are shown in Fig. 31B. These data demonstrate that dasatinib can inhibit the activation of allogeneic T (host T) cells, while Km-edited UCAR-T cells are tolerant to the inhibitory effect of dasatinib.

In summary, the inventors confirmed that in order to prevent UCART from immune rejection mediated by host T cells, it is feasible to combine Km editing with dasatinib.

### Example 16: Ponatinib (PN) with a concentration of 200nM can effectively inhibit the activation of T cells in vitro

The previous examples basically clarify the strategy and principle of the invention. There are two core inventions: (1) special treatment with drugs: the inventors introduce the tyrosine kinase inhibitor dasatinib into the universal CAR-T pretreatment in that dasatinib inhibits the activation of host T and NK cells, enabling the universal CAR-T to survive in the host cell environment; (2) introduction of LCK-T316I mutation: the inventors use the single base editing technology A3A-CBE3 system to introduce LCK-T316I (Km) mutation into universal CAR-T cells, making them tolerant to inhibition of CAR signaling by dasatinib. Finally, with dasatinib treatment, Km-edited UCAR-T can be normally activated to target and kill tumor cells, and its clearance by host T and NK cells is also avoided.

The tyrosine kinase inhibitor (TKI) used in the present invention is not only dasatinib. The inventors found that ponatinib can also be used as a special treatment drug to realize the universal CAR-T strategy in the present invention.

In the study of ponatinib, the inventors first used the Miltenyi CD3-positive magnetic bead sorting kit to obtain CD3-positive T cells from donor PBMCs, and identified the purity and efficiency of the sorted CD3-positive cells by a flow cytometer. Then, the sorted CD3-positive T cells were divided into three groups, namely the control group electroporated with CBE3 protein (CBE3), the nonsense mutation control group electroporated with the complex of CBE3 protein and sgRNA12 (Sg12), and the LCK-T316I mutation group electroporated with the complex of CBE3 and sgRNA16 (Sg16-Km). The inventors used the Lonza electroporation instrument to carry out single-base editing on the LCK gene. The editing efficiency is shown in Fig. 32A. The LCK-T316I mutation efficiency in the Sg16-Km group was 30%, while the other two groups of control cells did not carry this mutation (Fig. 32A).

The experimental procedure is shown in Fig. 32B. The inventors divided each of the three groups of edited cells into 6 sub-groups and cultured them in a flat-bottomed 96-well plate. Each sub-group contained two technical replicates, and each well contained 0.15 × 10⁶ cells. The edited cells in each group were first pretreated with ponatinib for 2hr, and the corresponding concentrations of PN in the groups were 0, 10nM, 50nM, 100nM and 200nM, respectively (Fig. 32B). Two hours later, an appropriate amount of anti-CD3/CD38 DynaBeads was taken to activate the cells of each group, while a blank control group treated with 0nM ponatinib and not activated by magnetic beads was retained. Subsequently, the cells were cultured for 18hr. The activated T cell surface marker molecule 4-1BB was detected by using the APC-labeled murine IgG monoclonal antibody, and the activated T cell surface marker molecule CD25 was detected by using the PE-labeled murine IgG monoclonal antibody, and the activated T cell surface marker CD69 was detected by FITC-labeled murine IgG monoclonal antibody.

The results of flow cytometry (Figs. 32C, D) showed that ponatinib treatment at a concentration of 100nM and below was not sufficient to inhibit the TCR signal activation of T cells in the normal control group, and the expression of 4-1BB and CD69 was still induced, indicating that ponatinib at concentrations of 100nM and below cannot inhibit T cell activation. However, with 200nM ponatinib treatment, 1.00% and 1.85% of the cells in the CBE3 and Sg12 control groups expressed CD25 and 4-1BB double-positive molecules, respectively, while 24.1% of the cells in the Km group were double-positive (Fig. 32C); meanwhile, 5.59% and 5.31% of the cells in the CBE3 and Sg12 control groups expressed CD25 and CD69 double-positive molecules, respectively, while 17.7% of the cells in the Km group were double-positive cells (Fig. 32D). Based on the above, the inventors confirmed the inhibitory function of ponatinib on T cell activation. At the same time, the inventors proved that the Km-edited T cells (carrying LCK-T316I mutation) can tolerate the inhibition of T cell activation by 200nM ponatinib. Therefore, by selecting an appropriate concentration (>200nM) of ponatinib for pretreatment, the inhibition of normal TCR signal activation (inhibition of host T cell function) can be achieved, and UCAR-T cells with LCK-T316I mutation can tolerate the inhibition of its activation by ponatinib at this concentration.

In order to further determine the concentration range in which ponatinib inhibits T cell activation in vitro and the inhibition is tolerant for the T316I mutation, the inventors tested the inhibition of T cell activation with treatment with higher concentrations of ponatinib.

The inventors conducted research using two groups of T cells (CBE3 control group and Km edited group) in the above experiments. The experimental procedure is basically the same as the above-mentioned experiment (Fig. 32B). The two groups of edited cells were each divided into 7 sub-groups and cultured them in a flat-bottomed 96-well plate. Each sub-group contained two technical replicates, and each well contained 0.15 × 10⁶ cells. The edited cells in each group were first pretreated with ponatinib for 2hr, and the corresponding concentrations of PN in the groups were 0, 10nM, 50nM, 200nM, 500nM and 1000nM, respectively (Figs. 33A, B). Two hours later, an appropriate amount of anti-CD3/CD38 DynaBeads was taken to activate the cells of each group, while a blank control group treated with 0nM ponatinib and not activated by magnetic beads was retained. Subsequently, the cells were cultured for 18hr. The activated T cell surface marker molecule 4-1BB was detected by using the APC-labeled murine IgG monoclonal antibody, and the activated T cell surface marker molecule CD25 was detected by using the PE-labeled murine IgG monoclonal antibody, and the activated T cell surface marker CD69 was detected by FITC-labeled murine IgG monoclonal antibody.

The results of flow cytometry (Figs. 33A, B) showed that ponatinib treatment at a concentration of 50nM and below was not sufficient to inhibit the TCR signal activation of T cells in the normal control group, and the expression of 4-1BB and CD69 was still induced, indicating that ponatinib at concentrations of 50nM and below cannot inhibit T cell activation. However, consistent with the above experiments, with 200nM ponatinib treatment, almost no cells in the CBE3 control group were CD25/4-1BB double-positive, while 19.71% of the cells in the Km group still expressed CD25 and 4-1BB molecules (Fig. 33A, the fifth column); at the same time, almost no cells in the CBE3 control group were CD25/CD69 double-positive, while 23.9% of the cells in the Km group still expressed CD25 and CD69 molecules (Fig. 33B, the fifth column). The results are completely consistent with the previous experiment.

In addition, with the treatment with 500nM ponatinib, the activation of T cells in the CBE3 control group was completely inhibited, and only 2.42% of CD25 and 4-1BB double-positive cells and 6.75% of CD25 and CD69 double-positive cells were found in the Km edited group (Figs. 33A, B, the sixth column). It is suggested that the ability of T cells with LCK-T316I mutation to be activated is significantly weakened with the treatment with 500nM ponatinib, proving that Km editing can not effectively tolerate the inhibition of TCR signals by 500nM ponatinib. With the treatment with 1000nM ponatinib, the activation of T cells in both the CBE3 control group and the Km edited group were completely inhibited (Figs. 33A, B, the seventh column), and the number of cells was significantly reduced, suggesting that 1000nM ponatinib had a great influence on the survival of T cells, and the LCK-T316I mutation cannot effectively tolerate the inhibition of TCR signals by 1000nM ponatinib.

Based on the above, the inventors believed that 200nM ponatinib can effectively inhibit the activation of T cells. For T cells with LCK-T316I mutation, the mutation can tolerate the inhibition of T cell activation by ponatinib at a concentration of 200nM, but when the concentration of ponatinib rises to 500nM, this tolerance is significantly reduced. Ponatinib at 500nM can block TCR signal activation in Km-edited T cells. Therefore, ponatinib can be used as a pretreatment solution for the universal CAR-T strategy in the present invention, and the function of ponatinib at a concentration of 200 nM is consistent with that of dasatinib, i.e. inhibiting host T from killing UCAR-T while ensuring that LCK -T316I-UCAR-T cells still have normal functions, and can be activated and mediate the killing of targeted tumor cells. When the concentration of ponatinib increases to 500nM, it can act as a molecular switch to inhibit the activation of LCK-T316I-UCAR-T cells.

### Example 17: Ponatinib at a concentration of 200nM can effectively inhibit the activation of NK cells in vitro

In the above examples, the inventors proved that ponatinib at a concentration of 200nM can effectively inhibit the activation of T cells. The inhibition of NK cell activation by ponatinib and the concentration threshold are further explored, to determine that ponatinib can effectively inhibit the activation of both T cells and NK cells and determine the concentration threshold.

The inventors used CD3-negative cells in human PBMCs, which contained more than 5% of CD56-positive NK cells. The inventors used treatments with ponatinib of four concentrations of 0, 50nM, 200nM and 500nM respectively to study the effects of different concentrations on NK activation. The procedure is as follows: The cells were treated with different concentrations of ponatinib overnight. The next day, the cells were resuspended with NK medium and activated with K562. Lymphocyte activator was used as a positive control for activation, and a part of NK cells that were not activated by K562 were also kept. Two replicates were set for each group. anti-CD107a antibody and monensin were added while carrying out activation, and after co-incubation for 6hr, the CD107a release of CD56-positive NK cells was detected by flow cytometry.

As shown in Fig. 34A, without ponatinib treatment, the CD107a translocation of NK cells added with lymphocyte activator was more than 96% (Fig. 34A, the upper row, the second column), and the activation of NK cells by K562 also reached more than 77% (Fig. 34, the upper row, the third column). As the concentration of ponatinib increased, the percentage of NK cells activated by K562 to express CD107a gradually decreased. When the concentration of ponatinib reaches 200nM, K562 was basically unable to activate NK cells to promote the release of CD107a (Fig. 34A, the lower row, the second column), proving that 200nM ponatinib can effectively inhibit the activation of NK cells in vitro. As shown in Fig. 34B, the inventors analyzed the significance of the above experimental data using the histogram, and basically confirmed that compared with the treatment group without ponatinib, the concentration of 200nM can significantly inhibit the activation of NK cells.

Therefore, the above data further show that ponatinib can be used as the pretreatment protocol of the universal CAR-T strategy in the present invention, and ponatinib at a concentration of 200nM can inhibit the activation of T and NK cells, thus avoiding the killing of UCAR-T by host T and NK cells. At the same time, the LCK-T316I mutation can make UCAR-T cells tolerant to the inhibition of T cell activation by ponatinib, and can be activated normally and mediate the killing of targeted tumor cells.

### Example 18: The inhibition of T cell activation by ponatinib in vitro has an extended effect

In Example 16, the inventors proved that ponatinib at a concentration of 200nM can effectively inhibit the activation function of T cells in vitro, so as to guide the concentration used in vivo. Considering the drug metabolism of ponatinib in vivo, the inventors designed a dynamic concentration experiment of ponatinib in vitro to detect whether its inhibition of T cell activation is still effective with dynamic concentration changes.

In this example, the prepared wild-type anti-BCMA CAR-T cells were used for experiments. Treatment with ponatinib at a concentration of 0nM, 200nM or 500nM was carried out, respectively, and the concentration was decreased the next day. In Fig. 35A, the 0nM pretreatment group needed not to reduce the concentration any more, the concentration was adjusted to 0, 50, 100 and 200nM after 200nM pretreatment overnight, and the concentration was adjusted to 0, 50, 100, 200nM and 500nM after 500nM pretreatment overnight. At the same time, RPMI-8226 cells expressing BCMA antigen were used as positive target cells. When the concentration was adjusted on the next day, the CAR-T cells and target cells were co-incubated at an effector-to-target ratio of 1:1, and the control was blank medium without target cells. CD107a and monensin were added at the same time. After 4hr of co-incubation, CD107a release in CD8 and CAR double-positive cells was detected.

The results are shown in Fig. 35. CAR-T cells in the non-ponatinib treatment group were activated by RMPI-8266 target cells, and about 40% of the cells released CD107a, proving that CAR-T cells can be effectively activated by positive target cells (Fig. 35A, the first and second rows, the upper and lower are two parallel replicate wells respectively).

After pretreatment with 200nM ponatinib, the concentration was adjusted to 0nM the next day, and the percentage of CD107a positive cells was reduced from about 40% to about 16%, which proved that the inhibition of T cell activation by ponatinib has an extended effect (Fig. 35A, the third and fourth rows, the leftmost column). After pretreatment with 200nM ponatinib, the concentration was adjusted to 50nM or higher the next day, and the percentage of CD107a positive cells decreased to the same level as the negative control group, suggesting that when the concentration of ponatinib is reduced from 200nM to 50nM, it can completely inhibit the activation of T cells (Fig. 35A, the third and fourth rows, the last three columns).

After pretreatment with 500nM ponatinib, the concentration was adjusted to 0nM the next day, and the percentage of CD107a positive cells was reduced from about 40% to about 3%, which proved once again that the inhibition of T cell activation by ponatinib has an extended effect, and the higher the concentration of pretreatment, the more obvious the extended effect of inhibition (Fig. 35A, the fifth and sixth rows, the leftmost column). After pretreatment with 500nM ponatinib, the concentration was adjusted to 50nM or higher the next day, and the percentage of CD107a positive cells decreased to the same level as the negative control group, suggesting that when the concentration of ponatinib is reduced from 500nM to 50nM, it can also completely inhibit the activation of T cells (Fig. 35A, the fifth and sixth rows, the last three columns).

As shown in Fig. 35B, the inventors performed a statistical analysis on the above data, proving that compared with the cells in the non-ponatinib treatment group, for CAR-T cells pretreated with 200nM ponatinib and then treated with ponatinib adjusted to 0nM for 4hr, the positive rate of CD107a released by the cells activated by the target cells was significantly reduced, and the CAR-T cells with the concentration of ponatinib adjusted to 50nM or above later could not be activated by the target cells within 4hr. Meanwhile, 500nM pretreatment basically completely inhibited the activation of CAR-T cells by target cells. The above data show that the inhibition of T cell activation by ponatinib has an extended effect, and the higher the drug concentration, the more obvious the extended effect.

### Example 19: The inhibition of NK cell activation function by ponatinib in vitro has an extended effect

In Example 17, the inventors proved that ponatinib at a concentration of 200nM can effectively inhibit the activation function of NK cells in vitro, so as to guide the concentration used in vivo. In order to fully consider the fluctuations in the concentration of ponatinib in vivo, the inventors designed a dynamic concentration experiment of ponatinib in vitro to detect whether its inhibition of NK cell activation is still effective with dynamic concentration changes.

The inventors isolated NK cells from human PBMCs, and then cultured and differentiated them in NK medium to obtain CD56-positive NK cells with a purity higher than 95%. Four pretreatments were done respectively: (1) absence of ponatinib pretreatment; (2) pretreatment with 50nM ponatinib; (3) pretreatment with 100nM ponatinib; (4) pretreatment with 200nM ponatinib, and the cells were cultured overnight. The next day, the concentration of ponatinib was reduced, and the concentration was adjusted to 0, 50, 100 and 200nM respectively. At the same time, K562 cells were used as positive target cells to activate NK cells, the cells were co-incubated at an effector-to-target ratio of 1:1, and CD107a and monensin were added at the same time. Two replicates were set for each group. After 5hr of co-incubation, CD107a release in CD56 positive NK cells was detected.

As shown in Fig. 36A, after pretreatment with four different concentrations of ponatinib, when the concentration was lowered to 0nM, NK cells could be significantly activated by K562, and the release of CD107a gradually decreased with the increase of pretreatment concentration, with the strongest being above 58% (Fig. 36A, the first and second rows, the second column) and the weakest being above 38% (Fig. 36A, the seventh and eighth rows, the second column). It proves that K562 can activate NK, the inhibition of NK activation by ponatinib has an extended effect, and the higher the concentration, the more obvious the extended effect. After pretreatment with four different concentrations of ponatinib, the concentration was adjusted to four concentrations of 0, 50, 100 and 200nM the next day. It can be found that with the increase of the concentration the next day, the percentage of NK cells activated by K562 to release CD107a in each group decreased gradually. Also, after 200nM ponatinib pretreatment, it needs to maintain its concentration to effectively inhibit the activation of NK. It proves that the inhibition of NK by ponatinib still requires maintaining a high concentration level.

As shown in Fig. 36B, the inventors analyzed the significance of the above experimental data using the histogram, and further clarified that with pretreatments with different concentrations, which were adjusted to a consistent concentration for 4hr, the ability of NK cells to be activated by K562 decreased with the increase of pretreatment concentration, proving that the inhibition of NK cell activation by ponatinib has an extended effect. Also, ponatinib needs to be maintained at 200nM to effectively inhibit NK activation.

### Example 20: Verifying the possibility of ponatinib as a combined drug and switch for universal CAR-T therapy of the present patent

In Example 16, the inventors proved that ponatinib at a concentration of 200nM can effectively inhibit the activation function of T cells in vitro, and for T cells with LCK-T316I mutation, the mutation can tolerate the inhibition of T cell activation by ponatinib at the concentration of 200nM, so as to guide the concentration used in vivo. When the concentration of ponatinib increases to 500nM, ponatinib can block TCR signal activation in Km-edited T cells. Therefore, it can act as a molecular switch to inhibit the activation of LCK-T316I-UCAR-T cells. In Example 18, the inventors proved that with dynamic concentration changes, the inhibition of T cell activation by ponatinib has an extended effect, and the higher the drug concentration, the more obvious the extended effect. However, when the concentration of ponatinib increases to 500nM, this tolerance is significantly reduced.

In order to verify that LCK-T316I mutation can not only make T cells tolerant to the inhibition of TCR activation by ponatinib, but also make CAR-T cells tolerant to the inhibition of CD107a translocation by ponatinib during CAR activation, and at the same time, fully considering the fluctuation of ponatinib concentration in vivo, the inventors designed and carried out the following experiments to prove that CD19-CAR-T cells with LCK-T316I mutation can tolerate the inhibition of CD107a translocation by ponatinib at a concentration of 200nM. However, when the concentration of ponatinib increased to 500nM, ponatinib can also block the CD107a translocation of CD19-CAR-T cells with LCK-T316I mutation. Therefore, the combination of LCKT316I mutation and ponatinib can be used as a switch for CD19-UCART therapy.

In this example, the inventors used the prepared CD19-UCART and Km-edited CD19-UCART cells in the experiments. Cells in both groups were treated with 200nM or 500nM ponatinib overnight (Figs. 37A and 37B). The next day, the concentration was adjusted to 0, 50, 100 and 200nM in the group of 200nM pretreatment overnight, and the concentration was adjusted to 0, 100, 200nM and 300nM in the group of 500nM pretreatment overnight. At the same time, Raji cells expressing CD19 antigen were used as positive target cells, the CD19-UCART cells and target cells were co-incubated at an effector-to-target ratio of 1:1, and the control was blank medium without target cells. anti-CD107a antibody and monensin were added at the same time. Two technical replicates were set for each group. After 4hr of co-incubation, CD107a release in CD8 and CAR double-positive cells was detected.

As shown in Fig. 37A, when the cells were pretreated overnight with 200nM ponatinib, the concentration was adjusted to 0, 50, 100 and 200nM the next day, and after co-incubation with Raji positive target cells expressing CD19 antigen for 4hr, the percentage of CD107a-positive cells in the CD19-UCART group decreased from 17.2% (the percentage of CD107a-positive cells was the average of two technical replicate groups) to 0.735%, and the percentage of CD107a-positive cells in the Km-edited CD19-UCART group decreased from 16.6% to 5.12%. When ponatinib was 50 nM, the translocation of CD107a in the CD19-UCART group was basically inhibited (0.485%), but the percentage of CD107a positive cells in the Km-edited CD19-UCART group was 12.7%. This group of experiments demonstrated that CD19-UCART cells with LCK-T316I mutation are tolerant to the inhibition of CD107a translocation by ponatinib at a concentration of 200 nM during CAR activation. Moreover, with the dynamic concentration change, the ability of CD19-UCART cells with LCK-T316I mutation to tolerate the inhibition of CD107a translocation by ponatinib during CAR activation has an extended effect.

As shown in Fig. 37B, when the cells were pretreated overnight with 500nM ponatinib, the concentration was adjusted to 0, 100, 200 and 300nM the next day, and after co-incubation with Raji positive target cells expressing CD19 antigen for 4hr, the percentage of CD107a-positive cells in the CD19-UCART group decreased from 1.73% to 0.495%, and the percentage of CD107a-positive cells in the Km-edited CD19-UCART group decreased from 11.15% to 1.47%. Moreover, in the Km-edited CD19-UCART group pretreated with 500nM ponatinib overnight, with the dynamic concentration change, the ability of CD19-UCART cells with LCK-T316I mutation to tolerate the inhibition of CD107a translocation by ponatinib during CAR activation still has an extended effect. Although the higher the drug concentration, the lower the tolerance (the percentage of CD107a-positive cells in the Km-edited CD19-UCART group was only 1.47% with treatment with ponatinib at a concentration of 300nM). This group of experiments demonstrated that the tolerance of CD19-UCART cells with LCK-T316I mutation to the inhibition of CD107a translocation by ponatinib at a concentration of 500 nM during CAR activation was significantly reduced.

To sum up, the inventors proved that CD19-UCART cells with LCK-T316I mutation are tolerant to the inhibition of CD107a translocation by ponatinib at a concentration of 200 nM during CAR activation. Moreover, with the dynamic concentration change, the ability of CD19-UCART cells with LCK-T316I mutation to tolerate the inhibition of CD107a translocation by ponatinib during CAR activation has an extended effect. However, pretreatment with ponatinib at a concentration of 500nM basically completely inhibits the activation of CD19-UCART cells by target cells, while CD19-UCART cells with LCK-T316I mutation are basically unable to be activated by Raji target cells with pretreatment with ponatinib at a concentration of 300nM. Therefore, the inventors believe that ponatinib at a concentration of 200nM and LCK-T316I mutation can be combined for the activation of CD19-UCART, while ponatinib at a concentration of 500nM can shut down the activation of CD19-UCART with LCK - T316I mutation.

### Example 21: The strategy of combining LCKT316I mutation and dasatinib makes B2M-knockout CD19-UCART have the most advantages in expansion and survival in in-vitro mixed lymphocyte reaction.

Considering that it has been proved in the above examples that dasatinib can effectively inhibit the activation of T cells and NK cells, and CAR-T cells carrying LCK - T316I mutation can effectively resist the inhibition of the activation of CAR molecules by dasatinib, so as to normally target tumor cells for activation and killing. Moreover, the inventors have demonstrated in Examples 14 and 15 that the version of CD5-UCART with B2M not knocked out can effectively resist the killing by allogeneic T cells in the in-vitro mixed lymphocyte reaction with allogeneic T cells to gain the advantage of expansion. Therefore, in order to further verify whether the B2M-knockout version of UCART can effectively resist the clearance by NK cells and gain the advantage of expansion based on the combination with dasatinib, the inventors designed the following experiments.

The inventor isolated CD3-positive T cells from PBMCs, knocked out B2M and TRAC genes after activation, and infected them with anti-CD19 CAR molecule through lentivirus infection (see SEQ ID Nos: 35-56 for specific sequences). As shown in Fig. 38A, the percentage of B2M and TRAC double knockout accounted for more than 74% (Fig. 38A, left column), and the CAR positive rate was 28.8% (Fig. 38A, right column). On Day 6 after activation, the cells were subjected to LCK-T316I editing, and the editing efficiency was over 50%.

Subsequently, the inventors used these prepared anti-CD19 UCART cells to conduct an in-vitro mixed lymphocyte reaction test. The inventors removed CD3-positive T cells from fresh PBMCs from donors different from UCAR-T, and kept CD3-removed PBMCs (mainly containing CD56-positive NK cells and CD19-positive B cells) to undergo mixed lymphoid reaction with UCART. The effector-to-target ratio (allogeneic cells: UCART) was set as four gradients of 20:1, 10:1, 5:1 and 2.5:1. There were two types of CD19 UCART cells tested: LCK edited group (Km) and LCK unedited group, which were respectively treated with DMSO or 50nM dasatinib, cultured in NK medium, and 24hr (D1), 48 h (D2) and 96hr (D4) after co-incubation, cell counting and flow cytometry for detection of the percentage of each component were carried out, and the molecules detected by flow cytometry included CD19, CAR molecules, TRAC and CD56, etc.

The results are shown in Fig. 38B. At an effector-to-target ratio of 20:1, the UCART cells in the DMSO-treated control group could not expand effectively, suggesting that CD19-UCART is activated by B cells but can not gain the advantage of expansion. The main reason is that after B2M is knocked out, activated NK cells clear it quickly (red and purple lines). CD19-UCART unedited group cells (green line) treated with dasatinib also did not expand, suggesting that dasatinib effectively inhibits the activation of CAR molecules. Only the CD19-UCART with LCK-T316I editing could obtain rapid expansion with the treatment with dasatinib, suggesting that dasatinib can not inhibit the activation of CD19-UCART with LCK-T316I mutation, and the activity of NK cells is inhibited, such UCART cells with B2M knockout can not be effectively cleared (blue line).

Similarly, as shown in Fig. 38C, the CAR positive rate of CD19-UCART was significantly increased only in Km-CD19-UCART treated with dasatinib, but there was no significant increase in the CAR positive rate in Km unedited CD19-UCART and DMSO treated groups.

In addition, as shown in Fig. 38D, the B cells in the DMSO control group could not survive, suggesting that they are killed by CD19-UCART (red and purple lines). With dasatinib treatment, the activation of unedited CD19-UCART was inhibited and B cells could not be killed, so the B cells in this group were maintained at a high level (green line). The CD19-UCART cells in the Km edited group could effectively resist inhibition by dasatinib, and kill B cells, resulting in a decrease in B cells (blue line).

Finally, as shown in Fig. 38E, the changes in the number of LCK-T316I-CD19-CAR-T cells in the LCK mutation group with the treatment with 50nM dasatinib at four different effector-to-target ratios were statistically analyzed. The results indicate that the total number of CAR-positive cells was significantly expanded at each effector-to-target ratio, and it basically shows a trend that the lower the effector-to-target ratio, the stronger the expansion. These results show that the strategy of combining LCKT316I mutation and dasatinib can make B2M-knockout CD19-UCART have the most advantages in expansion and survival in in-vitro mixed lymphocyte reaction rich in NK cells.

### Example 22: The strategy of combining LCK^{T316I} mutation and dasatinib makes CD19-UCART with both MHC class I molecules (B2M) and MHC class II molecules (CIITA) knocked out have the most advantages in expansion and survival in in-vitro mixed lymphocyte reaction.

The method comprises the steps of:
(1) Recovery and activation of T cells;
(2) TRAC, B2M and CIITA gene knockout, and T316I editing of LCK gene (Km) in activated T cells;
(3) Lentivirus infection of T cells;
(4) TRAC, B2M, CIITA gene knockout efficiency; TRAC and B2M double knockout efficiency is about 70%, and CIITA gene knockout efficiency is 50%. In order to avoid the interference of positive cells on subsequent experiments, cells were subjected to negative selection for TRAC, B2M, and CIITA , the results of cells of which are shown in Fig. 39A. The knockout efficiency of TRAC gene is >97%, the knockout efficiency of B2M gene is 97%, and the knockout efficiency of CIITA gene is >99%.
(5) Detection of CAR positive rate, >15% as shown in Fig. 39A;
(6) Detection of the efficiency of Km editing, as shown in Fig. 39B: the Ctrl group has no Km editing; the mutation efficiency of T316I in CD19-UCART cells in the Km group is 20%. At that time, the inventors believed that Km-CD19-UCART cells that can be used for in vitro testing have been obtained;
(7) Simulated host T cells in in-vitro experiments: In order to test the function of Km-CD19-UCART in the environment of host T, B, NK cells, the inventors also need to obtain donor-derived T, B and NK cells different from the HLA-ABC protein of the donor involved in the preparation of Km-CD19-UCART as host T, B and NK cells. This batch of T, B, NK cells were PBMCs.
(8) Using mixed lymphocyte reaction to test the survival of Km-CD19-UCART cells in the environment of host T and NK cells: this mixed lymphocyte reaction aims to test the ability of Km-CD19-UCART cells to resist T cell and NK cell mediated immune rejection in vitro, so the inventors regard Km-CD19-UCART as theoretical target cells. Host T cells and NK cells were used as effector cells.

The target cells in this mixed lymphocyte reaction were divided into 2 groups: (1) the Ctrl group was the control group of CD19-UCAT cells with same-sense mutation of LCK; (2) the Km group was the experimental group of CD19-UCAT cells with LCKT316I editing. Only homologous host PBMC cells from the same donor source were used as the effector cells in this mixed lymphocyte reaction.

This example was planned to be carried out with an effector-to-target ratio of 10:1. The 2 groups of target cells were separately plated in 6 wells in a flat-bottomed 48-well plate at 1.5×10⁵ CAR positive cells (500µl volume), and the effector cells were plated at 1.5×10⁶ cells (500µL volume) respectively and mixed with the effector cells, the total volume in each well was 1000 µL, and all were cultured in T cell complete medium. There were currently 2 groups of mixed wells of effector cells and target cells, 6 wells for each, and 2 treatments were given respectively, and 2 technical replicates were set for each treatment: treatment 1 was DMSO, treatment 2 was 50nM dasatinib. The plate was placed in a 37°C incubator to culture. Dasatinib and DMSO in the culture system were supplemented every two days.

In this test, the inventors predict that the effector cells in the Km group could exhibit a growth advantage over other groups in the dasatinib treatment groups. To verify the above hypothesis, the inventors needed to collect the following data during the mixed lymphocyte reaction: cell count at different time points; percentage and absolute number of effector cells and target cells at different time points; CAR positive rate of effector cells.

In order to obtain the above data, the inventors took part of the cells from each well on day 4, 9, 12, and 16 of the co-culture (the day of plating was defined as day 0) for cell counting. It needs to determine the percentage of the cells taken out relative to the overall cells in the well, and the absolute number of cells in each well is calculated by using this percentage and the counting result of the cells taken out. The cells taken out also need to be used for flow cytometric analysis to determine the percentages of effector cells and target cells and the CAR positive rate of effector cells.

In this mixed lymphocyte reaction, the inventor combined the results of cell counting and flow cytometry analysis at each time point to calculate the indicators of interest. The actual number of plated cells on day 0 is obtained by cell counting and flow cytometry after plating, not by the amount of cells to be added in the experimental design. Because the error caused by adding samples will cause the actual plated volume to be inconsistent with the planned plated cell volume, and secondly, because technical repetitions are set, counting the actual plated volume makes it easier to perform biostatistical analysis on the results. The actual CAR-positive rate of effector cells was about 18% (Ctrl group) and 25% (Km group) during the test. After the test, according to the indicators obtained at each time point, the inventors carried out exhaustive statistical analysis on the dynamic changes in the number of target cells (Fig. 40A), the dynamic changes in the number of effector cells (Figs. 40C, 40D) and the positive CAR rate in target cells (Fig. 40E), respectively.

As shown in Figs. 40A and 40B, with DMSO treatment, both Ctrl-CD19-UCART and Km-CD19-UCART could not effectively expand in the environment of host T cells and NK cells, while with 50nM dasatinib treatment, the control groups Ctrl-CD19-UCART and Km-CD19-UCART could expand effectively.

For the dynamic changes of host T cells and NK cells, as shown in Figs. 40C and 40D, with DMSO treatment, host T cells and NK cells in the Ctrl-CD19-UCART and Km-CD19-UCART groups were significantly higher than those in the DS treatment group. Consistent with the phenomenon observed in Figs. 40A and 40B, the reason for the decrease in the number of UCARTs in the DMSO treatment group is that the activation and expansion of host T cells and NK cells lead to the clearance of UCART cells.

Figs. 40E and 40F reveal that CAR-positive cells in UCART cells increased continuously in the Km-CD19-UCART group treated with dasatinib, but slightly increased in the Ctrl-CD19-UCART group, and decreased continuously in the DMSO group. It suggests that the main functional population in the Km-CD19-UCART group treated with dasatinib is CAR-positive cells, and the combination of Km editing and dasatinib give CAR-positive cells a survival advantage and enrichment. The CAR positive rate was significantly increased only in the Km-CD19-UCART of the dasatinib treatment group, and there was no significant increase in the CAR positive rate in the Ctrl-CD19-UCART and DMSO treatment groups.

### Example 23: Optimization and confirmation of the preparation process of UCAR-T with LCK^{T316I} mutation

Considering that the UCAR-T cells with LCK-T316I mutation prepared in the above examples have different LCK-T316I mutation efficiencies, in order to maintain high-efficiency LCK-T316I mutation in the application process, the inventors optimized the preparation process of UCAR-T with LCK^{T316I} mutation, and confirmed the preparation method that can ensure the high mutation rate of LCK-T316I.

The final preparation process we confirmed is as follows:
On Day 0, Miltenyi's Pan T Cell Isolation Kit, human kit was used to sort T cells from PBMCs; the T cells was cultured in a cytokine-free medium; after resting for 5hr, a Celetrix electroporation instrument was used to perform resting electroporation, while Cas9 protein was used to knock out TRAC and B2M genes, and CBE3 protein was used to edit Km gene; 50nM-100nM dasatinib was immediately added for treatment after editing; and 2hr later, Miltenyi's Human CD2/CD3/CD28 activator was used for T cell activation at 25ul/ml by volume to stimulate cells for 2 days;
On Day 1, no operation, only the culture was observed;
On Day 2, after activation of T cells for 48hr, lentiviral transduction was performed with MOI=1-3, using 1% DMSO or 100x lentiboost as an assistant;
On Day 3, the virus medium was changed 24hr after viral transduction, and culture of the cells was continued;
On Day 4, no operation, only the culture was observed;
On Day 6, negative selection was carried out on TRAC and B2M double negative cells, CD3 microbeads was used to sort TRAC negative cells, PE-anti-B2M antibody and PE microbeads were used to sort B2M negative cells, and finally the negative cells were culgured in Grex;
On Day 7, no operation, only the culture was observed;
On Day 8, half of the medium was replaced or the medium was supplemented;
On Day 9, no operation, only the culture was observed;
On Day 10/11, cell formulation and cryopreservation were performed.

The inventors isolated CD3-positive T cells from PBMCs from three different donors using PanT kits, knocked out the B2M and TRAC genes, and performed LCK-T316I editing on the cells. Then the cells were divided into two portions, to one portion, 100nM DS was added (experimental group), and to the other portion, a corresponding amount of DMSO was added as a control (control group). After 2hr, to the experimental group and the control group, T cell activators (CD2/CD3/ CD28, 25ul/1M/mL) were added. After 48hr of activation, the cells were infected with the anti-CD19 CAR molecule through lentivirus infection, and the medium was replaced 24hr after the virus infection, and DS or DMSO was removed at the same time.

As shown in Fig. 41A, the percentage of B2M and TRAC double knockout in the experimental group accounted for more than 50% (Fig. 41A, left column), and the percentages of B2M and TRAC double knockout in the control groups varied (Fig. 41A, right column), but all lower than that in the experimental group. It can be seen from the flow cytometry diagram that the reason why the percentage of B2M and TRAC double knockout in the control group is lower than that in the experimental group is that DS inhibits the activation of TCR positive cells (proving the conclusion of Example 3). The positive rate of CAR was greater than 15% (Fig. 41B). On Day 8 of culture, the efficiency of LCK-T316I of the cells was detected. LCK-T316I in the group with 100 nM DS (blue) was significantly higher than that in the group with a corresponding amount of DMSO (red) (Fig. 41C). Therefore, the inventors confirmed that adding 100nM dasatinib during the preparation of UCART can significantly enrich cells with T316I mutation, thereby increasing the mutation rate in the final product.

### Example 24: Optimization and confirmation of the third signal element carried by UCAR-T with LCK^{T316I} mutation

A sufficient number of the prepared CD19-UCART containing different third signal structures and Raji cells treated with mitomycin-C underwent repeated antigen stimulation to simulate the process where UCART cells continuously killed target cells and continuously expanded in the environment of repeated contact between target cells and UCART cells in vivo.

The present invention uses gene editing technology to simultaneously knock out the TRAC gene and the B2M gene in T cells to prepare CD19-UCART cells.

CD19-UCART cells were prepared from healthy donors in a small number. In the cell samples prepared in this batch, the inventors measured by flow cytometry that the knockout efficiency of TRAC gene was >90%; and the expression of CAR in different UCART cells was about 10-50% as determined by detecting CD19 antigen marker.

The present invention intends to measure the proliferation and cytotoxicity characteristics of UCAR-T cells with different third signal elements through repeated stimulation of target cell antigens, so that the UCAR-T carrying third signal element LCK^{T316I} mutation is preferred. A total of two experiments were carried out, which were distinguished as Experiment I and Experiment II.

The method comprises the steps of:
(1) Construction of plasmids and lentiviruses carrying different third signal elements, with the plasmid schematic diagram shown in Fig. 42A;
(2) Sorting of CD3+ T cells;
(3) TRAC, B2M gene knockout, and T316I editing of LCK gene (Km) in unactivated T cells;
(4) Activation of cells after electroporation;
(5) Infection of cells with different constructed lentiviruses;
(6) The knockout efficiency results of TRAC and CD5 genes are shown in Fig. 42B;
(7) Detection of CAR positive rate; due to the lack of molecular markers, CD19 antigen in combination with the expressed Anti-CD19 CAR structure was used to indicate the CAR+ percentage, and the results are shown in Fig. 42C;
(8) Enrichment of TRAC/B2M double-negative cells: In order to obtain UCART cells with higher purity, the inventors used FITC-B2M antibody + FITC microbeads combined with CD3+ microbeads to perform filtration in the LS column to obtain UCART cells with TRAC/B2M-/- percentage >99%;
(9) Treatment of target cells for antigen stimulation in vitro experiments: In order to test the proliferation and killing of CD19-UCART under repeated antigen stimulation, the inventors needed to obtain CD19 antigen that can bind to CD19-UCART. Therefore, Raji cells were treated with mitomycin C at a concentration of 1ug/M/ml. Such treatment was intended to render Raji cells incapable of dividing, but to retain their surface antigenic properties;
(10) Stimulation of ultimate proliferation of CD19-UCART cells after repeatedly killing of tumor cells in vivo using repeated antigen stimulation: This repeated antigen stimulation experiment aimed to compare the ultimate proliferation ability of CD19-UCART cells containing different third signal structures under repeated stimulation by external antigens, and verify whether the cells could maintain a strong ability to kill tumor cells after continuous proliferation. Therefore, the inventors kept the CD19-UCART cells in an activated state at a stimulation frequency of once every 3 days, and regarded it as a process of continuously clearing tumors by CD19-UCART in vivo.

The flow chart of this example is shown in Fig. 43A. The effector cells in the repeated stimulation experiments of Experiment I and Experiment II were grouped according to different third signal structures. On Day-1, Raji cells pretreated with mitomycin C (1ug/M/ml) for 16hr were used as target cells for repeated antigen stimulation. On Day0, the pretreated target cells were washed 3 times with 10 ml PBS before the start of co-culture. According to the total cell volume of 2M, CD19-UCART cells in each group were collected, and then UCART cells and Raji cells were mixed at an effector-to-target ratio of 2:1 and then co-cultured in 3m1 of medium. Thereafter, the above process was repeated every three days to replenish the target cells and perform counting and flow cytometry. The whole experiment used antigen stimulation four times in total.

This example was carried out with a 2:1 effector-to-target ratio. What needs to be explained here is that the inventors define the effector-to-target ratio as the ratio of the absolute number of CD19-UCART cells expressing CAR molecules to the absolute number of simulated host T cells. The effector cells with a total cell number of 2M in each group were plated in two wells of a flat-bottomed 12-well plate (1.5ml volume), and the target cells were respectively plated with a cell number of (number of effector cells*CAR+ percentage/2) (1.5ml volume) and mixed with effector cells, with a total volume of 3ml per well and a culture density of effector cells of 0.67M/ml. The effector cells were fixed to a total cell number of 2M each time the target cells were replenished repeatedly. If the effector cells were less than 2M after the previous stimulation, all of them were used for the next plating.

When patients take immunosuppressants, a series of immune cells are suppressed and their ability to secrete cytokines is reduced. Perhaps, a certain type of pretreatment lymphodepletion drugs can also cause the decrease of macrophages in patients, resulting in poor secretion of cytokines, such as etoposide. In order to compare whether there is any difference in the expansion of various UCART cells containing the third signal in a cytokine-poor environment and a cytokine-rich environment, The inventors set the co-culture conditions into two groups, namely M1 and M2. Among them, M1 was CTS basal T cell medium without supplementing any cytokine, and M2 medium was CTS basal T cell medium + 200U/ml IL2. In addition, for each group of effector cells, a group without target cells was set up under the corresponding medium conditions to observe their basal proliferation.

In this test, the inventors predicted that the proliferation ability of UCART cells containing the third signal would be stronger than that of the control group, but it is necessary to confirm the UCART cells with the best expansion ability. Therefore, the inventors needed to collect the following data during the repeated antigen stimulation experiment: cell counts at different time points; the CAR positive rate of effector cells at different time points and the cytotoxicity of different effector cells after repeated antigen stimulation.

In order to obtain the above data, the inventors took part of the cells from each well on day 3, day 6, day 9, day 12 and day 15 of the co-culture (the day of plating was defined as day 0) for cell counting. It needs to determine the percentage of the cells taken out relative to the overall cells in the well, and the absolute number of cells in each well is calculated by using this percentage and the counting result of the cells taken out. The cells taken out also need to be used for flow cytometric analysis to determine the CAR positive rate of effector cells.

The inventor combined the results of cell counting and flow cytometry analysis at each time point to calculate the indicators of interest. Figs. 43B and 43C show the expansion of CD19 CAR+ cells in the various groups in Experiment I during repeated stimulation. In Experiment I, the CAR+ enrichment rate of 2759 in both M1 medium and M2 medium was faster than that of other CD19-UCART cells. 2661 could also maintain a high level of CAR+ rate after killing target cells. However, CAR+ in the 2758 group did not increase significantly under the M1 and M2 culture conditions. In Figs. 43D and 43E, the UCART cells containing the third signal in each group in Experiment II had more CAR+ cell expansion than the control group in M1 and M2 medium, wherein the 2661 UCART cells obviously had the best expansion, also had high CAR+ enrichment factor and remained robust expansion after multiple rounds of stimulation. 2759 still had the expansion advantage of CAR+ cells in M1 medium, but the expansion advantage in M2 medium was not obvious.

The previous experiments had proved that 2759 and 2661 had the advantage of CAR+ expansion after repeated antigen stimulation. In order to further verify whether the cells of 2759 and 2661 have the advantage of cytotoxicity after antigen stimulation, the inventors co-cultured the effector cells and Raji-luc cells after four rounds of repeated stimulation in Experiment I in the complete culture medium of T cells at an effector-to-target ratio of 1:1, and co-cultured the effector cells and Raji-luc cells after four rounds of repeated stimulation in the complete culture medium of T cells at an effector-to-target ratio of 2:1 and 0.5:1, and then detected the luminosity of luciferase at 24hr and 48hr, so as to judge the c of different effector cells on Raji cells. The inventors converted the luminosity of luciferase each time into the corresponding percentage of cytotoxic cells and displayed the cytotoxicity of different effector cells in Figs. 44A-C. In experiment I, when measured at 24hr, the cytotoxicity of 1175 was significantly weaker than that of UCART cells containing the third signal structure under the condition of M1 medium. Under the two medium conditions, the relative cytotoxicity of 2759 was the strongest, followed by 2661. The killing rates of various UCART cells were not much different when measured at 48hr. In Experiment II, 2759 had an obvious cytotoxicity advantage over the other two UCART cells under the M1 culture condition. Under the M2 medium condition, the three UCART cells were not much different in cytotoxicity.

In order to further verify whether 2759 and 2661 cells have advantages in cytotoxicity after repeated antigen stimulation, in Experiment II, the inventors co-incubated UCART cells after four rounds of repeated antigen stimulation with different target cells (positive target cells Raji expressing CD19 antigen and negative target cells CCRF that do not express CD19 antigen) and different groups of CD19-CAR-T cells to stimulate the activation of CAR signals, mediate CAR-T to kill target cells and accumulation of CD107a in CAR-T. 5hr before detection, monensin (1:1000) and anti-CD107a-PEcy7 antibody (1:100) were added, and staining for CD8 and CAR positive was performed 5hr later to detect the enrichment of CD107a translocation of CD8 positive CAR positive cells. The statistical results are shown in Fig. 44D. The UCART cells in the various groups had little difference in the release rate of 107a, and the release rate in 2661 was the highest under the M1 culture condition.

The aforementioned experiments have proved that 2759 and 2661 have CAR+ expansion advantages and cytotoxicity advantages after repeated antigen stimulation. In order to further verify whether the cells 2759 and 2661 have the advantage of cytokine secretion after antigen stimulation, the inventors collected the cell culture supernatants after four rounds of repeated antigen stimulation in the aforementioned experiments I and II, quickly stored them in a refrigerator at -80°C, and frozen and thawed the supernatants for cytokine detection within one week. Experiment I detected TNF-alpha, as shown in Fig. 45A; Experiment II detected IL2, TNF-alpha, IFN-gamma, as shown in Fig. 45B-D. Only TNF-alpha and IFN-gamma were detected because the medium contained IL2 under the M2 culture condition.

The inventors used the human IL2 kit, human TNF-alpha kit, and human IFN-gamma kit from Cisbio to detect the cytokine supernatant after freezing and thawing. Because the supernatant concentration of IFN-gamma is generally too high, the inventors diluted the cytokine supernatant by 20 times and then tested for IFN-gamma. The detection process was operated in full accordance with the instruction manual.

It can be found that under the M1 and M2 culture environment, TNF-alpha and IFN-gamma of 2759 had obvious secretion advantages, followed by 2661. The IL2 secretion of 2661, 2759, and 2842 did not exceed that of the control group (1175). The IL2TNF-alpha and IFN-gamma secretion of 2758 was lower than that of the control group.

Based on the above experimental results, the inventors have proved that part of the third signaling structure can indeed promote the expansion ability of UCART cells, and make them still retain a considerable degree of cytotoxicity after massive expansion. The inventors finally selected 2661 and 2759 as the third signal elements that need to be carried by UCAR-T to further improve the LCK^{T316I} mutation.

Some of the amino acid or nucleotide sequences mentioned herein are as follows:
SEQ ID NO: 1 LCK-sgRNA12-21nt target DNA sequence
   TCTACATCATCACTGAATACA
SEQ ID NO: 2 LCK-sgRNA12-20nt target DNA sequence
   CTACATCATCACTGAATACA
SEQ ID NO: 3 LCK-sgRNA12-19nt target DNA sequence
   TACATCATCACTGAATACA
SEQ ID NO: 4 LCK-sgRNA12-18nt target DNA sequence
   ACATCATCACTGAATACA
SEQ ID NO: 5 LCK-sgRNA16-21nt target DNA sequence
   CATCACTGAATACATGGAGAA
SEQ ID NO: 6 LCK-sgRNA16-20nt target DNA sequence
   ATCACTGAATACATGGAGAA
SEQ ID NO: 7 LCK-sgRNA16-19nt target DNA sequence
   TCACTGAATACATGGAGAA
SEQ ID NO: 8 LCK-sgRNA16-18nt target DNA sequence
   CACTGAATACATGGAGAA
SEQ ID NO: 9 LCK-T316I mutant protein sequence 
MGCGCSSHPEDDWMENIDVCENCHYPIVPLDGKGTLLIRNGSEVRDPLVTYEGSNPP
ASPLQDNLVIALHSYEPSHDGDLGFEKGEQLRILEQSGEWWKAQSLTTGQEGFIPFNF
VAKANSLEPEPWFFKNLSRKDAERQLLAPGNTHGSFLIRESESTAGSFSLSVRDFDQN
QGEVVKHYKIRNLDNGGFYISPRITFPGLHELVRHYTNASDGLCTRLSRPCQTQKPQK
PWWEDEWEVPRETLKLVERLGAGQFGEVWMGYYNGHTKVAVKSLKQGSMSPDAFL
AEANLMKQLQHQRLVRLYAVVTQEPIYIIIEYMENGSLVDFLKTPSGIKLTINKLLDMA
AQIAEGMAFIEERNYIHRDLRAANILVSDTLSCKIADFGLARLIEDNEYTAREGAKFPI
KWTAPEAINYGTFTIKSDVWSFGILLTEIVTHGRIPYPGMTNPEVIQNLERGYRMVRPD
NCPEELYQLMRLCWKERPEDRPTFDYLRSVLEDFFTATEGQYQPQP*
 Here * stands for protein termination
SEQ ID NO: 10 LCK-T316A mutant protein sequence 
MGCGCSSHPEDDWMENIDVCENCHYPIVPLDGKGTLLIRNGSEVRDPLVTYEGSNPP
ASPLQDNLVIALHSYEPSHDGDLGFEKGEQLRILEQSGEWWKAQSLTTGQEGFIPFNF
VAKANSLEPEPWFFKNLSRKDAERQLLAPGNTHGSFLIRESESTAGSFSLSVRDFDQN
QGEVVKHYKIRNLDNGGFYISPRITFPGLHELVRHYTNASDGLCTRLSRPCQTQKPQK
PWWEDEWEVPRETLKLVERLGAGQFGEVWMGYYNGHTKVAVKSLKQGSMSPDAFL
AEANLMKQLQHQRLVRLYAVVTQEPIYIIAEYMENGSLVDFLKTPSGIKLTINKLLDM
AAQIAEGMAFIEERNYIHRDLRAANILVSDTLSCKIADFGLARLIEDNEYTAREGAKFP
IKWTAPEAINYGTFTIKSDVWSFGILLTEIVTHGRIPYPGMTNPEVIQNLERGYRMVRP
DNCPEELYQLMRLCWKERPEDRPTFDYLRSVLEDFFTATEGQYQPQP*
 Here * stands for protein termination
SEQ ID NO: 11 LCK-T316M mutant protein sequence 
MGCGCSSHPEDDWMENIDVCENCHYPIVPLDGKGTLLIRNGSEVRDPLVTYEGSNPP
ASPLQDNLVIALHSYEPSHDGDLGFEKGEQLRILEQSGEWWKAQSLTTGQEGFIPFNF
VAKANSLEPEPWFFKNLSRKDAERQLLAPGNTHGSFLIRESESTAGSFSLSVRDFDQN
QGEVVKHYKIRNLDNGGFYISPRITFPGLHELVRHYTNASDGLCTRLSRPCQTQKPQK
PWWEDEWEVPRETLKLVERLGAGQFGEVWMGYYNGHTKVAVKSLKQGSMSPDAFL
AEANLMKQLQHQRLVRLYAVVTQEPIYIIMEYMENGSLVDFLKTPSGIKLTINKLLDM
AAQIAEGMAFIEERNYIHRDLRAANILVSDTLSCKIADFGLARLIEDNEYTAREGAKFP
IKWTAPEAINYGTFTIKSDVWSFGILLTEIVTHGRIPYPGMTNPEVIQNLERGYRMVRP
DNCPEELYQLMRLCWKERPEDRPTFDYLRSVLEDFFTATEGQYQPQP*
 Here * stands for protein termination
SEQ ID NO: 12 LCK-saCas9-sgRNA1 target DNA sequence
   CTACATCATCACTGAATACATG
SEQ ID NO: 13 LCK-saCas9-sgRNA2 target DNA sequence
   ATCACTGAATACATGGAGAATG
SEQ ID NO: 14 LCK-sgRNA1 target DNA sequence
   GCTACCCGAGTCGGCTACCA
SEQ ID NO: 15 LCK-sgRNA11 target DNA sequence
   GCTCTACGCTGTGGTCACCC
SEQ ID NO: 16 LCK-sgRNA8 target DNA sequence
   GTATTCAGTGATGATGTAGA
SEQ ID NO: 17 LCK-sgRNA9 target DNA sequence
   TATTCAGTGATGATGTAGAT
SEQ ID NO: 18 LCK-sgRNA12 target DNA sequence
   CTACATCATCACTGAATACA
SEQ ID NO: 19 ssDNA-T316I-for-sgRNA8 DNA sequence as repair template 
A*C*G*CCTTCCTGGCCGAGGCCAACCTCATGAAGCAGCTGCAACACCAGCGGCTG
GTTCGGCTCTACGCTGTGGTCACCCAGGAGCCCATCTACATCATCattGAATACATGG
AGAATGgtgggtgctacccga*g*t*c
 Here * represents the thio modification between bases, three at each of the 5' and 3' ends
SEQ ID NO: 20 ssDNA-T316I-for-sgRNA9 DNA sequence as repair template 
G*A*C*GCCTTCCTGGCCGAGGCCAACCTCATGAAGCAGCTGCAACACCAGCGGCT
GGTTCGGCTCTACGCTGTGGTCACCCAGGAGCCCATCTACATCATCattGAATACATG
GAGAATGgtgggtgctacccg*a*g*t
 Here * represents the thio modification between bases, three at each of the 5' and 3' ends
SEQ ID NO: 21 ssDNA-T316I-for-sgRNA12 DNA sequence as repair template 
a*a*a*aggtgtatttccaccctctggcagggacagcagggagagcagtatcccctggtagccgactcgggtagcacccacCATT
CTCCATGTATTCaatGATGATGTAGATGGGCTCCTGGGTGACCACAGCGT*A*G*A
 Here * represents the thio modification between bases, three at each of the 5' and 3' ends
SEQ ID NO: 22 ssDNA-T316M-for-sgRNA8 DNA sequence as repair template 
A*C*G*CCTTCCTGGCCGAGGCCAACCTCATGAAGCAGCTGCAACACCAGCGGCTG
GTTCGGCTCTACGCTGTGGTCACCCAGGAGCCCATCTACATCATCatgGAATACATGG
AGAATGgtgggtgctacccga*g*t*c
 Here * represents the thio modification between bases, three at each of the 5' and 3' ends
SEQ ID NO: 23 ssDNA-T316M-for-sgRNA9 DNA sequence as repair template 
G*A*C*GCCTTCCTGGCCGAGGCCAACCTCATGAAGCAGCTGCAACACCAGCGGCT
GGTTCGGCTCTACGCTGTGGTCACCCAGGAGCCCATCTACATCATCatgGAATACATG
GAGAATGgtgggtgctacccg*a*g*t
 Here * represents the thio modification between bases, three at each of the 5' and 3' ends
SEQ ID NO: 24 ssDNA-T316M-for-sgRNA12 DNA sequence as repair template 
a*a*a*aggtgtatttccaccctctggcagggacagcagggagagcagtatcccctggtagccgactcgggtagcacccacCATT
CTCCATGTATTCcatGATGATGTAGATGGGCTCCTGGGTGACCACAGCGT*A*G*A
 Here * represents the thio modification between bases, three at each of the 5' and 3' ends
SEQ ID NO: 25 ssDNA-T316A-for-sgRNA8 DNA sequence as repair template 
A*C*G*CCTTCCTGGCCGAGGCCAACCTCATGAAGCAGCTGCAACACCAGCGGCTG
GTTCGGCTCTACGCTGTGGTCACCCAGGAGCCCATCTACATCATCgccGAATACATG
GAGAATGgtgggtgctacccga*g*t*c
 Here * represents the thio modification between bases, three at each of the 5' and 3' ends
SEQ ID NO: 26 ssDNA-T316A-for-sgRNA9 DNA sequence as repair template 
G*A*C*GCCTTCCTGGCCGAGGCCAACCTCATGAAGCAGCTGCAACACCAGCGGCT
GGTTCGGCTCTACGCTGTGGTCACCCAGGAGCCCATCTACATCATCgccGAATACAT
GGAGAATGgtgggtgctacccg*a*g*t
 Here * represents the thio modification between bases, three at each of the 5' and 3' ends
SEQ ID NO: 27 ssDNA-T316A-for-sgRNA12 DNA sequence as repair template 
a*a*a*aggtgtatttccaccctctggcagggacagcagggagagcagtatcccctggtagccgactcgggtagcacccacCATT
CTCCATGTATTCcggGATGATGTAGATGGGCTCCTGGGTGACCACAGCGT*A*G*A
 Here * represents the thio modification between bases, three at each of the 5' and 3' ends
SEQ ID NO: 28 ssDNA-T316I-for-sgRNA1+sgRNA11 DNA sequence as repair template 
aggaaagctcaagaaaccctccttgcttgagctttgggccagaagaaaaggtgtatttccaccctctggcagggacagcagggagag
cagtatcccctggtagccgactcgggtagcacccacCATTCTCCATGTATTCaatGATGATGTAGATGGGC
TCCTGGGTGACCACAGCGTAGAGCCGAACCAGCCGCTGGTGTTGCAGCTGCTTCA
TGAGGTTGGCCTCGGCCAGGAAGGCGTCCGGGGACATGCTGCCCTGCTTCAG
SEQ ID NO: 29 ssDNA-T316M-for-sgRNA11 DNA sequence as repair template 
aggaaagctcaagaaaccctccttgcttgagctttgggccagaagaaaaggtgtatttccaccctctggcagggacagcagggagag
cagtatcccctggtagccgactcgggtagcacccacCATTCTCCATGTATTCcatGATGATGTAGATGGGC
TCCTGGGTGACCACAGCGTAGAGCCGAACCAGCCGCTGGTGTTGCAGCTGCTTCA
TGAGGTTGGCCTCGGCCAGGAAGGCGTCCGGGGACATGCTGCCCTGCTTCAG
SEQ ID NO: 30 ssDNA-T316A-for-sgRNA1+sgRNA11 DNA sequence as repair template 
aggaaagctcaagaaaccctccttgcttgagctttgggccagaagaaaaggtgtatttccaccctctggcagggacagcagggagag
cagtatcccctggtagccgactcgggtagcacccacCATTCTCCATGTATTCcggGATGATGTAGATGGGC
TCCTGGGTGACCACAGCGTAGAGCCGAACCAGCCGCTGGTGTTGCAGCTGCTTCA
TGAGGTTGGCCTCGGCCAGGAAGGCGTCCGGGGACATGCTGCCCTGCTTCAG
SEQ ID NO: 31 ssDNA-T316I-for-sgRNA1+sgRNA12 DNA sequence as repair template 
aggaaagctcaagaaaccctccttgcttgagctttgggccagaagaaaaggtgtatttccaccctctggcagggacagcagggagag
cagtatcccctggtagccgactcgggtagcacccacCATTCTCCATGTATTCaatGATGATGTAGATGGGC
TCCTGGGTGACCACAGCGTAGAGCCGAACCAGCCGCTGGTGTTGCAGCTGCTTCA
TGAGGTTGGCCTCGGCCAGGAA
SEQ ID NO: 32 ssDNA-T316M-for-sgRNA1+sgRNA12 DNA sequence as repair template 
aggaaagctcaagaaaccctccttgcttgagctttgggccagaagaaaaggtgtatttccaccctctggcagggacagcagggagag
cagtatcccctggtagccgactcgggtagcacccacCATTCTCCATGTATTCcatGATGATGTAGATGGGC
TCCTGGGTGACCACAGCGTAGAGCCGAACCAGCCGCTGGTGTTGCAGCTGCTTCA
TGAGGTTGGCCTCGGCCAGGAA
SEQ ID NO: 33 ssDNA-T316A-for-sgRNA1+sgRNA12 DNA sequence as repair template 
aggaaagctcaagaaaccctccttgcttgagctttgggccagaagaaaaggtgtatttccaccctctggcagggacagcagggagag
cagtatcccctggtagccgactcgggtagcacccacCATTCTCCATGTATTCcggGATGATGTAGATGGGC
TCCTGGGTGACCACAGCGTAGAGCCGAACCAGCCGCTGGTGTTGCAGCTGCTTCA
TGAGGTTGGCCTCGGCCAGGAA
SEQ ID NO: 34 Ecoli-A3A-CBE3-protein protein sequence purified from Escherichia coli 
MEASPASGPRHLMDPHIFTSNFNNGIGRHKTYLCYEVERLDNGTSVKMDQHRGFLHN
QAKNLLCGFYGRHAELRFLDLVPSLQLDPAQIYRVTWFISWSPCFSWGCAGEVRAFL
QENTHVRLRIFAARIYDYDPLYKEALQMLRDAGAQVSIMTYDEFKHCWDTFVDHQG
CPFQPWDGLDEHSQALSGRLRAILQNQGNSGSETPGTSESATPESDKKYSIGLAIGTNS
VGWAVITDEYKVPSKKFKVLGNTDRHSIKKNLIGALLFDSGETAEATRLKRTARRRYT
RRKNRICYLQEIFSNEMAKVDDSFFHRLEESFLVEEDKKHERHPIFGNIVDEVAYHEKY
PTIYHLRKKLVDSTDKADLRLIYLALAHMIKFRGHFLIEGDLNPDNSDVDKLFIQLVQT
YNQLFEENPINASGVDAKAILSARLSKSRRLENLIAQLPGEKKNGLFGNLIALSLGLTP
NFKSNFDLAEDAKLQLSKDTYDDDLDNLLAQIGDQYADLFLAAKNLSDAILLSDILRV
NTEITKAPLSASMIKRYDEHHQDLTLLKALVRQQLPEKYKEIFFDQSKNGYAGYIDGG
ASQEEFYKFIKPILEKMDGTEELLVKLNREDLLRKQRTFDNGSIPHQIHLGELHAILRR
QEDFYPFLKDNREKIEKILTFRIPYYVGPLARGNSRFAWMTRKSEETITPWNFEEVVDK
GASAQSFIERMTNFDKNLPNEKVLPKHSLLYEYFTVYNELTKVKYVTEGMRKPAFLS
GEQKKAIVDLLFKTNRKVTVKQLKEDYFKKIECFDSVEISGVEDRFNASLGTYHDLL
KIIKDKDFLDNEENEDILEDIVLTLTLFEDREMIEERLKTYAHLFDDKVMKQLKRRRYT
GWGRLSRKLINGIRDKQSGKTILDFLKSDGFANRNFMQLIHDDSLTFKEDIQKAQVSG
QGDSLHEHIANLAGSPAIKKGILQTVKVVDELVKVMGRHKPENIVIEMARENQTTQK
GQKNSRERMKRIEEGIKELGSQILKEHPVENTQLQNEKLYLYYLQNGRDMYVDQELD
INRLSDYDVDHIVPQSFLKDDSIDNKVLTRSDKNRGKSDNVPSEEVVKKMKNYWRQ
LLNAKLITQRKFDNLTKAERGGLSELDKAGFIKRQLVETRQITKHVAQILDSRMNTKY
DENDKLIREVKVITLKSKLVSDFRKDFQFYKVREINNYHHAHDAYLNAVVGTALIKK
YPKLESEFVYGDYKVYDVRKMIAKSEQEIGKATAKYFFYSNIMNFFKTEITLANGEIR
KRPLIETNGETGEIVWDKGRDFATVRKVLSMPQVNIVKKTEVQTGGFSKESILPKRNS
DKLIARKKDWDPKKYGGFDSPTVAYSVLVVAKVEKGKSKKLKSVKELLGITIMERSSF
EKNPIDFLEAKGYKEVKKDLIIKLPKYSLFELENGRKRMLASAGELQKGNELALPSKY
VNFLYLASHYEKLKGSPEDNEQKQLFVEQHKHYLDEIIEQISEFSKRVILADANLDKV
LSAYNKHRDKPIREQAENIIHLFTLTNLGAPAAFKYFDTTIDRKRYTSTKEVLDATLIHQ
SITGLYETRIDLSQLGGDSGGSTNLSDIIEKETGKQLVIQESILMLPEEVEEVIGNKPESD
ILVHTAYDESTDENVMLLTSDAPEYKPWALVIQDSNGENKIKMLSGGSPKKKRKVHH
HHHHHHH*
 Here * stands for protein termination
SEQ ID NO: 35 CD19-78 VL nucleic acid sequence 
CAGGCTGTGCTGACTCAGCCACCCTCGGTGTCTGAAGCCCCCAGGCAGAGGGTCA
CCATCTCCTGTTCTGGAAGCAGCTCCAACATCGGAAATAATGCTGTAAGCTGGTAC
CAGCAGCTCCCAGGAAAGGCTCCCAAACTCCTCATCTATTATGATGATCTGCTCCC
CTCAGGGGTCTCTGACCGATTCTCTGGCTCCAAGTCTGGCACCTCAGCCTCCCTGG
CCATCAGTGGGCTCCAGTCTGAGGATGAGGCTGATTATTACTGTGCAGCATGGGAT
GACAGCCTGAATGGTTGGGTGTTCGGCGGAGGGACCAAGGTCACCGTCCTAGGT
SEQ ID NO: 36 CD19-78 VL protein sequence 
QAVLTQPPSVSEAPRQRVTISCSGSSSNIGNNAVSWYQQLPGKAPKLLIYYDDLLPSGV
SDRFSGSKSGTSASLAISGLQSEDEADYYCAAWDDSLNGWVFGGGTKVTVLG
SEQ ID NO: 37 CD19-78 VH nucleic acid sequence 
GAGGTGCAGCTGGTGCAGTCTGGAGCAGAGGTGAAAAAGCCCGGGGAGTCTCTG
AAGATCTCCTGTAAGGGTTCTGGATACAGCTTTACCAGCTACTGGATCGGCTGGGT
GCGCCAGATGCCCGGGAAAGGCCTGGAGTGGATGGGGATCATCTATCCTGGTGAC
TCTGATACCAGATACAGCCCGTCCTTCCAAGGCCAGGTCACCATCTCAGCCGACAA
GTCCATCAGCACCGCCTACCTGCAGTGGAGCAGCCTGAAGGCCTCGGACACCGCC
ATGTATTACTGTGCGCGCCTGTCTTACTCTTGGTCTTCTTGGTACTGGGATTTCTGG
GGTCAAGGTACTCTGGTGACCGTCTCCTCA
SEQ ID NO: 38 CD19-78 VH protein sequence 
EVQLVQSGAEVKKPGESLKISCKGSGYSFTSYWIGWVRQMPGKGLEWMGIIYPGDSD
TRYSPSFQGQVTISADKSISTAYLQWSSLKASDTAMYYCARLSYSWSSWYWDFWGQ
GTLVTVSS
SEQ ID NO: 39 CD19-Linker1 nucleic acid sequence
   GGTGGTGGTGGTAGCGGCGGCGGCGGCTCTGGTGGTGGTGGATCC
SEQ ID NO: 40 CD19-Linker1 protein sequence
   GGGGSGGGGSGGGGS
SEQ ID NO: 41 CD19-CD8a hinge nucleic acid sequence 
ACTACTACCCCTGCACCTAGGCCTCCCACCCCAGCCCCAACAATCGCCAGCCAGCC
TCTGTCTCTGCGGCCCGAAGCCTGTAGACCTGCTGCCGGCGGAGCCGTGCACACC
AGAGGCCTGGACTTCGCCTGCGAC
SEQ ID NO: 42 CD19-CD8a hinge protein sequence
   TTTPAPRPPTPAPTIASQPLSLRPEACRPAAGGAVHTRGLDFACD
SEQ ID NO: 43 CD19-CD8a TM nucleic acid sequence 
ATCTACATCTGGGCCCCTCTGGCCGGCACCTGTGGCGTGCTGCTGCTGAGCCTGGT
GATCACCCTGTACTGC
SEQ ID NO: 44 CD19-CD8a TM protein sequence
   IYIWAPLAGTCGVLLL SLVITLYC
SEQ ID NO: 45 CD19-CD28 intracellular domain (IC) nucleic acid sequence 
AGAAGCAAGCGGAGCCGGCTGCTGCACAGCGACTACATGAACATGACCCCAAGA
CGGCCTGGCCCCACCCGGAAGCACTACCAGCCTTACGCCCCTCCCAGAGACTTCG
CCGCCTACCGGTCC
SEQ ID NO: 46 CD19-CD28 intracellular domain (IC) protein sequence
   RSKRSRLLHSDYMNMTPRRPGPTRKHYQPYAPPRDFAAYRS
SEQ ID NO: 47 CD19-CD3z intracellular signaling domain nucleic acid sequence 
AGAGTGAAGTTCAGCAGATCCGCCGACGCCCCTGCCTACCAGCAGGGACAGAAC
CAGCTGTACAACGAGCTGAACCTGGGCAGACGGGAAGAGTACGACGTGCTGGAC
AAGCGGAGAGGCCGGGACCCCGAGATGGGCGGAAAGCCCAGACGGAAGAACCC
CCAGGAAGGCCTGTATAACGAACTGCAGAAAGACAAGATGGCCGAGGCCTACAG
CGAGATCGGCATGAAGGGCGAGCGGAGGCGCGGCAAGGGCCACGATGGCCTGTA
CCAGGGCCTGAGCACCGCCACCAAGGACACCTACGACGCCCTGCACATGCAGGCC
CTGCCCCCCAGA
SEQ ID NO: 48 CD19-CD3z intracellular signaling transduction domain protein sequence 
RVKFSRSADAPAYQQGQNQLYNELNLGRREEYDVLDKRRGRDPEMGGKPRRKNPQE

GLYNELQKDKMAEAYSEIGMKGERRRGKGHDGLYQGLSTATKDTYDALHMQALPPR
SEQ ID NO: 49 CD19-T2A nucleic acid sequence
   GAGGGAAGGGGCAGCTTATTAACATGTGGCGATGTGGAAGAGAACCCCGGTCCC
SEQ ID NO: 50 CD19-T2A protein sequence
   EGRGSLLTCGDVEENPGP
SEQ ID NO: 51 CD19-CSF2RA signal nucleic acid sequence 
ATGCTGCTGCTCGTGACCTCTTTACTGTTATGTGAGCTGCCCCACCCCGCTTTTTTA
CTGATCCCT
SEQ ID NO: 52 CD19-CSF2RA signal protein sequence
   MLLLVTSLLLCELPHPAFLLIP
SEQ ID NO: 53 CD19-tEGFR nucleic acid sequence 
CGTAAGGTGTGTAACGGAATCGGCATTGGCGAGTTCAAGGACTCTTTAAGCATCAA
CGCCACAAACATCAAGCACTTCAAGAATTGTACCTCCATCAGCGGCGATTTACACA
TTCTCCCCGTGGCTTTTCGTGGCGATTCCTTCACCCACACCCCCCCTCTGGACCCC
CAAGAGCTGGACATTTTAAAAACCGTGAAGGAGATCACCGGCTTTCTGCTGATCC
AAGCTTGGCCCGAGAATCGTACCGACCTCCACGCCTTCGAGAATTTAGAGATTATT
CGTGGAAGGACCAAGCAGCACGGCCAGTTCTCTTTAGCCGTCGTGTCTTTAAACAT
TACCAGCCTCGGTTTAAGGTCTTTAAAGGAGATTAGCGACGGCGACGTGATCATCT
CCGGCAACAAGAACCTCTGCTACGCCAACACCATCAACTGGAAGAAGCTGTTCGG
AACCAGCGGCCAAAAGACCAAGATCATCAGCAATCGTGGAGAGAACTCTTGTAAG
GCCACTGGTCAAGTTTGCCACGCCCTCTGTAGCCCCGAAGGATGTTGGGGCCCCG
AGCCTAGGGACTGTGTTAGCTGCAGAAACGTGAGCAGAGGCAGAGAGTGTGTGG
ACAAATGCAATTTACTGGAAGGAGAGCCTAGGGAGTTCGTGGAGAACAGCGAATG
TATCCAGTGCCACCCCGAGTGTTTACCTCAAGCCATGAACATCACTTGTACCGGAA
GGGGCCCCGATAACTGCATCCAATGCGCCCACTACATCGACGGACCCCACTGCGTG
AAAACTTGTCCCGCCGGAGTGATGGGAGAGAATAACACTTTAGTGTGGAAGTACG
CCGACGCTGGCCACGTCTGCCATCTGTGCCACCCCAACTGTACCTACGGCTGCACT
GGTCCCGGTTTAGAGGGATGTCCTACCAACGGCCCCAAGATCCCCTCCATCGCCAC
CGGAATGGTGGGCGCTCTGTTATTACTGCTGGTGGTGGCTCTGGGCATCGGTTTATT

CATG
SEQ ID NO: 54 CD 19-tEGFR protein sequence 
RKVCNGIGIGEFKDSLSINATNIKHFKNCTSISGDLHILPVAFRGDSFTHTPPLDPQELDI
LKTVKEITGFLLIQAWPENRTDLHAFENLEIIRGRTKQHGQFSLAVVSLNITSLGLRSLK
EISDGDVIISGNKNLCYANTINWKKLFGTSGQKTKIISNRGENSCKATGQVCHALCSPE
GCWGPEPRDCVSCRNVSRGRECVDKCNLLEGEPREFVENSECIQCHPECLPQAMNIT
CTGRGPDNCIQCAHYIDGPHCVKTCPAGVMGENNTLVWKYADAGHVCHLCHPNCT
YGCTGPGLEGCPTNGPKIPSIATGMVGALLLLLVVALGIGLFM
SEQ ID NO: 55 CD19-CD28z-CAR nucleic acid sequence 
ATGGCCCTGCCTGTGACAGCTCTGCTCCTCCCTCTGGCCCTGCTGCTCCATGCCGC
CAGACCCCAGGCTGTGCTGACTCAGCCACCCTCGGTGTCTGAAGCCCCCAGGCAG
AGGGTCACCATCTCCTGTTCTGGAAGCAGCTCCAACATCGGAAATAATGCTGTAA
GCTGGTACCAGCAGCTCCCAGGAAAGGCTCCCAAACTCCTCATCTATTATGATGA
TCTGCTCCCCTCAGGGGTCTCTGACCGATTCTCTGGCTCCAAGTCTGGCACCTCAG
CCTCCCTGGCCATCAGTGGGCTCCAGTCTGAGGATGAGGCTGATTATTACTGTGC
AGCATGGGATGACAGCCTGAATGGTTGGGTGTTCGGCGGAGGGACCAAGGTCAC
CGTCCTAGGTGGTGGTGGTAGCGGCGGCGGCGGCTCTGGTGGTGGTGGATCC
GAGGTGCAGCTGGTGCAGTCTGGAGCAGAGGTGAAAAAGCCCGGGGAGTCTCTG
AAGATCTCCTGTAAGGGTTCTGGATACAGCTTTACCAGCTACTGGATCGGCTGGG
TGCGCCAGATGCCCGGGAAAGGCCTGGAGTGGATGGGGATCATCTATCCTGGTG
ACTCTGATACCAGATACAGCCCGTCCTTCCAAGGCCAGGTCACCATCTCAGCCGA
CAAGTCCATCAGCACCGCCTACCTGCAGTGGAGCAGCCTGAAGGCCTCGGACACC
GCCATGTATTACTGTGCGCGCCTGTCTTACTCTTGGTCTTCTTGGTACTGGGATTT
CTGGGGTCAAGGTACTCTGGTGACCGTCTCCTCATTCGTGCCCGTGTTCCTGCCCG
CCAAACCTACTACTACCCCTGCACCTAGGCCTCCCACCCCAGCCCCAACAATCGC
CAGCCAGCCTCTGTCTCTGCGGCCCGAAGCCTGTAGACCTGCTGCCGGCGGAGCC
GTGCACACCAGAGGCCTGGACTTCGCCTGCGACATCTACATCTGGGCCCCTCTGG
CCGGCACCTGTGGCGTGCTGCTGCTGAGCCTGGTGATCACCCTGTACTGCAACCA
CCGGAACAGAAGCAAGCGGAGCCGGCTGCTGCACAGCGACTACATGAACATGAC
CCCAAGACGGCCTGGCCCCACCCGGAAGCACTACCAGCCTTACGCCCCTCCCAGA
GACTTCGCCGCCTACCGGTCCAGAGTGAAGTTCAGCAGATCCGCCGACGCCCCTG
CCTACCAGCAGGGACAGAACCAGCTGTACAACGAGCTGAACCTGGGCAGACGGG
AAGAGTACGACGTGCTGGACAAGCGGAGAGGCCGGGACCCCGAGATGGGCGGA
AAGCCCAGACGGAAGAACCCCCAGGAAGGCCTGTATAACGAACTGCAGAAAGAC
AAGATGGCCGAGGCCTACAGCGAGATCGGCATGAAGGGCGAGCGGAGGCGCGG
CAAGGGCCACGATGGCCTGTACCAGGGCCTGAGCACCGCCACCAAGGACACCTA
CGACGCCCTGCACATGCAGGCCCTGCCCCCCAGAGGATCCGGAGAGGGAAGGGG
CAGCTTATTAACATGTGGCGATGTGGAAGAGAACCCCGGTCCCATGCTGCTGCTC
GTGACCTCTTTACTGTTATGTGAGCTGCCCCACCCCGCTTTTTTACTGATCCCTCGT
AAGGTGTGTAACGGAATCGGCATTGGCGAGTTCAAGGACTCTTTAAGCATCAACG
CCACAAACATCAAGCACTTCAAGAATTGTACCTCCATCAGCGGCGATTTACACAT
TCTCCCCGTGGCTTTTCGTGGCGATTCCTTCACCCACACCCCCCCTCTGGACCCCC
AAGAGCTGGACATTTTAAAAACCGTGAAGGAGATCACCGGCTTTCTGCTGATCCA

AGCTTGGCCCGAGAATCGTACCGACCTCCACGCCTTCGAGAATTTAGAGATTATT
CGTGGAAGGACCAAGCAGCACGGCCAGTTCTCTTTAGCCGTCGTGTCTTTAAACA
TTACCAGCCTCGGTTTAAGGTCTTTAAAGGAGATTAGCGACGGCGACGTGATCAT
CTCCGGCAACAAGAACCTCTGCTACGCCAACACCATCAACTGGAAGAAGCTGTTC
GGAACCAGCGGCCAAAAGACCAAGATCATCAGCAATCGTGGAGAGAACTCTTGT
AAGGCCACTGGTCAAGTTTGCCACGCCCTCTGTAGCCCCGAAGGATGTTGGGGCC
CCGAGCCTAGGGACTGTGTTAGCTGCAGAAACGTGAGCAGAGGCAGAGAGTGTG
TGGACAAATGCAATTTACTGGAAGGAGAGCCTAGGGAGTTCGTGGAGAACAGCG
AATGTATCCAGTGCCACCCCGAGTGTTTACCTCAAGCCATGAACATCACTTGTAC
CGGAAGGGGCCCCGATAACTGCATCCAATGCGCCCACTACATCGACGGACCCCA
CTGCGTGAAAACTTGTCCCGCCGGAGTGATGGGAGAGAATAACACTTTAGTGTGG
AAGTACGCCGACGCTGGCCACGTCTGCCATCTGTGCCACCCCAACTGTACCTACG
GCTGCACTGGTCCCGGTTTAGAGGGATGTCCTACCAACGGCCCCAAGATCCCCTC
CATCGCCACCGGAATGGTGGGCGCTCTGTTATTACTGCTGGTGGTGGCTCTGGGC
ATCGGTTTATTCATGTGA
SEQ ID NO: 56 CD19-CD28z-CAR protein sequence 
MALPVTALLLPLALLLHAARPQAVLTQPPSVSEAPRQRVTISCSGSSSNIGNNAVSWYQ
QLPGKAPKLLIYYDDLLPSGVSDRFSGSKSGTSASLAISGLQSEDEADYYCAAWDDSL
NGWVFGGGTKVTVLGGGGGSGGGGSGGGGSEVQLVQSGAEVKKPGESLKISCKGSG
YSFTSYWIGWVRQMPGKGLEWMGIIYPGDSDTRYSPSFQGQVTISADKSISTAYLQWS
SLKASDTAMYYCARLSYSWSSWYWDFWGQGTLVTVSSFVPVFLPAKPTTTPAPRPPT
PAPTIASQPLSLRPEACRPAAGGAVHTRGLDFACDIYIWAPLAGTCGVLLLSLVITLYCN
HRNRSKRSRLLHSDYMNMTPRRPGPTRKHYQPYAPPRDFAAYRSRVKFSRSADAPAY
QQGQNQLYNELNLGRREEYDVLDKRRGRDPEMGGKPRRKNPQEGLYNELQKDKMA
EAYSEIGMKGERRRGKGHDGLYQGLSTATKDTYDALHMQALPPRGSGEGRGSLLTCG
DVEENPGPMLLLVTSLLLCELPHPAFLLIPRKVCNGIGIGEFKDSLSINATNIKHFKNCT
SISGDLHILPVAFRGDSFTHTPPLDPQELDILKTVKEITGFLLIQAWPENRTDLHAFENL
EIIRGRTKQHGQFSLAVVSLNITSLGLRSLKEISDGDVIISGNKNLCYANTINWKKLFGT
SGQKTKIISNRGENSCKATGQVCHALCSPEGCWGPEPRDCVSCRNVSRGRECVDKCN
LLEGEPREFVENSECIQCHPECLPQAMNITCTGRGPDNCIQCAHYIDGPHCVKTCPAG
VMGENNTLVWKYADAGHVCHLCHPNCTYGCTGPGLEGCPTNGPKIPSIATGMVGAL
LLLLVVALGIGLFM*
 Here * stands for protein termination
SEQ ID NO: 57 CD5-Ab20001-61-VH nucleic acid sequence 
CAGGTGCAGCTGGTGCAGTCTGGGGCTGAGGTGAAGAAGCCTGGGTCCTCGGTG
AAGGTCTCCTGCAAGGCTTCTGGAGGCACCTTCAGCAACTATGCTATCAGCTGGGT
GCGACAGGCCCCTGGACAAGGGCTTGAGTGGATGGGATGGATCAGCGCCTACAAT
GGTGACACAAAATATGCACAGAGGCTCCAGGGCAGAGTCACCATGACCACAGAC
ACATCCACGAGCACAGCCTACATGGAGCTGAGGAACCTAAGATCTGACGACACGG
CCGTGTATTACTGTGCGCGCTACGAATCTATGTCTGGTCAGGATATCTGGGGTCAAG
GTACTCTGGTGACCGTCTCCTCA
SEQ ID NO: 58 CD5-Ab20001-61-VH protein sequence 
QVQLVQSGAEVKKPGSSVKVSCKASGGTFSNYAISWVRQAPGQGLEWMGWISAYNG
DTKYAQRLQGRVTMTTDTSTSTAYMELRNLRSDDTAVYYCARYESMSGQDIWGQGT
LVTVSS
SEQ ID NO: 59 CD5-Ab20001-42-VH nucleic acid sequence 
GAAGTTCAGCTGCTGGAAAGCGGTGGTGGTCTGGTTCAGCCTGGTGGTAGCCTGC
GTCTGAGCTGTGCAGCAAGCGGTTTTACCTTTAGCCATAGCGCCATGGGTTGGGTT
CGTCAGGCACCTGGTAAAGGTCTGGAATGGGTTAGCAGCATCTATGCCCGCGGCG
GCTATACCTATTATGCAGATAGCGTTAAAGGTCGTTTTACCATTAGCCGTGATAACA
GCAAAAATACCCTGTACCTGCAGATGAATAGTCTGCGTGCAGAGGATACCGCAGTG
TATTATTGTGCGCGCGGTTACCATCTGGAATACATGGTTTCTCAGGATGTTTGGGGT
CAAGGTACTCTGGTGACCGTCTCCTCA
SEQ ID NO: 60 CD5-Ab20001-42-VH protein sequence 
EVQLLESGGGLVQPGGSLRLSCAASGFTFSHSAMGWVRQAPGKGLEWVSSIYARGG
YTYYADSVKGRFTISRDNSKNTLYLQMNSLRAEDTAVYYCARGYHLEYMVSQDVW
GQGTLVTVSS
SEQ ID NO: 61 CD5-Linker1 nucleic acid sequence
   GGTGGTGGTGGTAGCGGCGGCGGCGGCTCTGGTGGTGGTGGATCC
SEQ ID NO: 62 CD5-Linker1 protein sequence
   GGGGSGGGGSGGGGS
SEQ ID NO: 63 CD5-CD8a nucleic acid sequence 
TTTGTGCCTGTATTTCTGCCTGCCAAGCCCACCACAACACCTGCCCCTAGACCACC
CACCCCTGCCCCCACCATTGCTTCTCAGCCCCTTAGCTTAAGACCTGAAGCCTGTA
GACCTGCTGCTGGGGGGGCTGTGCACACAAGAGGCCTGGACTTTGCCTGTACTAC

TACCCCTGCACCTAGGCCTCCCACCCCAGCCCCAACAATCGCCAGCCAGCCTCTGT
CTCTGCGGCCCGAAGCCTGTAGACCTGCTGCCGGCGGAGCCGTGCACACCAGAGG
CCTGGACTTCGCCTGCGACATCTACATCTGGGCCCCCCTGGCTGGCACCTGTGGGG
TGCTGCTGCTGAGCCTGGTGATCACCCTGTACTGCAACCACAGAAAC
SEQ ID NO: 64 CD5-CD8a protein sequence 
FVPVFLPAKPTTTPAPRPPTPAPTIASQPLSLRPEACRPAAGGAVHTRGLDFACTTTPAP
RPPTPAPTIASQPLSLRPEACRPAAGGAVHTRGLDFACDIYIWAPLAGTCGVLLLSLVIT
LYCNHRN
SEQ ID NO: 65 CD5-CD28 intracellular domain (IC) nucleic acid sequence 
AGGAGTAAGAGGAGCAGGCTCCTGCACAGTGACTACATGAACATGACTCCCCGCC
GCCCCGGGCCCACCCGCAAGCATTACCAGCCCTATGCCCCACCACGCGACTTCGC
AGCCTATCGCTCC
SEQ ID NO: 66 CD5-CD28 intracellular domain (IC) protein sequence
   RSKRSRLLHSDYMNMTPRRPGPTRKHYQPYAPPRDFAAYRS
SEQ ID NO: 67 CD5-CD3z intracellular signaling domain nucleic acid sequence 
AGAGTGAAGTTCAGCAGATCTGCTGATGCCCCTGCCTATCAGCAAGGGCAGAATC
AGCTGTACAATGAGCTGAATCTGGGCAGAAGAGAGGAGTATGATGTGCTGGACAA
GAGAAGAGGCAGAGACCCTGAGATGGGGGGCAAGCCTAGAAGAAAGAACCCCC
AAGAGGGCCTGTATAATGAGCTGCAGAAGGACAAGATGGCTGAGGCCTACTCTGA
GATTGGCATGAAGGGGGAGAGAAGAAGAGGCAAGGGCCATGATGGCCTGTACCA
AGGCCTGAGCACAGCCACCAAGGACACCTATGATGCCCTACACATGCAAGCTCTG
CCTCCTAGA
SEQ ID NO: 68 CD5-CD3z intracellular signaling domain protein sequence 
RVKFSRSADAPAYQQGQNQLYNELNLGRREEYDVLDKRRGRDPEMGGKPRRKNPQE
GLYNELQKDKMAEAYSEIGMKGERRRGKGHDGLYQGLSTATKDTYDALHMQALPPR
SEQ ID NO: 69 CD5-T2A nucleic acid sequence
   GAAGGAAGGGGCAGCCTACTGACCTGTGGGGATGTGGAGGAGAACCCTGGCCCC
SEQ ID NO: 70 CD5-T2A protein sequence
   EGRGSLLTCGDVEENPGP
SEQ ID NO: 71 CD5-CSF2RA signal nucleic acid sequence 
ATGTTGCTATTAGTAACCAGCCTGCTGCTGTGTGAGCTGCCCCACCCTGCCTTCCTG
TTAATCCCA
SEQ ID NO: 72 CD5-CSF2RA signal protein sequence
   MLLLVTSLLLCELPHPAFLLIP
SEQ ID NO: 73 CD5-tEGFR nucleic acid sequence 
CGAAAGGTATGTAATGGCATTGGCATTGGGGAGTTTAAGGACAGCCTGAGCATCAA
TGCCACCAACATCAAGCACTTCAAGAACTGCACAAGCATCAGTGGGGACTTGCAC
ATCCTGCCTGTGGCCTTCAGAGGGGACAGCTTCACCCACACCCCCCCCCTGGACC
CCCAAGAGCTGGACATCCTGAAGACAGTGAAGGAGATCACTGGCTTCTTGCTGAT
CCAAGCCTGGCCTGAGAACAGAACAGACCTGCATGCCTTTGAGAACCTGGAGATC
ATCAGAGGCAGAACCAAGCAGCATGGGCAGTTCAGCCTGGCTGTGGTGAGCCTGA
ACATCACAAGCCTGGGCCTGAGAAGCTTAAAGGAGATCTCTGATGGGGATGTGAT
CATCTCTGGCAACAAGAACCTGTGCTATGCCAACACCATCAACTGGAAGAAGCTG
TTTGGCACCTCTGGGCAGAAGACCAAGATCATCAGCAACAGAGGGGAGAACTCCT
GTAAGGCCACTGGCCAAGTGTGTCATGCCCTATGCAGCCCTGAGGGGTGCTGGGG
CCCTGAGCCTAGAGACTGTGTGAGCTGCAGAAATGTGAGCAGAGGCAGAGAGTG
TGTGGACAAGTGCAACCTGCTGGAGGGGGAGCCTAGAGAGTTTGTGGAGAACTC
TGAGTGTATTCAGTGTCATCCTGAGTGCCTGCCCCAAGCCATGAACATCACCTGCA
CTGGCAGAGGCCCTGACAACTGCATTCAGTGTGCCCACTACATTGATGGCCCCCAC
TGTGTGAAGACCTGCCCTGCTGGGGTGATGGGGGAGAACAACACCCTGGTGTGGA
AGTATGCTGATGCTGGCCATGTGTGTCACCTGTGCCATCCCAACTGCACCTATGGCT
GCACTGGCCCTGGCCTGGAGGGCTGCCCCACCAATGGTCCCAAGATTCCTAGCATT
GCCACTGGCATGGTGGGGGCCCTGCTCCTACTTCTGGTGGTTGCCCTGGGCATTGG
CCTGTTCATG
SEQ ID NO: 74 CD5-tEGFR protein sequence 
RKVCNGIGIGEFKDSLSINATNIKHFKNCTSISGDLHILPVAFRGDSFTHTPPLDPQELDI
LKTVKEITGFLLIQAWPENRTDLHAFENLEIIRGRTKQHGQFSLAVVSLNITSLGLRSLK
EISDGDVIISGNKNLCYANTINWKKLFGTSGQKTKIISNRGENSCKATGQVCHALCSPE
GCWGPEPRDCVSCRNVSRGRECVDKCNLLEGEPREFVENSECIQCHPECLPQAMNIT
CTGRGPDNCIQCAHYIDGPHCVKTCPAGVMGENNTLVWKYADAGHVCHLCHPNCT
YGCTGPGLEGCPTNGPKIPSIATGMVGALLLLLVVALGIGLFM
SEQ ID NO: 75 CD5-VHH-CD28z-CAR nucleic acid sequence 
ATGGCCCTACCTGTGACAGCCCTACTGTTACCCCTGGCCCTCCTTCTGCATGCTGCT
AGACCTCAGGTGCAGCTGGTGCAGTCTGGGGCTGAGGTGAAGAAGCCTGGGTCC
TCGGTGAAGGTCTCCTGCAAGGCTTCTGGAGGCACCTTCAGCAACTATGCTATCAG
CTGGGTGCGACAGGCCCCTGGACAAGGGCTTGAGTGGATGGGATGGATCAGCGCC
TACAATGGTGACACAAAATATGCACAGAGGCTCCAGGGCAGAGTCACCATGACCA
CAGACACATCCACGAGCACAGCCTACATGGAGCTGAGGAACCTAAGATCTGACGA
CACGGCCGTGTATTACTGTGCGCGCTACGAATCTATGTCTGGTCAGGATATCTGGGG
TCAAGGTACTCTGGTGACCGTCTCCTCAGGGGGGGGGGGCTCTGGGGGGGGTGGC
TCAGGTGGCGGTGGCTCTGAAGTTCAGCTGCTGGAAAGCGGTGGTGGTCTGGTTC
AGCCTGGTGGTAGCCTGCGTCTGAGCTGTGCAGCAAGCGGTTTTACCTTTAGCCAT
AGCGCCATGGGTTGGGTTCGTCAGGCACCTGGTAAAGGTCTGGAATGGGTTAGCA
GCATCTATGCCCGCGGCGGCTATACCTATTATGCAGATAGCGTTAAAGGTCGTTTTA
CCATTAGCCGTGATAACAGCAAAAATACCCTGTACCTGCAGATGAATAGTCTGCGT
GCAGAGGATACCGCAGTGTATTATTGTGCGCGCGGTTACCATCTGGAATACATGGTT
TCTCAGGATGTTTGGGGTCAAGGTACTCTGGTGACCGTCTCCTCATTTGTGCCTGTA
TTTCTGCCTGCCAAGCCCACCACAACACCTGCCCCTAGACCACCCACCCCTGCCCC
CACCATTGCTTCTCAGCCCCTTAGCTTAAGACCTGAAGCCTGTAGACCTGCTGCTG
GGGGGGCTGTGCACACAAGAGGCCTGGACTTTGCCTGTGACATCTACATCTGGGC
CCCCCTGGCTGGCACCTGTGGGGTGCTGCTGCTGAGCCTGGTGATCACCCTGTACT
GCAACCACAGAAACAGGAGTAAGAGGAGCAGGCTCCTGCACAGTGACTACATGA
ACATGACTCCCCGCCGCCCCGGGCCCACCCGCAAGCATTACCAGCCCTATGCCCCA
CCACGCGACTTCGCAGCCTATCGCTCCAGAGTGAAGTTCAGCAGATCTGCTGATGC
CCCTGCCTATCAGCAAGGGCAGAATCAGCTGTACAATGAGCTGAATCTGGGCAGA
AGAGAGGAGTATGATGTGCTGGACAAGAGAAGAGGCAGAGACCCTGAGATGGGG
GGCAAGCCTAGAAGAAAGAACCCCCAAGAGGGCCTGTATAATGAGCTGCAGAAG
GACAAGATGGCTGAGGCCTACTCTGAGATTGGCATGAAGGGGGAGAGAAGAAGA
GGCAAGGGCCATGATGGCCTGTACCAAGGCCTGAGCACAGCCACCAAGGACACCT
ATGATGCCCTACACATGCAAGCTCTGCCTCCTAGAGGCTCTGGGGAAGGAAGGGG
 
CAGCCTACTGACCTGTGGGGATGTGGAGGAGAACCCTGGCCCCATGTTGCTATTAG
TAACCAGCCTGCTGCTGTGTGAGCTGCCCCACCCTGCCTTCCTGTTAATCCCACGA
AAGGTATGTAATGGCATTGGCATTGGGGAGTTTAAGGACAGCCTGAGCATCAATGC
CACCAACATCAAGCACTTCAAGAACTGCACAAGCATCAGTGGGGACTTGCACATC
CTGCCTGTGGCCTTCAGAGGGGACAGCTTCACCCACACCCCCCCCCTGGACCCCC
AAGAGCTGGACATCCTGAAGACAGTGAAGGAGATCACTGGCTTCTTGCTGATCCA
AGCCTGGCCTGAGAACAGAACAGACCTGCATGCCTTTGAGAACCTGGAGATCATC
AGAGGCAGAACCAAGCAGCATGGGCAGTTCAGCCTGGCTGTGGTGAGCCTGAAC
ATCACAAGCCTGGGCCTGAGAAGCTTAAAGGAGATCTCTGATGGGGATGTGATCAT
CTCTGGCAACAAGAACCTGTGCTATGCCAACACCATCAACTGGAAGAAGCTGTTT
GGCACCTCTGGGCAGAAGACCAAGATCATCAGCAACAGAGGGGAGAACTCCTGT
AAGGCCACTGGCCAAGTGTGTCATGCCCTATGCAGCCCTGAGGGGTGCTGGGGCC
CTGAGCCTAGAGACTGTGTGAGCTGCAGAAATGTGAGCAGAGGCAGAGAGTGTG
TGGACAAGTGCAACCTGCTGGAGGGGGAGCCTAGAGAGTTTGTGGAGAACTCTG
AGTGTATTCAGTGTCATCCTGAGTGCCTGCCCCAAGCCATGAACATCACCTGCACT
GGCAGAGGCCCTGACAACTGCATTCAGTGTGCCCACTACATTGATGGCCCCCACTG
TGTGAAGACCTGCCCTGCTGGGGTGATGGGGGAGAACAACACCCTGGTGTGGAA
GTATGCTGATGCTGGCCATGTGTGTCACCTGTGCCATCCCAACTGCACCTATGGCTG
CACTGGCCCTGGCCTGGAGGGCTGCCCCACCAATGGTCCCAAGATTCCTAGCATTG
CCACTGGCATGGTGGGGGCCCTGCTCCTACTTCTGGTGGTTGCCCTGGGCATTGGC
CTGTTCATGTGA
SEQ ID NO: 76 CD5-VHH-CD28z-CAR protein sequence 
MALPVTALLLPLALLLHAARPQVQLVQSGAEVKKPGSSVKVSCKASGGTFSNYAISW
VRQAPGQGLEWMGWISAYNGDTKYAQRLQGRVTMTTDTSTSTAYMELRNLRSDDTA
VYYCARYESMSGQDIWGQGTLVTVSSGGGGSGGGGSGGGGSEVQLLESGGGLVQPG
GSLRLSCAASGFTFSHSAMGWVRQAPGKGLEWVSSIYARGGYTYYADSVKGRFTISR
DNSKNTLYLQMNSLRAEDTAVYYCARGYHLEYMVSQDVWGQGTLVTVSSFVPVFLP
AKPTTTPAPRPPTPAPTIASQPLSLRPEACRPAAGGAVHTRGLDFACDIYIWAPLAGTCG
VLLLSLVITLYCNHRNRSKRSRLLHSDYMNMTPRRPGPTRKHYQPYAPPRDFAAYRSR
VKFSRSADAPAYQQGQNQLYNELNLGRREEYDVLDKRRGRDPEMGGKPRRKNPQEG
LYNELQKDKMAEAYSEIGMKGERRRGKGHDGLYQGLSTATKDTYDALHMQALPPRG
SGEGRGSLLTCGDVEENPGPMLLLVTSLLLCELPHPAFLLIPRKVCNGIGIGEFKDSLSI
NATNIKHFKNCTSISGDLHILPVAFRGDSFTHTPPLDPQELDILKTVKEITGFLLIQAWPE
 
NRTDLHAFENLEIIRGRTKQHGQFSLAVVSLNITSLGLRSLKEISDGDVIISGNKNLCYA
NTINWKKLFGTSGQKTKIISNRGENSCKATGQVCHALCSPEGCWGPEPRDCVSCRNV
SRGRECVDKCNLLEGEPREFVENSECIQCHPECLPQAMNITCTGRGPDNCIQCAHYID
GPHCVKTCPAGVMGENNTLVWKYADAGHVCHLCHPNCTYGCTGPGLEGCPTNGPKI
PSIATGMVGALLLLLVVALGIGLFM*
 Here * stands for protein termination
SEQ ID NO: 77 BCMA-CAR nucleic acid sequence 
tacccatacgatgttccagattacgctgacatccagatgacccagtctccatcctccctgtctgcatctgtaggagacagagtcaccatca
cttgccgggcaagtcagagcattagcagctatttaaattggtatcagcagaaaccagggaaagcccctaagctcctgatctatgctgcat
ccagtttgcaaagtggggtcccatcaaggttcagtggcagtggatctgggacagatttcactctcaccatcagcagtctgcaacctgaa
gattttgcaacttactactgtcagcaaaaatacgacctcctcacttttggcggagggaccaaggttgagatcaaaggcagcaccagcgg
ctccggcaagcctggctctggcgagggcagcacaaagggacagctgcagctgcaggagtcgggcccaggactggtgaagccttcg
gagaccctgtccctcacctgcactgtctctggtggctccatcagcagtagtagttactactggggctggatccgccagcccccagggaa
ggggctggagtggattgggagtatctcctatagtgggagcacctactacaacccgtccctcaagagtcgagtcaccatatccgtagaca
cgtccaagaaccagttctccctgaagctgagttctgtgaccgccgcagacacggcggtgtactactgcgccagagatcgtggagaca
ccatactagacgtatggggtcagggtacaatggtcaccgtcagctcattcgtgcccgtgttcctgcccgccaaacctaccaccacccct
gcccctagacctcccaccccagccccaacaatcgccagccagcctctgtctctgcggcccgaagcctgtagacctgctgccggcgga
gccgtgcacaccagaggcctggacttcgcctgcgacatctacatctgggcccctctggccggcacctgtggcgtgctgctgctgagcc
tggtgatcaccctgtactgcaaccaccggaacagaagcaagcggagccggctgctgcacagcgactacatgaacatgaccccaaga
cggcctggccccacccggaagcactaccagccttacgcccctcccagagacttcgccgcctaccggtccagagtgaagttcagcag
atccgccgacgcccctgcctaccagcagggacagaaccagctgtacaacgagctgaacctgggcagacgggaagagtacgacgtg
ctggacaagcggagaggccgggaccccgagatgggcggaaagcccagacggaagaacccccaggaaggcctgtataacgaact
gcagaaagacaagatggccgaggcctacagcgagatcggcatgaagggcgagcggaggcgcggcaagggccacgatggcctgt
accagggcctgagcaccgccaccaaggacacctacgacgccctgcacatgcaggccctgccccccaga
SEQ ID NO: 78 BCMA-CAR protein sequence 
YPYDVPDYADIQMTQSPSSLSASVGDRVTITCRASQSISSYLNWYQQKPGKAPKLLIY
AASSLQSGVPSRFSGSGSGTDFTLTISSLQPEDFATYYCQQKYDLLTFGGGTKVEIKGS
TSGSGKPGSGEGSTKGQLQLQESGPGLVKPSETLSLTCTVSGGSISSSSYYWGWIRQPP
GKGLEWIGSISYSGSTYYNPSLKSRVTISVDTSKNQFSLKLSSVTAADTAVYYCARDR
GDTILDVWGQGTMVTVSSFVPVFLPAKPTTTPAPRPPTPAPTIASQPLSLRPEACRPAA
GGAVHTRGLDFACDIYIWAPLAGTCGVLLLSLVITLYCNHRNRSKRSRLLHSDYMNM
TPRRPGPTRKHYQPYAPPRDFAAYRSRVKFSRSADAPAYQQGQNQLYNELNLGRREE
YDVLDKRRGRDPEMGGKPRRKNPQEGLYNELQKDKMAEAYSEIGMKGERRRGKGH
DGLYQGLSTATKDTYDALHMQALPPR*
 Here * stands for protein termination
SEQ ID NO: 79hIL7 amino acid sequence 
MFHVSFRYIFGLPPLILVLLPVASSDCDIEGKDGKQYESVLMVSIDQLLDSMKEIGSNC
LNNEFNFFKRHICDANKEGMFLFRAARKLRQFLKMNSTGDFDLHLLKVSEGTTILLN
CTGQVKGRKPAALGEAQPTKSLEENKSLKEQKKLNDLCFLKRLLQEIKTCWNKILMG
TKEH
SEQ ID NO: 80 CCL19 amino acid sequence 
MALLLALSLLVLWTSPAPTLSGTNDAEDCCLSVTQKPIPGYIVRNFHYLLIKDGCRVP
AVVFTTLRGRQLCAPPDQPWVERIIQRLQRTSAKMKRRSS
SEQ ID NO: 81 IL2RB-CD3z amino acid sequence 
NCRNTGPWLKKVLKCNTPDPSKFFSQLSSEHGGDVQKWLSSPFPSSSFSPGGLAPEISP
LEVLERDKVTQLLPLNTDAYLSLQELQGQDPTHLVRVKFSRSADAPAYQQGQNQLY
NELNLGRREEYDVLDKRRGRDPEMGGKPRRKNPQEGLYNELQKDKMAEAYSEIGM
KGERRRGKGHDGLYQGLSTATKDTYDAYRHQALPPR
SEQ ID NO: 82 IL7RaMut amino acid sequence

MTILGTTFGMVFSLLQVVSGESGYAQNGDLEDAELDDYSFSCYSQLEVNGSQHSLTC
AFEDPDVNITNLEFEICGALVEVKCLNFRKLQEIYFIETKKFLLIGKSNICVKVGEKSLT
CKKIDLTTIVKPEAPFDLSVVYREGANDFVVTFNTSHLQKKYVKVLMHDVAYRQEK
DENKWTHVNLSSTKLTLLQRKLQPAAMYEIKVRSIPDHYFKGFWSEWSPSYYFRTPEI
NNSSGEMDPILLTCPTISILSFFSVALLVILACVLWKKRIKPIVWPSLPDHKKTLEHLCK
KPRKNLNVSFNPESFLDCQIHRVDDIQARDEVEGFLQDTFPQQLEESEKQRLGGDVQS
PNCPSEDVVITPESFGRDSSLTCLAGNVSACDAPILSSSRSLDCRESGKNGPHVYQDLL
LSLGTTNSTLPPPFSLQSGILTLNPVAQGQPILTSLGSNQEEAYVTMSSFYQNQ

References:
1 Blake, S., Hughes, T. P., Mayrhofer, G. & Lyons, A. B. The Src/ABL kinase inhibitor dasatinib (BMS-354825) inhibits function of normal human T-lymphocytes in vitro. Clin Immunol 127, 330-339, doi:10.1016/j.clim.2008.02.006 (2008).
2 Schade, A. E. et al. Dasatinib, a small-molecule protein tyrosine kinase inhibitor, inhibits T-cell activation and proliferation. Blood 111, 1366-1377, doi:10.1182/blood-2007-04-084814 (2008).
3 Weber, E. W. et al. Pharmacologic control of CAR-T cell function using dasatinib. Blood advances 3, 711-717, doi:10.1182/bloodadvances.2018028720 (2019).
4 Mestermann, K. et al. The tyrosine kinase inhibitor dasatinib acts as a pharmacologic on/off switch for CAR T cells. Science translational medicine 11, doi:10.1126/scitranslmed.aau5907 (2019).
5 Scheiter, M. et al. Protein Kinase Inhibitors CK59 and CID755673 Alter Primary Human NK Cell Effector Functions. Front Immunol 4, 66, doi:10.3389/fimmu.2013.00066 (2013).
6 Lee, K. C. et al. Lck is a key target of imatinib and dasatinib in T-cell activation. Leukemia 24, 896-900, doi:10.1038/leu.2010.11 (2010).
7 Deguchi, Y. et al. Comparison of imatinib, dasatinib, nilotinib and INNO-406 in imatinib-resistant cell lines. LeukRes 32, 980-983, doi:10.1016/j.leukres.2007.11.008 (2008).
8 Burgess, M. R., Skaggs, B. J., Shah, N. P., Lee, F. Y. & Sawyers, C. L. Comparative analysis of two clinically active BCR-ABL kinase inhibitors reveals the role of conformation-specific binding in resistance. Proc Natl Acad Sci U S A 102, 3395-3400, doi:10.1073/pnas.0409770102 (2005).
9 Bradeen, H. A. et al. Comparison of imatinib mesylate, dasatinib (BMS-354825), and nilotinib (AMN107) in an N-ethyl-N-nitrosourea (ENU)-based mutagenesis screen: high efficacy of drug combinations. Blood 108, 2332-2338, doi:10.1182/blood-2006-02-004580 (2006).
10 Wang, X. et al. Efficient base editing in methylated regions with a human APOBEC3A-Cas9 fusion. Nat Biotechnol 36, 946-949, doi:10.1038/nbt.4198 (2018).
11 Kluesner, M. G. et al. EditR: A Method to Quantify Base Editing from Sanger Sequencing. CRISPR J 1, 239-250, doi:10.1089/crispr.2018.0014 (2018).
12 Scheiter, M. et al. Protein Kinase Inhibitors CK59 and CID755673 Alter Primary Human NK Cell Effector Functions. Front Immunol 4, 66, doi:10.3389/fimmu.2013.00066 (2013).

## Claims

1. A cell comprising a mutant protein that causes the cell to be insensitive to an inhibitor affecting its activity and/or cytotoxicity.

2. The cell of claim 1, wherein the mutant protein is a member of tyrosine kinase family.

3. The cell of claim 1 or 2, wherein the mutant protein is LCK.

4. The cell of any one of claims 1-3, wherein the LCK protein comprises T316 mutation.

5. The cell of any one of claims 1-4, wherein the LCK protein comprises T316I, T316A or T316M mutation.

6. The cell of any one of claims 1-5, wherein the inhibitor is a tyrosine kinase inhibitor.

7. The cell of any one of claims 1-6, wherein the inhibitor is dasatinib and/or ponatinib.

8. The cell of any one of claims 1-7, wherein the cell is a mammalian cell.

9. The cell of any one of claims 1-8, wherein the cell is a stem cell or immune cell.

10. The cell of any one of claims 1-9, wherein the immune cell is an NK cell or T cell.

11. The cell of any one of claims 1-10, wherein the cell expresses a chimeric antigen receptor (CAR).

12. The cell of any one of claims 1-11, wherein the mutant protein is formed by base editing, HDR and/or overexpression.

13. The cell of any one of claims 1-12, wherein the overexpression is achieved through transfection with lentivirus, retrovirus, adeno-associated virus and adenovirus.

14. A method for introducing T316 mutation into an Lck gene of a cell, comprising introducing a base editor into the cell.

15. The method of claim 14, wherein the base editor is an ABE or CBE base editor.

16. The method of claim 14 or 15, further comprising introducing sgRNA into the cell.

17. The method of any one of claims 14-16, wherein the sgRNA comprises a nucleotide sequence as set forth in any one of SEQ ID NOs: 5-8.

18. The method of any one of claims 14-17, wherein the CBE base editor is an A3A-CBE3 fusion protein.

19. The method of any one of claims 14-18, wherein the cell is a mammalian cell.

20. The method of any one of claims 14-19, wherein the cell is a stem cell or immune cell.

21. The method of any one of claims 14-20, wherein the immune cell is an NK cell or T cell.

22. The method of any one of claims 14-21, wherein the cell expresses a chimeric antigen receptor (CAR).

23. A cell expressing a chimeric antigen receptor, wherein the cell has cytotoxicity or is induced to have cytotoxicity, and the cell is engineered so that its cytotoxicity is insensitive to a cell activity inhibitor.

24. The cell of claim 23, wherein the cell activity inhibitor is a T cell activity inhibitor.

25. The cell of claim 23 or 24, wherein at least one bioactive molecule in the cell is engineered to be insensitive to the cell activity inhibitor, and the bioactive molecule can be inhibited by the cell activity inhibitor in a normal T cell.

26. The cell of any one of claims 23-25, wherein the bioactive molecule acts on a chimeric antigen receptor signal transduction pathway.

27. The cell of any one of claims 23-26, wherein the bioactive molecule is a protease.

28. The cell of any one of claims 23-27, wherein the protease is a protein tyrosine kinase.

29. The cell of any one of claims 23-28, wherein the protease is LCK protein.

30. The cell of any one of claims 23-29, wherein the Lck protein tyrosine kinase comprises T316 mutation.

31. The cell of any one of claims 23-30, wherein the Lck protein tyrosine kinase comprises T316I, T316A or T316M mutation.

32. The cell of any one of claims 23-31, wherein the engineering is accomplished by using a base editor.

33. The cell of any one of claims 23-32, wherein the base editor is an ABE or CBE base editor.

34. The cell of any one of claims 23-33, wherein the T316I mutation is obtained by introducing a base editor CBE and sgRNA into the cell, and the target sequence of the sgRNA comprises a nucleotide sequence as set forth in any one of SEQ ID NOs: 5-8.

35. The cell of any one of claims 23-34, wherein the LCK protein in the cell comprises an amino acid sequence as set forth in any one of SEQ ID NOs: 9-11.

36. The cell of any one of claims 23-35, wherein the cell is a T cell or NK cell.

37. The cell of any one of claims 23-36, wherein the cell is further engineered to eliminate or weaken cytotoxicity generated via TCR on the cell surface thereof.

38. The cell of any one of claims 23-37, wherein a TCR-related gene of the cell is knocked out.

39. The cell of any one of claims 23-38, wherein the TRAC gene of the cell is knocked out.

40. The cell of any one of claims 23-39, wherein the β2m gene of the cell is knocked out; and optionally, the CUT A gene of the cell is knocked out.

41. The cell of any one of claims 23-40, wherein the T cell activity inhibitor is a protein tyrosine kinase inhibitor.

42. The cell of any one of claims 23-41, wherein the T cell activity inhibitor is an LCK protein tyrosine kinase inhibitor.

43. The cell of any one of claims 23-42, wherein the T cell activity inhibitor is dasatinib and/or ponatinib.

44. Use of the cell of any one of claims 1-13 and 23-43 in preparation of a universal CAR-T cell.

45. A method for preparing a CAR cell, comprising engineering the CAR cell so that its CAR-mediated cytotoxicity is insensitive to a T cell activity inhibitor.

46. The method of claim 45, wherein at least one bioactive molecule in the CAR cell is engineered to be insensitive to the T cell activity inhibitor, and the bioactive molecule can be inhibited by the T cell activity inhibitor in a normal T cell.

47. The method of claim 45 or 46, wherein the bioactive molecule acts on a signal transduction pathway of the CAR.

48. The method of any one of claims 45-47, wherein the bioactive molecule is a protease.

49. The method of any one of claims 45-48, wherein the protease is a protein tyrosine kinase.

50. The method of any one of claims 45-49, wherein the enzyme is an LCK protein tyrosine kinase.

51. The method of any one of claims 45-50, wherein the LCK protein tyrosine kinase comprises T316 mutation.

52. The method of any one of claims 45-51, wherein the LCK protein tyrosine kinase compirses T316I mutation.

53. The method of any one of claims 45-52, wherein the T316I mutation is obtained by introducing a cytosine base editor and sgRNA into the CAR cell, and the sgRNA comprises a nucleotide sequence as set forth in any one of SEQ ID NOs: 5-8.

54. The method of any one of claims 45-53, wherein the LCK protein tyrosine kinase in the CAR cell comprises an amino acid sequence as set forth in any one of SEQ ID NOs: 9-11.

55. The method of any one of claims 45-54, wherein the CAR cell is a T cell or NK cell.

56. The method of any one of claims 45-55, wherein the CAR cell is further engineered to eliminate or weaken the cytotoxicity generated via TCR on the cell surface thereof.

57. The method of any one of claims 45-56, wherein a TCR-related gene of the CAR cell is knocked out.

58. The method of any one of claims 45-57, wherein the TRAC gene of the CAR cell is knocked out.

59. The method of any one of claims 45-58, wherein the β2m gene of the CAR cell is knocked out; and optionally, the CIITA gene of the CAR cell is knocked out.

60. The method of any one of claims 45-59, wherein the T cell activity inhibitor is a protein tyrosine kinase inhibitor.

61. The method of any one of claims 45-60, wherein the T cell activity inhibitor is an LCK protein tyrosine kinase inhibitor.

62. The method of any one of claims 45-61, wherein the T cell activity inhibitor is dasatinib and/or ponatinib.

63. The method of any one of claims 45-62, wherein during the preparation of the CAR cell from a T cell or NK cell, the T cell or NK cell is contacted with the T cell activity inhibitor.

64. The method of any one of claims 45-63, wherein the T cell or NK cell is contacted with the T cell activity inhibitor when the T316I mutation is carried out; preferably, the concentration of the T cell activity inhibitor is 100 nM.

65. The cell of any one of claims 23-43, the use of claim 44, or the method of any one of claims 45-64, wherein the intracellular signaling domain of the CAR comprises:
1) a signaling transduction domain from CD3z molecule and a co-stimulatory domain from CD28 molecule; and
2) optionally, i) hIL7 and CCL19, or ii) IL2RB and IL7Ra mutant,
wherein, preferably, the signaling transduction domain from CD3z molecule comprises an amino acid sequence as set forth in SEQ ID NO: 48; the co-stimulatory domain from CD28 molecule comprises an amino acid sequence as set forth in SEQ ID NO: 46; the hIL7 comprises an amino acid sequence as set forth in SEQ ID NO: 79; the CCL19 comprises an amino acid sequence as set forth in SEQ ID NO: 80; the IL2RB and the co-stimulatory domain from CD28 molecule form a IL2RB-CD3z peptide fragment which comprises an amino acid sequence as set forth in SEQ ID NO: 81; and the IL7Ra mutant comprises an amino acid sequence as set forth in SEQ ID NO: 82.

66. A method for treating a disease in a patient, comprising administering to the patient the cells of any one of claims 12-29 in combination with a cell activity inhibitor.

67. The method of claim 66, wherein the cells are not derived from the patient.

68. The method of claim 66 or 67, wherein the cells are T cells.

69. The method of any one of claims 66-68, wherein the cell inhibitor is a T cell activity inhibitor.

70. The method of any one of claims 66-69, wherein the cell activity inhibitor is a protein tyrosine kinase inhibitor.

71. The method of any one of claims 66-70, wherein the cell activity inhibitor is an LCK protein inhibitor.

72. The method of any one of claims 66-71, wherein the T cell activity inhibitor is dasatinib and/or ponatinib.

73. The method of any one of claims 66-72, wherein the disease is a tumor.

74. The method of any one of claims 66-73, wherein the method further comprises stopping the administration of the cell activity inhibitor, so that the patient's own T cells regain activity and clear the administered cells; or increasing the dose of the cell activity inhibitor to inhibit the cytotoxicity of the cells in the patient.

75. A pharmaceutical kit or pharmaceutical combination, comprising the cells of any one of claims 23-43 and a cell activity inhibitor.

76. The pharmaceutical kit or pharmaceutical combination of claim 75, wherein the cells are T cells.

77. The pharmaceutical kit or pharmaceutical combination of claim 75 or 76, wherein the cell activity inhibitor is a T cell activity inhibitor.

78. The pharmaceutical kit or pharmaceutical combination of any one of claims 75-77, wherein the cell inhibitor is a protein tyrosine kinase inhibitor.

79. The pharmaceutical kit or pharmaceutical combination of any one of claims 75-78, wherein the T cell activity inhibitor is an LCK protein tyrosine kinase inhibitor.

80. The pharmaceutical kit or pharmaceutical combination of any one of claims 75-79, wherein the T cell activity inhibitor is dasatinib and/or ponatinib.

81. Use of the cells of any one of claims 23-43 in combination with a cell activity inhibitor in preparing an anti-tumor drug.

82. The use of claim 81, wherein the cells are T cells.

83. The use of claim 81 or 82, wherein the cell activity inhibitor is a T cell activity inhibitor.

84. The use of any one of claims 81-83, wherein the T cell activity inhibitor is a protein tyrosine kinase inhibitor.

85. The use of any one of claims 81-84, wherein the T cell activity inhibitor is an LCK protein tyrosine kinase inhibitor.

86. The use of any one of claims 81-85, wherein the T cell activity inhibitor is dasatinib and/or ponatinib.
